(19)

**Europäisches Patentamt**
**European Patent Office**
**Office européen des brevets**

(11) **EP 4 432 290 A1**

(12) **EUROPEAN PATENT APPLICATION**
published in accordance with Art. 153(4) EPC

(43) Date of publication:
**18.09.2024 Bulletin 2024/38**

(21) Application number: **22890302.7**

(22) Date of filing: **31.10.2022**

(51) International Patent Classification (IPC):
***G16B 40/00*** (2019.01)          ***G16B 30/10*** (2019.01)
***G16B 20/20*** (2019.01)

(52) Cooperative Patent Classification (CPC):
**G16B 20/20; G16B 30/10; G16B 40/00**

(86) International application number:
**PCT/KR2022/016787**

(87) International publication number:
**WO 2023/080571 (11.05.2023 Gazette 2023/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.11.2021 KR 20210152018
08.11.2021 KR 20210152019**

(71) Applicant: **Seegene, Inc.
Seoul 05548 (KR)**

(72) Inventor: **YOON, Seok Hwan
Seoul 06218 (KR)**

(74) Representative: **Balder IP Law, S.L.
Paseo de la Castellana 93
5ª planta
28046 Madrid (ES)**

(54) **METHOD FOR SELECTING SEQUENCE IDENTIFIER FOR DETECTION OF TARGET ANALYTE**

(57)    Disclosed is a method for selecting a sequence identifier for detecting a target analyte, which is performed by a computing device. The method may include: obtaining a plurality of first sequence identifiers found in a first layer at which a target analyte is positioned in a biological taxonomy of organisms having a hierarchical structure, selecting second sequence identifiers among the first sequence identifiers at least partially based on an occurring frequency of the first sequence identifiers in the first layer and an occurring frequency of the first sequence identifiers in a second layer which is a higher layer than the first layer and selecting a third sequence identifier for detecting the target analyte among the second sequence identifiers at least partially based on an occurring frequency of the second sequence identifiers in the first layer and an occurring frequency of the second sequence identifiers in a third layer which is a higher layer than the second layer. A representative figure may be FIG. 6.

[FIG. 6]

**Description**

[Technical Field]

The present disclosure relates to a method for selecting a sequence identifier for detecting a target analyte, and more particularly, to a method for selecting a sequence identifier specific to a target analyte in order to detect the target analyte.

[Background Art]

As the next generation sequencing (NGS) technology has been developed and the set of a large number of base sequences can be processed in parallel, the base sequence analysis technology is utilized in the form applicable to various fields.

As the center of therapeutic medicine is transformed to preventive medicine, with the development of molecular genetic technology, the technology that searches base sequences specific to a specific organism group is particularly spotlighted in the field of in vitro diagnostic (IVD) (especially molecular diagnosis).

In order to explore the candidate base sequence specific to a specific organism group, a scheme that uses sequence search software commercially available for a single gene or multiple genes is used to contrast whether there is a sequence similar to the corresponding sequence with respect to an entire a organism group is generally used. As the amount of genetic information newly stored in a database increases rapidly, when such a scheme of sequence exploration technology is used, a lot of computing resources are unnecessarily consumed, and a relatively long time cannot but be consumed for sequence search. Further, the existing base sequence and genetic data structure is evaluated as a structure in which it is impossible to search a candidate gene such as a diagnostic marker for billions of gene data included in millions of genome data.

As such, it is difficult to discover candidate base sequences corresponding to a specific base sequence because a scheme of searching a base sequence similar or specific to a specific base sequence within the entire organism group requires a complicated calculation scheme.

Korean Patent Unexamined Publication No. 2017-0046315 presents a technology of aligning and comparing base sequences of a mother model and a father model in a known reference sequence in order to search a single base sequence mutation of each of the mother model and the father model obtained through next generation sequencing.

[Disclosure]

[Technical Problem]

The present disclosure is contrived in response to the background art, and has been made in an effort to search a base sequence having high sensitivity and having high specificity distinguished from other organism groups in a designated organism group by an efficient scheme.

[Technical Solution]

In order to achieve the object, according to an embodiment of the present disclosure, disclosed is a method for selecting a sequence identifier for detecting a target analyte, which is performed by a computing device. The method may comprise: obtaining a plurality of first sequence identifiers found in a first layer at which a target analyte is positioned in a biological taxonomy of organisms having a hierarchical structure, selecting second sequence identifiers among the first sequence identifiers at least partially based on an occurring frequency of the first sequence identifiers in the first layer and an occurring frequency of the first sequence identifiers in a second layer which is a higher layer than the first layer and selecting a third sequence identifier for detecting the target analyte among the second sequence identifiers at least partially based on an occurring frequency of the second sequence identifiers in the first layer and an occurring frequency of the second sequence identifiers in a third layer which is a higher layer than the second layer.

According to an embodiment of the present disclosure, the selecting of the second sequence identifiers may comprise obtaining the occurring frequency of the first sequence identifiers in the first layer by using the number of genomes to which the first sequence identifiers belong among first genomes found in the first layer, and obtaining the occurring frequency of the first sequence identifiers in the second layer by using the number of genomes to which the first sequence identifiers belong among second genomes found in the second layer, and wherein the selecting of the third sequence identifiers comprises obtaining the occurring frequency of the second sequence identifiers in the first layer by using the number of genomes to which the second sequence identifiers belong among the first genomes found in the first layer, and obtaining the occurring frequency of the second sequence identifiers in the third layer by using the number of genomes to which the second sequence identifiers belong among third genomes found in the third layer.

According to an embodiment of the present disclosure, the obtaining of the occurring frequency of the first sequence identifiers in the second layer may comprise obtaining the occurring frequency of the first sequence identifiers in which the first genomes are excluded from the second genomes found in the second layer.

According to an embodiment of the present disclosure, the obtaining of the occurring frequency of the second sequence identifiers in the third layer may comprise obtaining the occurring frequency of the second sequence identifiers in which the first genomes are excluded from the third genomes found in the third layer or in which the second genomes are excluded from the third genomes.

According to an embodiment of the present disclosure, the selecting of the second sequence identifiers may comprise computing the occurring frequency of the first sequence identifiers in a difference set of the first layer for the second layer.

According to an embodiment of the present disclosure, the selecting of the third sequence identifier may comprise computing an occurring frequency for the second sequence identifiers in a difference set of the first layer for the third layer or a difference set of the second layer for the third layer.

According to an embodiment of the present disclosure, the selecting of the second sequence identifiers may comprise selecting, as the second sequence identifiers, sequence identifiers in which the occurring frequency of each of the first sequence identifiers in the first layer is equal to or more than a first threshold and the occurring frequency of each of the first sequence identifiers in the second layer is equal to or less than a second threshold, and wherein the first threshold is a minimum reference value for the occurring frequency of each of the first sequence identifiers in the first layer, and the second threshold is a maximum reference value for the occurring frequency of each of the first sequence identifiers in the second layer.

According to an embodiment of the present disclosure, the selecting of the second sequence identifiers may comprise changing at least one of the first threshold or the second threshold when the number of first sequence identifiers in which the occurring frequency in the first layer for each of the first sequence identifiers is equal to or more than a first threshold and the occurring frequency in the second layer for each of the first sequence identifiers is equal to or less than a second threshold is less than a predetermined number or more than the predetermined number.

According to an embodiment of the present disclosure, the method may further comprise removing a sequence identifier of a specific organism in which exclusivity is predetermined among the selected second sequence identifiers.

According to an embodiment of the present disclosure, the predetermined sequence identifier may comprise the sequence identifier found in the resident flora or the housekeeping gene.

According to an embodiment of the present disclosure, the selecting of the third sequence identifier may comprise selecting, as the third sequence identifiers, sequence identifiers in which the occurring frequency of each of the second sequence identifiers in the third layer is equal to or less than a third threshold and the occurring frequency of each of the second sequence identifiers in the first layer is equal to or more than a fourth threshold, and wherein the third threshold is a maximum reference value for the occurring frequency of each of the second sequence identifiers in the third layer, and the fourth threshold is a minimum reference value for the occurring frequency of each of the second sequence identifiers in the first layer.

According to an embodiment of the present disclosure, the method may further comprise removing the sequence identifier of the specific organism in which exclusivity is predetermined among the selected third sequence identifiers.

According to an embodiment of the present disclosure, the predetermined sequence identifier may comprise the sequence identifier found in a resident flora or a housekeeping gene.

According to an embodiment of the present disclosure, the selecting of the third sequence identifier may comprise selecting the third sequence identifier for detecting the target analyte among the second sequence identifiers by additionally considering the occurring frequency of the second sequence identifiers in the second layer.

According to an embodiment of the present disclosure, the selecting of the third sequence identifier may comprise selecting the third sequence identifier among the second sequence identifiers by calculating a score of each of the second sequence identifiers based on the occurring frequency of each of the second sequence identifiers in the third layer, the occurring frequency of each of the second sequence identifiers in the first layer, and the occurring frequency of each of the second sequence identifiers in the second layer, and wherein the more the occurring frequency of each of the second sequence identifiers in the first layer, a higher score is calculated, the lower the occurring frequency of each of the second sequence identifiers in the third layer, a higher score is calculated, and the lower the occurring frequency of each of the second sequence identifiers in the second layer, a higher score is calculated.

According to an embodiment of the present disclosure, the score may be calculated by $Score = ((F_{in}/G_{in}) \times ((G_{total}+G_{in})/G_{total})) / (((F_{exp}/G_{exp})/((G_{total}+G_{exp})/G_{total})) + (F_{ext}/G_{ext}))$.

$F_{in}$ is the number of genomes having a second sequence identifier in the first layer, $G_{in}$ is the number of genomes found in the first layer, $G_{total}$ is the number of genomes found in the third layer, $F_{exp}$ is the number of genomes having a second sequence identifier in the second layer, $F_{ext}$ is the number of genomes having a second sequence identifier in the third layer, $G_{exp}$ is the number of genomes found in a difference of a set of the second layer and a set of the first layer, and Gext is the number of genomes found in a difference of a set of the third layer and a set of the first layer or a difference of a set of the third layer and a set of the second layer.

According to an embodiment of the present disclosure, the hierarchical structure may be a biological systematic structure in which a higher layer encompasses a lower layer.

According to an embodiment of the present disclosure, the second layer has a hierarchical position immediately higher from the first layer and the second layer has the number of genomes being more than a threshold number.

According to an embodiment of the present disclosure, the third layer may be a highest layer in the hierarchical structure.

According to an embodiment of the present disclosure, the third sequence identifier may be a candidate sequence identifier to be considered as a sequence identifier specific to the target analyte.

According to an embodiment of the present disclosure, the occurring frequency of the first sequence identifiers in the first layer may be obtained from a first data structure representing which source genome among the genomes in the first layer the plurality of first sequence identifiers belongs to.

According to an embodiment of the present disclosure, the occurring frequency of the first sequence identifiers in the second layer may be obtained from a second data structure representing which source genome among the genomes in the second layer the plurality of first sequence identifiers belongs to.

Disclosed is a computer program stored in a computer readable medium according to an embodiment of the present disclosure. When the computer program is executed by one or more processors, the computer program may comprise instructions for allowing the one or more processors to perform the following steps. The method may comprise: obtaining a plurality of first sequence identifiers found in a first layer at which a target analyte is positioned in a biological taxonomy of organisms having a hierarchical structure, selecting second sequence identifiers among the first sequence identifiers at least partially based on an occurring frequency of the first sequence identifiers in the first layer and an occurring frequency of the first sequence identifiers in a second layer which is a higher layer than the first layer and selecting a third sequence identifier for detecting the target analyte among the second sequence identifiers at least partially based on an occurring frequency of the second sequence identifiers in the first layer and an occurring frequency of the second sequence identifiers in a third layer which is a higher layer than the second layer.

According to an embodiment of the present disclosure, disclosed is a computing device for selecting a sequence identifier. The computing device may comprise: a memory, and a processor. The processor may obtain a plurality of first sequence identifiers found in a first layer at which a target analyte is positioned in a biological taxonomy of organisms having a hierarchical structure, select second sequence identifiers among the first sequence identifiers at least partially based on an occurring frequency of the first sequence identifiers in the first layer and an occurring frequency of the first sequence identifiers in a second layer which is a higher layer than the first layer and select a third sequence identifier for detecting the target analyte among the second sequence identifiers at least partially based on an occurring frequency of the second sequence identifiers in the first layer and an occurring frequency of the second sequence identifiers in a third layer which is a higher layer than the second layer.

According to an embodiment of the present disclosure, disclosed is a method for selecting a sequence identifier for detecting a target analyte, which is performed by a computing device. The method may comprise: obtaining a plurality of first sequence identifiers found in a first layer at which a target analyte is positioned in a biological taxonomy of organisms having a hierarchical structure, obtaining the occurring frequency of the first sequence identifiers in the first layer by using a first data structure representing which source genome among the genomes in the first layer the plurality of first sequence identifiers belong to, obtaining the occurring frequency of the first sequence identifiers in the second layer by using a second data structure representing which source genome among the genomes in the second layer higher than the first layer the plurality of first sequence identifiers belong to and selecting second sequence identifiers for detecting the target analyte among the first sequence identifiers at least partially based on an occurring frequency of the first sequence identifiers in the first layer and an occurring frequency of the first sequence identifiers in a second layer.

According to an embodiment of the present disclosure, in the first data structure, the plurality of first sequence identifiers corresponds to a key, and a genome to which the sequence identifiers as the key in the first layer belong corresponds to a value.

According to an embodiment of the present disclosure, in the second data structure, the plurality of first sequence identifiers corresponds to a key, and a genome to which the sequence identifiers as the key in the second layer belong corresponds to a value..

According to an embodiment of the present disclosure, the first data structure and the second data structure are data structures in which inverted-indexing is applied to a data structure having a plurality of sequence identifiers mapped to each of a plurality of genomes.

According to an embodiment of the present disclosure, the data structure to which the inverted-indexing generates a data structure in which the plurality of genomes are mapped to each of the plurality of sequence identifiers.

According to an embodiment of the present disclosure, the first data structure may be generated from a plurality of tokens for a plurality of first sequence identifiers listed to belong to each genome in the first layer, and here, one sequence identifier may correspond to one token.

According to an embodiment of the present disclosure, the second data structure may be generated from a plurality of tokens for a plurality of first sequence identifiers listed to belong to each genome in the second layer, and here, one sequence identifier may correspond to one token.

According to an embodiment of the present disclosure, the token may be generated by tokenization using space or comma as a stopword that exists in an array of the plurality of the first sequence identifiers.

According to an embodiment of the present disclosure, the second data structure comprises data in a difference of a set of the second layer and a set of the first layer.

According to an embodiment of the present disclosure, at least one of the plurality of first sequence identifier may be at least partial region of the genic region and/or at least partial region of the intergenic region.

According to an embodiment of the present disclosure, the selecting of the second sequence identifiers may comprise selecting, as the second sequence identifiers, sequence identifiers in which the occurring frequency of each of the first sequence identifiers in the first layer is equal to or more than a first threshold and the occurring frequency of each of the first sequence identifiers in the second layer is equal to or less than a second threshold, and wherein the first threshold is a minimum reference value for the occurring frequency of each of the first sequence identifiers in the first layer , and the second threshold is a maximum reference value for the occurring frequency of each of the first sequence identifiers in the second layer.

According to an embodiment of the present disclosure, the selecting of the second sequence identifiers may comprise changing at least one of the first threshold or the second threshold when the number of first sequence identifiers in which the occurring frequency in the first layer for each of the first sequence identifiers is equal to or more than a first threshold and the occurring frequency in the second layer for each of the first sequence identifiers is equal to or less than a second threshold is less than a predetermined number or more than the predetermined number.

According to an embodiment of the present disclosure, the method may further comprise removing the sequence identifier of the specific organism in which exclusivity is predetermined among the selected second sequence identifiers.

According to an embodiment of the present disclosure, the predetermined sequence identifier of the specific organism may comprise the sequence identifier found in a resident flora or a housekeeping gene.

According to an embodiment of the present disclosure, the method may further comprise selecting a third sequence identifier for detecting the target analyte among the second sequence identifiers at least partially based on an occurring frequency of the second sequence identifiers inthe first layer and an occurring frequency of the second sequence identifiers in a third layer which is a higher layer than the second layer.

According to an embodiment of the present disclosure, disclosed is a method for generating a data structure for detecting a target analyte, which is performed by a computing device. The method may comprise: generating a first data structure in which inverted-indexing is applied to a data structure in which a plurality of first sequence identifiers are mapped to a plurality of genomes found in a first layer at which a target analyte is positioned, respectively in a biological taxonomy of organisms having a hierarchical structure, generating a second data structure in which the inverted-indexing is applied to a data structure in which the plurality of sequence identifiers are mapped to a plurality of genomes found in a second layer which is a higher layer than the first layer at which the target analyte is positioned, respectively and selecting second sequence identifiers for detecting the target analyte among the first sequence identifiers at least partially based on an occurring frequency of the first sequence identifiers in the first layer obtained from the first data structure and an occurring frequency of the first sequence identifiers in a second layer obtained from the second data structure.

According to an embodiment of the present disclosure, disclosed is a computing device for selecting a sequence identifier for detecting a target analyte. The computing device may comprise: a memory, and a processor, and the processor may obtain a plurality of first sequence identifiers found in a first layer at which a target analyte is positioned in a biological taxonomy of organisms having a hierarchical structure, obtain a first occurring frequency of the first sequence identifiers in the first layer by using a first data structure representing which source genome among the genomes in the first layer the plurality of first sequence identifiers belong to, obtain a second occurring frequency of the first sequence identifiers in the second layer by using a second data structure representing which source genome among the genomes in the second layer higher than the first layer the plurality of first sequence identifiers belongs to and select second sequence identifiers for detecting the target analyte among the first sequence identifiers at least partially based on an occurring frequency of the first sequence identifiers in the first layer and an occurring frequency of the first sequence identifiers in a second layer.

[Advantageous Effects]

According to an embodiment of the present disclosure, an effect that it is possible to search a base sequence having high sensitivity and having high specificity distinguished from other organism groups in a designated organism group by an efficient scheme can be achieved.

[Description of Drawings]

FIG. 1 schematically illustrates a block configuration diagram of a computing device according to an embodiment of the present disclosure.

FIG. 2 schematically illustrates a block configuration diagram of a system according to an embodiment of the present disclosure.

FIG. 3 exemplarily illustrates a conceptual view for a process of selecting a sequence identifier according to an embodiment of the present disclosure.

FIG. 4 exemplarily illustrates layers utilized for selecting the sequence identifier in a biological taxonomy of organisms having a hierarchical structure according to an embodiment of the present disclosure.

FIG. 5 illustrates an exemplary data structure used for computing an occurring frequency of sequence identifiers by considering a first layer and a second layer according to an embodiment of the present disclosure.

FIG. 6 illustrates an exemplary data structure used for computing the occurring frequency of the sequence identifiers by considering the first layer and a third layer according to an embodiment of the present disclosure.

FIG. 7 is a flowchart exemplarily illustrating a method for selecting a candidate sequence identifier specific to a target analyte according to an embodiment of the present disclosure.

FIG. 8 is a flowchart exemplarily illustrating a method for selecting a target sequence identifier specific to the target analyte according to an embodiment of the present disclosure.

FIG. 9 is a flowchart exemplarily illustrating a method for selecting the candidate sequence identifier specific to the target analyte according to an embodiment of the present disclosure.

FIG. 10 is a flowchart exemplarily illustrating a method for generating an index data structure for searching a sequence identifier according to an embodiment of the present disclosure.

FIG. 11 is a flowchart exemplarily illustrating a method for filtering sequence identifiers by using the index data structure according to an embodiment of the present disclosure.

FIG. 12 illustrates an exemplary index data structure according to an embodiment of the present disclosure.

FIG. 13 exemplarily illustrates a conceptual view of performing a full text search according to an embodiment of the present disclosure.

FIG. 14 exemplarily illustrates an inverted-indexing technique according to an embodiment of the present disclosure.

FIG. 15 exemplarily illustrates a sequence identifier selection result according to an embodiment of the present disclosure.

FIG. 16 is a schematic view of a computing environment according to an embodiment of the present disclosure.

[Best Mode]

Various exemplary embodiments and/or aspects will be now disclosed with reference to drawings. In the following description, for the purpose of a description, multiple detailed matters will be disclosed in order to help comprehensive appreciation of one or more aspects. In describing the present disclosure, a detailed description of known function or constitutions will be omitted if it is determined that it unnecessarily makes the gist of the present disclosure unclear. Furthermore, the terms or words used in this specification and the claims should be interpreted in line with the technical concept of the present disclosure, in accordance with the principle that the inventor can define the concepts of appropriate terms in order to describe their invention in the best possible manner.

"Component", "module", "system", "unit" and the like which are terms used in this specification may be used to be compatible with each other and refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Also, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

In addition, the word "comprises" and/or "comprising" means that the corresponding feature and/or component is present, but it should be appreciated that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case wherein only A is included", "a case where only B is included", or "a case where A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each specific application. However, such implementation decisions should not be interpreted as causing a departure from the scope of the present disclosure.

The description of the presented embodiments is provided so that those skilled in the art of the present disclosure use or implement the present disclosure. Various modifications of the exemplary embodiments will be apparent to those skilled in the art and general principles defined herein can be applied to other exemplary embodiments without departing from the scope of the present disclosure. Therefore, the present disclosure is not limited to the exemplary embodiments presented herein, but should be interpreted within the widest range which is coherent with the principles and new features presented herein.

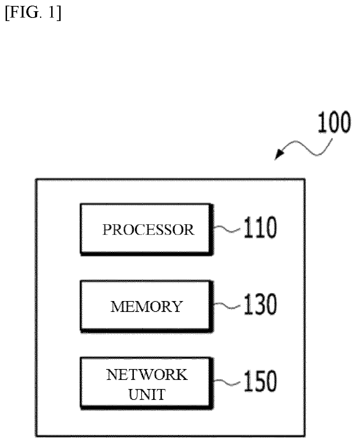

FIG. 1 schematically illustrates a block configuration diagram of a computing device 100 according to an embodiment of the present disclosure.

According to an embodiment of the present disclosure, the computing device 100 may comprise a processor 110, a memory 130, and a network unit 150.

A configuration of the computing device 100 illustrated in FIG. 1 is only an example shown through simplification. In an embodiment of the present disclosure, the computing device 100 may comprise other components for performing a computing environment of the computing device 100, and only some of the disclosed components may also constitute the computing device 100.

In the present disclosure, the computing device 100 may mean a node constituting a system for implementing embodiments of the present disclosure. The computing device 100 may mean any type of user terminal or any type of server. The components of the computing device 100 may be exemplary and some components may be excluded, or an additional component may be found in the computing device 100. As an example, when the computing device 100 comprises a terminal, an output unit (not illustrated) and an input unit (not illustrated) may be found inin the computing device 100.

The computing device 100 in the present disclosure may perform technical features according to embodiments of the present disclosure to be described below. The computing device 100 may perform processes for organizing an index structure. The computing device 100 may perform processes for selecting a sequence identifier. For example, the computing device 100 may obtain a plurality of first sequence identifiers found in a first layer where a target analyte is positioned on a biological taxonomy of organisms having a hierarchical structure. The computing device 100 may select second sequence identifiers among first sequence identifiers at least partially based on an occurring frequency in a first layer and an occurring frequency in a second layer which is a layer higher than the first layer with respect to each of the first sequence identifiers. The computing device 100 may select a third sequence identifier related to the target analyte among the second sequence identifiers at least partially based on an occurring frequency in a third layer which is a layer higher than the second layer and the occurring frequency in the first layer with respect to each of the second sequence identifiers.

The "target analyte" used in the present disclosure may comprise any form of organisms to be analyzed, obtained, or detected. For example, the organism may mean an organism which belongs to one genus, species, subspecies, sub type, geno type, sero type, strain, isolate, or cultivar. The organism may comprise prological cells (e.g., Mycoplasma pneumoniae, Chlamydophila pneumoniae, Legionella pneumophila, Haemophilus influenzae, Streptococcus pneumoniae, Bordetella pertussis, Bordetella parapertussis, Neisseria meningitidis, Listeria monocytogenes, Streptococcus agalactiae, Campylobacter, Clostridium difficile, Clostridium perfringens, Salmonella, Escherichia coli, Shigella, Vibrio, Yersinia enterocolitica, Aeromonas, Chlamydia trachomatis, Neisseria gonorrhoeae, Trichomonas vaginalis, Mycoplasma hominis, Mycoplasma genitalium, Ureaplasma urealyticum, Ureaplasma parvum, Mycobacterium tuberculosis, Treponema pallidum, Candida, Mobiluncus, Megasphaera, Lacto spp., Mycoplasma genitalium, Clostridium difficile, Helicobacter Pylori, ClariR, CPE, Group B Streptococcus, Enterobacter cloacae complex, Proteus mirabilis, Klebsiella aerogenes, Pseudomonas aeruginosa, Klebsiella oxytoca, Serratia marcescens, Klebsiella pneumoniae, Actinomycetaceae actinotignum, Enterococcus faecium Staphylococcus epidermidis, Enterococcus faecalis, Staphylococcus saprophyticus, Staphylococcus aureus, Acinetobacter baumannii, Morganella morganii, Aerococcus urinae, Pantoea aglomerans, Citrobacter Freundii, Providencia stuartii, Citrobacter koseri, Streptococcus anginosus, Trichophyton mentagrophytes complex, Microsporum spp., Trichophyton rubrum, Epidermophyton floccosum, and Trichophyton tonsurans), earl nuclear cells (e.g., protozoa and parasitic animals, fungi, yeast, higher plants, lower animals, and higher animals such as mammals and humans), virus, or biroid. Among the earl nuclear cells, parasite may comprise, for example, Giardia lamblia, Entamoeba histolytica, Cryptosporidium, Blastocystis hominis, Dientamoeba fragilis, Cyclospora cayetanensis, stercoralis, trichiura, hymenolepis, Necator americanus, Enterobius vermicularis, Taenia spp., Ancylostoma duodenale, Ascaris lumbricoides, Enterocytozoon spp./Encephalitozoon spp. The virus may comprise, for example, influenza A virus (flue A), influenza B virus (flu B), Respiratory syncytial virus A (RSV A), Respiratory syncytial virus B (RSV B), Covid-19 virus, Parine Fluenza Virus 1 (PIV 1), Parine Fluenza Virus 2 (PIV 2), Parine Fluenza Virus 3 (PIV 3), Parine Fluenza Virus 4 (PIV 4), metapneumovirus (MPV), human enterovirus (HEV), Human Boca Virus (HBOV), Human Rhinobirus (HRV), Coronavirus and Adenovirus causing respiratory diseases, and Norovirus, Rotavirus, Adenovirus, Astrovirus, and Sapo Virus causing gastrointestinal diseases. As another example, the virus may comprise human papillomavirus (HPV), Middle East respiratory syndrome-related coronavirus (MERS-CoV), Dengue virus, Herpes simplex virus (HSV), Human herpes virus (HHV), Epstein-Barr virus (EMV), Varicella zoster virus (VZV), Cytomegalovirus (CMV), HIV, Parvovirus B19, Parechovirus, Mumps, Dengue virus, Chikungunya virus, Zika virus, West Nile virus, hepatitis virus, and poliovirus. The target analyte may be GBS serotype, Bacterial colony, or v600e. The target analyte in the present disclosure may comprise various analysis targets such as bacteria in addition to the virus, and may also be a specific site of genes cut by using a CRISPR technology, and the target analyte is not limited to the examples.

In the present disclosure, "the biological taxonomy of the organisms" is a biological taxonomy for distinguishing the range to which the organism belongs. For example, a biological taxonomy expressed as Species, Genus, Family, Order, Class, Phylum, Kingdom, and Domain may be found inin "the biological taxonomy of the organisms" in the present disclosure. As an example, the biological taxonomy of the organisms according to an embodiment of the present disclosure may have a hierarchical structure. The hierarchical structure may mean a hierarchical structure which is a biological system structure of a form in which a higher layer encompasses a lower layer.

In the hierarchical structure, the second layer which is the higher layer may encompass all components found in the first layer which is the lower layer. For example, the higher layer for the lower layer including components A, B, and C at least comprises components A, B, and C, and may also comprise an additional component.

The term used herein "sequence identifier" refers to an identifier distinctly indicating a certain nucleic acid sequence (e.g., DNA sequence and RNA sequence) from any other sequences. For example, the sequence identifier comprises an annotated sequence name (e.g., gene name). Examples of annotated sequence names may comprise sequence names annotated in a public-accessible sequence database (e.g., GenBank, EMBL, DDBJ and GSD). For example, the sequence identifier may comprise an arbitrarily assigned sequence identifier. Examples of arbitrarily assigned sequence identifiers may comprise sequence identifiers (IDs) which are assigned to all sequence fragments produced by fragmentation of a full genome sequence. For example, the full genome sequence of Chlamydia trachomatis may be fragmented by a sequence fragmentation algorithm to cleave a genome sequence, if necessary cleave and merge, and all sequence fragments thus produced may be assigned with sequence identifiers. For example, where the sequence fragments comprise 100 fragments, they may be assigned with SEQID1 to SEQID 100.

As one implementation example, a sequence segment may be segmented as a length of the gene. When the gene comprises 27000 sequences, one sequence segment may be a segment including 27000 sequences.

The sequence segment may be a segment configured by a length of any one of 1 sequence or 100 millions of sequences.

Further, the length of the sequence segment may be a length which may comprise a minimum of primary pairs, and the length of the sequence segment, which is not limited thereto, may be implemented as an appropriate length as necessary.

In the present disclosure, "genome" may be used as a meaning of collectively calling genetic information which a specific object (e.g., organism) has. The genome may mean a total base sequence including both the genes and parts that are not the genes. For example, each of all genomes which belong to the specific object may be identified to have a genome ID, and each of the genomes may comprise one or more sequence identifiers.

As another example, the computing device 100 may receive index target data including a genome ID and a plurality of sequence identifiers found in a genome corresponding to the genome ID. The computing device 100 may generate a plurality of tokens by tokening the plurality of sequence identifiers in the received index target data. The computing device 100 may generate an index data structure based on the plurality of generated tokens.

The processor 110 may be constituted by one or more cores and may comprise processors for data analysis and/or processing, which comprise a central processing unit (CPU), a general purpose graphics processing unit (GPGPU), a tensor processing unit (TPU), and the like of the computing device 100. The processor 110 may read a computer program stored in the memory 130 to perform data processing for selecting the sequence identifier and data processing for organizing the index data structure related to the sequence identifier according to an embodiment of the present disclosure.

According to an additional embodiment of the present disclosure, the processor 110 may also perform a computation for learning a neural network. The processor 110 may perform calculations for learning the neural network, which comprise processing of input data for learning in deep learning (DL), extracting a feature in the input data, calculating an error, updating a weight of the neural network using backpropagation, and the like. At least one of the CPU, GPGPU, and TPU of the processor 110 may process learning of a network function. For example, both the CPU and the GPGPU may process the learning of the network function and data classification using the network function. Further, in an embodiment of the present disclosure, processors of a plurality of computing devices may be used together to process the learning of the network function and the data classification using the network function. Further, the computer program executed in the computing device according to an embodiment of the present disclosure may be a CPU, GPGPU, or TPU executable program.

According to an embodiment of the present disclosure, the memory 130 may store any type of information generated or determined by the processor 110 or any type of information received by the network unit.

According to an embodiment of the present disclosure, the memory 130 may mean any type of storage medium, and comprise, for example, at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the memory 130 used in the present disclosure is not limited to the examples.

In the present disclosure, the network unit 150 may be configured regardless of communication modes such as wired and wireless modes and constituted by various communication networks including a personal area network (PAN), a wide area network (WAN), and the like. Further, the network unit 150 may operate based on known World Wide Web (WWW) and may adopt a wireless transmission technology used for short-distance communication, such as infrared data association (IrDA) or Bluetooth.

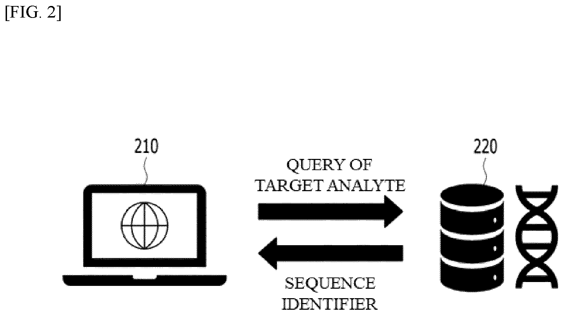

FIG. 2 schematically illustrates a block configuration diagram of a system according to an embodiment of the present disclosure.

The system for implementing embodiments of the present disclosure may be constituted by a user terminal 210 and a server 220. The user terminal 210 and the server 220 may mean a node(s) in a system having a mechanism for communication through the network. Components for the system illustrated in FIG. 2 are just an example, and a system constituted by a plurality of terminals or a plurality of servers may also be found in the scope of the present disclosure.

In the present disclosure, the user terminal 210 and the server 220 are found in the computing device 100 described in FIG. 1. The components illustrated in FIG. 1 may be found in each of the user terminal 210 and the server 220.

Hereinafter, the embodiments of the present disclosure will be described through an example for a process in which the user terminal 210 transmits a query for a target analyte to the server 220, and the server 220 transmits a sequence identifier corresponding to the target analyte to the user terminal 210. However, according to another embodiment, a design in which the user terminal 210 obtains the sequence identifier corresponding to the target analyte may also be found in the scope of the present disclosure.

The user terminal 210 may comprise any type of terminal which is capable of interacting with the server 220. The user terminal 210 may comprise, for example, a cellular phone, a smart phone, a laptop computer, personal digital assistants (PDA), a slate PC, a tablet PC, and an Ultrabook.

The server 220 may perform a process of selecting the sequence identifier and/or a process of generating an index structure according to an embodiment of the present disclosure. The server 220 may comprise, for example, a predetermined type of computing system or computing device such as a microprocessor, a mainframe computer, a digital processor, a portable device, and a device controller.

The server 220 may mean an entity storing and managing base sequence information or gene information. The server 220 may comprise a storage unit (not illustrated) for storing the base sequence information, the gene information, and/or index information, and the storage unit may be found in the server 220 or may be present under the management of the server 220. As another example, the storage unit may also be present outside the server 220, and may be implemented in a form which is capable of communicating with the server. In this case, the storage unit may be managed and controlled by another external server different from the server 220.

As illustrated in FIG. 2, the user terminal 210 may generate a query for a target analyte and/or a classification identifier (e.g., Taxonomy ID (TAX ID)) corresponding to a classification name of a organism such as strain type, species, genus, etc. for example. The server 220 may search and select a sequence identifier specific and sensitive to the target analyte and/or the classification identifier in response to the query. The server 220 may select the sequence identifier specific and sensitive to the input TAX ID.

As an additional embodiment, the user terminal 210 may generate a query including one or more sequence identifiers to be searched. The server 220 may select a specific sequence identifier by computing the number of or a frequency of genomes belonging to the sequence identifier in response to the query. The sequence identifier selected by the server 220 may comprise a sequence identifier specific and sensitive to the target analyte and/or the classification identifier among one or more sequence identifiers found in the query.

In FIG. 2, an embodiment of delivering the query from the user terminal 210 to the server 220 is illustrated, but it will also be apparent to those skilled in the art that an embodiment of searching and selecting the sequence identifier by the user terminal 210 may also be found in the scope of the present disclosure.

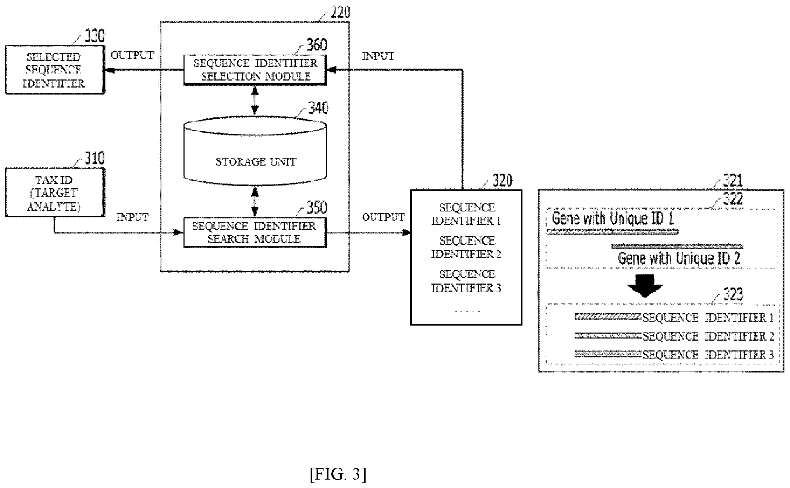

FIG. 3 exemplarily illustrates a conceptual view for a process of selecting a sequence identifier according to an embodiment of the present disclosure.

In FIG. 3, components in the server 220 for performing a function of searching the sequence identifier and selecting the sequence identifier in the server 220 which is one example of the computing device 100 are described.

The storage unit 340 may store and manage any information which may be generated and obtained according to the embodiments of the present disclosure. For example, the storage unit may comprise a genome DB including annotation information to allow search or analysis for the base sequence. In the present disclosure, annotation may mean a process of allocating an identifier to the base sequences found in each genome. As an example, the annotation may comprise a process of allocating a gene ID for the base sequences found in the genome.

As another example, the storage unit 340 may store an index data structure in which inverted-indexing is applied to a data structure in which a plurality of sequence identifiers are mapped to one genome ID. In this case, since a full text search for each of the sequence identifiers is enabled, it may easy to compute the number of genomes to which the sequence identifiers belong.

The storage unit 340 may comprise a database management system (DBMS) and a persistent storage. Here, the DBMS as a program for permitting the server 220 or the terminal 210 to perform predetermined types of operations of the storage unit including search, insertion, modification, and/or deletion of required data, and generating the index and accessing the index may be implemented by the processor of the server 220. The server 220 may generate the index structure according to an embodiment of the present disclosure by accessing the storage unit 340 and controlling the storage unit 340, and support an efficient search for selecting the sequence identifier based on the generated index structure.

The server 220 may receive an input for a target analyte 310 from the terminal 210. In an embodiment, the server 220 may receive an input for a TAX ID 310 from the terminal 210. For example, the TAX ID may comprise a vibrio cholera. In FIG. 3, the TAX ID is used as an example for input data for convenience of description, but any types of inputs related to a target analyte or sequence identifiers to be searched may be found in the input data 310. For example, the input data 310 may comprise any type of information associated with the target analyte such as specific sequence identification information, identification information for a specific organism, identification information for specific species, and/or specific gene ID information. The TAX ID 310 may be an identifier for Taxonomy, a scientific name, nomenclature, and specifier.

The sequence identifier search module 350 may generate a list 320 including one or more sequence identifiers corresponding to the input data such as TAX ID. For example, the sequence identifier search module 350 may obtain sequence identifiers found in reference numeral 323 in response to information of the input target analyte. The sequence identifier search module 350 may retrieve sequence identifiers corresponding to the input TAX ID 310 from the storage unit 340 by searching data stored in the storage unit 340.

In order to search a gene or sequence pattern selected for detecting the diagnostic marker or the target analyte, a mark is required for distinguishing both a gene sequence and a non-gene sequence including a common sequence. In addition to the gene ID which is distinguished as a function unit, when the base sequence is distinguished as the sequence pattern, it is possible to distinguish a sequence pattern which may not be distinguished with the existing gene ID, so it may be possible to search a new sequence portion through grouping the sequence pattern itself.

Differently from an existing annotation scheme of identifying the base sequences based on the gene ID, according to an embodiment of the present disclosure, as illustrated by reference numeral 321, sequence identifiers may be grouped and identified by considering base sequence corresponding to an intergenic region in addition to base sequences corresponding to a genic region.

The sequence identifier may be at least partial region of the genic region and/or at least a partial region of the intergenic region. The intergenic region may be all regions other than the genic region in the genome. As an example, the intergenic region may be misc, repeat, assembly_gap, centromere, gap, and telomere other than the gene in genome information stored in the GenBank of the National Center for Biotechnology Information (NCBI). The intergenic region is not limited to the above-described example.

Further, the intergenic region means a region which may be collected by using Feature Key in the genome information. However, here, some misc_feature may correspond to the genic region. It should be noted that such contents are not limited to the intergenic region term.

Further, as illustrated in reference numeral 322, according to the annotation scheme of identifying the base sequences based on the existing gene ID, there may be a pattern of base sequences overlapped or shared between two gene IDs in spite of a unique gene ID. According to an annotation technique according to an embodiment of the present disclosure, new base sequence identifiers 323 with forms which are unique and clearly distinguished may be generated as compared with gene IDs illustrated in reference numeral 322. As illustrated in reference numeral 323, in a new base sequence identification system according to an embodiment of the present disclosure, three sequence identifiers (sequence identifier 1, sequence identifier 2, and sequence identifier 3) 323 may be formed so that the base sequences do not overlap with each other. Accordingly, since more unique IDs may be allocated to the sequence identifiers according to an embodiment of the present disclosure, a more accurate result may be derived in gene analysis and base sequence search.

According to an embodiment of the present disclosure, the storage unit 340 may store sequence identifiers in a form of sequence identifiers to which a new annotation scheme illustrated in reference numeral 323 is applied. The sequence identifiers may be stored while the TAX ID and the genome information corresponding to the TAX ID are associated with each other. Accordingly, the sequence identifier search module 350 may output the sequence identifiers found in the target analyte in response to an input for the target analyte 310 including the TAX ID.

In FIG. 3, a new scheme of annotating the sequence identifiers is described as an example, but this corresponds to one embodiment, and a scheme of grouping the base sequences based on the gene ID without considering the intergenic region may also be found in the scope of the present disclosure according to the design.

In an embodiment of the present disclosure, the sequence identifier selection module 360 may select a sequence identifier 330 related to the target analyte 310 by using the sequence identifiers 320 output by the sequence identifier search module 350 as an input. The sequence identifier selection module 360 may select the sequence identifier 330 specific to the target analyte 310 among the sequence identifiers 320 found in the target analyte 310 by using the biological taxonomy of the organism related to the target analyte 310. The sequence identifier selection module 360 may select the sequence identifiers specific to the target analyte 310 by utilizing a first layer where the target analyte 310 is positioned, a second layer which is a higher layer than the first layer, and a third layer which is a higher layer than the second layer.

The sequence identifier selection module 360 may quickly select a gene or a sequence pattern (sequence identifier) which has a higher sensitivity to a organism group to which the target analyte 310 belongs and is distinguished from other organism groups.

In FIG. 3, the sequence identifier search module 350 obtaining the sequence identifiers found in the target analyte 310 and the sequence identifier selection module 360 selecting the sequence identifier specific to the target analyte 310 among the obtained sequence identifiers are distinguished and illustrated, but a structure and a process in which one integrated module outputs the sequence identifier 330 specific to the target analyte 310 by receiving the input of the target analyte 310 according to the design may also be available.

In FIG. 3, the storage unit 340 is illustrated as one entity, but a combination of storage units of a form being distinguished like a first storage unit which interacts with the sequence identifier search module 350 and a second storage unit which interacts with the sequence identifier selection module 360 may also be found inin the storage unit 340 in FIG. 3.

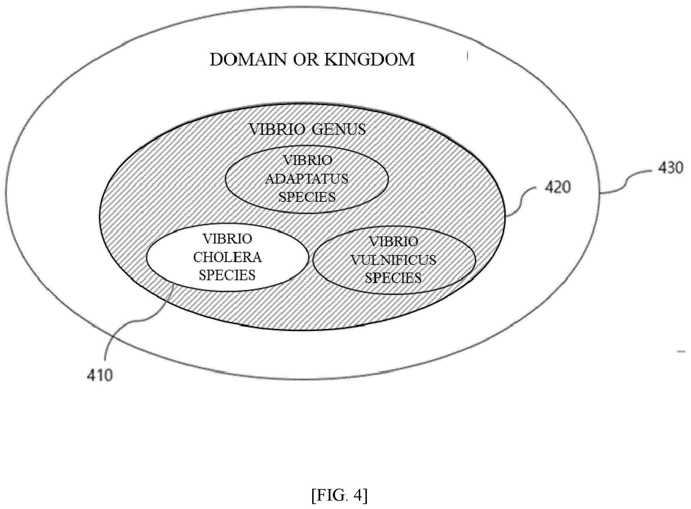

FIG. 4 exemplarily illustrates layers utilized for selecting the sequence identifier in a biological taxonomy of organisms having a hierarchical structure according to an embodiment of the present disclosure.

In FIG. 4, a selection technique of the sequence identifier according to an embodiment of the present disclosure by using vibrio cholera species as an example for the target analyte will be described.

In an embodiment of the present disclosure, "layer" represents classification on the biological taxonomy of the organisms. An N-th layer is used for representing a layer according to the classification on the biological taxonomy of the organisms, and N is a natural number. Here, a larger value of N corresponds to the higher layer.

Specifically, the biological taxonomy of the organisms may be defined as species, genus, family, order, class, phylum, kingdom, and/or domain. As an example, a first layer 410, a second layer 420, and the third layer 430 may be present, which correspond to any one of species, genus, family, order, class, phylum, kingdom, and/or domain the biological taxonomy of the organisms. Each of the first layer 410, the second layer 420, and the third layer 430 means a position of the layer. The second layer 420 means a layer positioned at a higher position than the first layer 410, and the third layer 430 means a layer positioned at a higher position than the second layer 420.

Further, the N-th layer may comprise lower layers of the N-th layer on the biological taxonomy of the organisms. That is, the higher layer on the biological taxonomy of the organism may have a hierarchical structure of a form of encompassing the lower layers. As an example, the second layer 420 may comprise the first layer as the higher layer of the first layer, and the third layer may comprise the first layer 410 and the second layer 420 as the higher layers of the first layer 410 and the second layer 420.

In an embodiment of the present disclosure, the first layer 410 may be a layer corresponding to species, the second layer 420 may be a layer corresponding to genus, and the third layer 430 may be a layer corresponding to domain or kingdom. Here, the first layer 410 may be defined by the layer on which the target analyte is positioned, the second layer 420 may be defined by a higher layer positioned closest to the first layer 410 among the higher layers of the first layer 410, and the third layer 430 may be defined by a highest layer in the biological taxonomy of the organism. The layer may be defined differently for each embodiment.

In an embodiment of the present disclosure, the layer is defined by a name of the target analyte (taxonomy ID). As an example, when the target analyte is vibrio cholera species (vibrio cholerae), the computing device 100 may determine a layer to which vibrio cholera belongs as "vibrio cholerae species" in response to the input of the target analyte (e.g., vibrio cholera). The computing device 100 may determine a layer corresponding to vibrio species 1 as the first layer 410. As an example, when the first layer is "species", species other than vibrio cholerae does not correspond to the first layer. The first layer may mean a biological taxonomy of organisms other than other "species" among all organism biological taxonomys such as Neisseria gonorrhoeae and Escherichia coli different from vibrio cholera in terms of species. That is, the first layer 410 may mean a specific layer in the biological taxonomy of the organisms at which the target analyte is positioned.

In another embodiment, it may also be defined which layer the layer is by the name of the target analyte (taxonomy ID). As an example, when the target analyte is vibrio cholera species (vibrio cholerae), the computing device 100 may determine the layer as "all organism species" in response to the input of the target analyte (e.g., vibrio cholera). That is, when the first layer is "species", the first layer may also mean all species corresponding to "species" among all organism biological taxonomys such as Neisseria gonorrhoeae and Escherichia coli different from vibrio cholera in terms of species in addition to vibrio cholerae species. Further, similarly to the second layer and the third layer to be described below, the second layer and the third layer may also mean all layers which positioned at an equivalent position on the biological taxonomy of the organisms. That is, the first layer 410 may mean a general layer in the biological taxonomy of the organisms at which the target analyte is positioned.

Hereinafter, in the present disclosure, for convenience of description, it will be described that the first layer 410 means a specific layer in the biological taxonomy of the organisms at which the target analyte is positioned as defined in an embodiment.

When the target analyte is vibrio cholerae species, the first layer may correspond to vibrio cholerae species (or including the lower layer when there is the lower layer). In this case, it is assumed that vibrio cholerae corresponds to one of a plurality of species which belong to vibrio genus.

In this case, the second layer may correspond to vibrio genus which is the higher layer than the first layer. The second layer may comprise a lower layer (e.g., the first layer) which belongs to vibrio genus. In this case, the second layer may comprise all species (e.g., Vibrio adaptatus species, Vibrio cholerae species, Vibrio vulnificus species, etc.) found in vibrio genus.

The third layer may correspond to bacteria kingdom which is the higher layer than the second layer. The third layer may comprise lower layers (e.g., all phylums, all classes, all orders, all families, all genuses, and all species found in bacteria kingdom) which belong to bacteria kingdom.

In the above-described embodiment, the first layer, the second layer, and the third layer may be terms which encompass the lower layers of the corresponding layer. As an example, when the first layer is species, the first layer may be a term that encompasses subspecies which belongs to the specifies of the first layer, and when the second layer is genus, the second layer may be a term that encompasses species which belong to the second layer. Further, when the third layer is kingdom, the third layer may be a term that encompasses phylum or division which belongs to the third layer.

In an embodiment different from the above-described embodiment, the first layer, the second layer, and the third layer may be terms which encompass lower layers found in the corresponding layer, and different layers having the same height as the corresponding layer in addition to the lower layers. That is, the first layer may be a term which encompasses the lower layers found in the first layer, and all different layers positioned at the same height on the biological taxonomy of the organisms in addition to the lower layers. In the embodiment, the N-th layer may be described as a concept including a layer having a horizontal relationship with the N-th layer jointly, and in the above-described embodiment, the N-th layer may be described as a concept other than the layer having the horizontal relationship with the N-th layer.

As an example, when the first layer is species, the first layer may be a term that encompasses subspecies found in the specifies of the first layer, and other species found in genus which belong to the first layer in the biological taxonomy of the organisms in addition to subspecies.

When the second layer is genus, the second layer may be a term that encompasses species found in the genus of the second layer, and other genuses found in family to which the second layer belongs in the biological taxonomy of the organisms in addition to species.

When the third layer is kingdom, the third layer may be a term that encompasses phylums or divisions found in the family of the third layer, and other kingdoms found in domain to which the third layer belongs in the biological taxonomy of the organisms in addition to phylums or divisions.

In yet another embodiment different from the above-described embodiment, the first layer may be a term that encompasses the lower layers found in the corresponding layer, and the second layer and the third layer may be terms which encompass lower layers found in the corresponding layer, and different layers having the same height as the corresponding layer. The embodiment may be described as an embodiment in which contents described in another embodiment different from the above-described embodiment are combined.

In yet another embodiment different from the above-described embodiment, the first layer and the second layer may be terms that encompass the lower layers found in the corresponding layer, and the third layer may be a term which encompasses lower layers found in the corresponding layer, and different layers having the same height as the corresponding layer. The embodiment may be described as an embodiment in which contents described in another embodiment different from the above-described embodiment are combined.

In a first embodiment of the present disclosure, the second layer may be a higher layer of the first layer, and the third layer may be a highest layer according to the embodiment. For example, in the first embodiment, the second layer may be a layer corresponding to immediately higher hierarchical position of the first layer in the hierarchical structure, and the third layer may be a second highest layer or the highest layer in the hierarchical structure.

In a second embodiment of the present disclosure, the second layer may be the higher layer of the first layer, and the second layer may be the highest layer according to the embodiment. For example, in the second embodiment, the second layer may be the layer corresponding to immediately higher hierarchical position, the second highest layer, or the highest layer of the first layer in the hierarchical structure.

As such, various embodiments including the first embodiment and the second embodiment are merged in the present disclosure, and the layer may be applied differently according to the embodiment.

Hereinafter, the embodiment will be described based on the terms of the layer described in the above-described embodiment, and it should be noted that the terms of the layers described in other embodiments may be applied to the embodiments throughout the present disclosure.

The computing device 100 may obtain the sequence identifiers found in the genomes which belong to the first layer 410. The computing device 100 may obtain the sequence identifiers found in the genomes which vibrio cholera corresponding to the first layer 410 has. Data for the sequence identifier for identifying the base sequences which each genome has in the genomes found in the first layer 410 may be obtained.

Each genome may comprise sequence identifiers for identifying one or more base sequences. For example, genome 1 may comprise sequence identifier A having a pattern of AAGGCTTT, sequence identifier B having a pattern of GCTTAAACC, and sequence identifier C having a pattern of CCTCCTATTTTTCCAA. Genome 2 may comprise sequence identifier A, and sequence identifier D having a pattern of CAAATGGCTGCCCA. Further, each sequence identifier may correspond to one or more genomes. For example, sequence identifier A may be found in genome 1, genome 2, and genome 3, and sequence identifier B may be found in genome 1, genome 3, genome 4, and genome 5.

The computing device 100 may compute the number of genomes of the first layer 410 to which each of the sequence identifiers found in the genomes which exist in the first layer 410 belongs. For example, identifier A may belong to a total of 100 among 100 genomes in the first layer 410, identifier B may belong to a total of 97 among 100 genomes in the first layer 410, and identifier C may belong to a total of 65 among 100 genomes in the first layer 410. As such, the computing device 100 filters the sequence identifiers found in the genomes found in the first layer 410 according to the computation result to select the filtered sequence identifiers in the genomes found in the first layer 410. For example, the filtered sequence identifiers in the genomes found in the first layer 410 may mean a list of sequence identifiers which are highly likely to exist in the genomes found in the first layer 410.

In an embodiment of the present disclosure, "a layer" may be used for representing ranges of genomes positioned on the corresponding layer. Specifically, "the layer" may mean a list of all genomes which belong to the corresponding layer or lowers layer found in the corresponding layer. As described above, since the layer is defined by the name of the target analyte, the layer is also be defined by the name of the target analyte.

For example, when the target analyte is vibrio cholerae species, the first layer becomes vibrio cholerae species. When the firs layer is vibrio cholerae species, the first layer may mean a list of genomes (organisms) which belong to vibrio cholerae species. In this case, the genomes may be a plurality of whole genomes. In vibrio cholerae species, there may be various genomes due to a cause such as polymorphism or genetic variation of the genes. That is, in vibrio cholerae species, there may be countless objects, and respective objects may be formed as the same genome, or even though the respective objects are the same species, the respective objects may be formed as genomes of which some are different.

When the second layer is vibrio genus, the second layer may mean a list of all genomes found in vibrio genus. Specifically, the second layer may mean all genomes (e.g., Vibrio cholerae species, Vibrio adaptatus species, Vibrio vulnificus species, etc.) found in all vibrio species which belong to vibrio genus, respectively.

When the third layer is bacteria kingdom, the third layer may mean all genomes found in bacteria kingdom. Specifically, the third layer may mean genomes found in all species, all genuses, all families, all orders, all classes, and all phlyums which belong to bacteria kingdom.

The computing device 100 may compute the number or a frequency of sequence identifiers which are present in the genomes found in the first layer 410, which are present in the first layer 410. The computing device 100 may obtain an occurring frequency of the first sequence identifiers in the first layer by using the number of genomes to which the first sequence identifiers belong among the first genomes found in the first layer. Specifically, the computing device 100 may obtain the occurring frequency of the first sequence identifiers in the first layer 410 by a scheme of computing how many genomes among the genomes found in the first layer the respective first sequence identifiers belong to.

The computing device 100 may compute the number of genomes in the second layer 420 to which each of the sequence identifiers belongs with respect to each sequence identifier by a scheme corresponding to the computation scheme used in relation to the first layer 410. The computing device 100 may obtain an occurring frequency of the first sequence identifiers in the second layer by using the number of genomes to which the first sequence identifiers belong among the second genomes found in the second layer. Specifically, the computing device 100 may obtain the occurring frequency of the first sequence identifiers in the second layer 420 by a scheme of computing how many genomes among the genomes found in the second layer 420 the respective first sequence identifiers found in the second layer 420 belong to.

In an embodiment, the computing device 100 may obtain the occurring frequency for the first sequence identifiers within which the first genomes found in the first layer are excluded among the second genomes found in the second layer. That is, the computing device 100 may obtain the occurring frequency for the first sequence identifiers within which the first genomes are excluded among the second genomes. As an example, the computing device 100 may obtain the occurring frequency for the first sequence identifiers within (a hatched region in reference numeral 420 of FIG. 4) in which the first layer 410 to which the first target analyte 310 belongs is excluded from the second layer 420. As a similar embodiment, the computing device 100 may obtain the occurring frequency for the first sequence identifiers within a difference set the first layer 410 to which the first target analyte 310 belongs for the second layer 420.

A range in which the first genomes found in the first layer 410 are excluded among the second genomes found in the second layer 420 may mean a list of genomes in which genomes comprise in vibrio cholerae species are excluded among the genomes found in vibrio genus. In which the first genomes found in the first layer 410 are excluded among the second genomes found in the second layer 420 may mean a difference set of genomes which belong to vibrio cholerae species for genomes which belong to vibrio genus.

As an example, when there are a total of 1100 genomes in the second layer 420, and there are a total of 100 genomes in the first layer 410, a range acquired by excluding the first layer 410 from the second layer 420 may mean a list of a total of 1000 genomes after subtracting 100 from 1100.

According to an embodiment of the present disclosure, among the sequence identifiers which are in the first layer 410, sequence identifiers which exist a lot in the genomes found in the first layer 410 and exist a few in the genomes found in the second layer 420 may be primarily selected.

As such, the computing device 100 may perform a filtering operation considering both the first layer 410 and the second layer 420 with respect to the sequence identifiers found inin the first layer 410.

As described above, when the sequence identifiers are filtered by considering both the first layer 410 and the second layer 420, sequence identifiers may be selected, which are highly likely to be found in genomes which vibrio cholerae has, but is less likely to be found in genomes which the remaining species other than vibrio cholerae in vibrio genus have. In FIG. 4, the identifiers selected by such a scheme will be referred to as a primarily filtered identifier.

The computing device 100 may apply a filtering operation (i.e., a secondary filtering operation) considering the third layer 430 jointly with respect to the primary filtered identifiers. The third layer 430 may correspond to domain which belongs to the highest layer or kingdom which belongs to the second highest layer in a biological taxonomy to which vibrio cholerae species belong. The computing device 100 may select, as a secondarily filtered identifier, identifiers which exist a lot in the first layer 410 and which exist a few in the third layer 430 among the primary filtered identifiers.

The number of sequence identifiers found in the first layer 410 may be primarily reduced through primary filtering considering the first layer 410 and the second layer 420. Furthermore, by applying the secondary filtering operation additionally considering the third layer 430 for the sequence identifiers of which the number is reduced through the primary filtering operation, one or more sequence identifiers which are highly likely to be specific to the target analyte may be finally selected among the sequence identifiers found in the first layer 410.

The primary filtering related operation and the secondary filtering related operation may require a calculation amount at a level which may be performed even by a performance of a personal computer. In the primary filtering operation, an occurring frequency in a organism group to which the target analyte belongs and a higher organism group thereof is calculated, and sequence identifiers which are not appropriate to be selected as the target analyte may be pre-emptively selected and excluded through a primary filtering result, so the number of sequence identifiers to be searched in a whole organism group may be reduced. Since an occurring frequency for all highest-layer organism groups of the organism group to which the target analyte belongs based on the primarily filtered sequence identifiers may be calculated in a secondary filtering operation step, a search result specific to the target analyte may be derived by a smaller calculation amount than a scheme of performing the search in a whole the organism group without primary filtering.

There may be various schemes in relation to selection of sequence identifiers (i.e., sequence identifiers specific to a target organism group) which are comprised a lot in the target organism group and which are not almost comprised (or not comprised at all) in other organism groups other than the target organism group.

For example, it is assumed that the total number of genomes which the organism group (i.e., vibrio cholerae species) to which the target analyte belongs has is 100, and the total number of sequence identifiers found in 100 genomes is 1000. Further, it is assumed that the total number of genomes which the higher organism group (i.e., vibrio genus) of the organism group to which the target analyte belongs has is 500, and the total number of sequence identifiers found in 500 genomes is 5000. Further, it is assumed that the total number of genomes which the highest organism group (i.e., bacteria domain or bacteria kingdom) of the organism group to which the target analyte belongs has is 10000, and the total number of sequence identifiers found in 10000 genomes is 100000.

A first scheme is a scheme of selecting a sequence identifier specific to the target organism group within the whole organism group. The first scheme is a scheme of selecting sequence identifiers which a lot of genomes have in the target organism group and a few genomes have in all organism groups. In such a first scheme, all of 1000 sequence identifiers found in 100 genomes of the organism group to which the target analyte belongs are obtained, and then an operation related to which genomes among 100 genomes within vibrio cholerae species each of a total of 1000 sequence identifiers being found in and a frequency thereof are first required. In such an operation, an operation of a frequency at which each of 1000 sequence identifiers belongs to 100 genomes (an operation amount of 1000 X 100) will be performed. Through this, relatively many sequence identifiers found in 100 genomes may be selected in the target organism group.

Then, an operation related to which genomes among 10000 genomes within a domain or a kingdom which is a highest biological taxonomy in organism classification each of a total of 1000 sequence identifiers being found in and a frequency thereof is additionally required. In such an operation, 1000 sequence identifiers and 10000 genomes will be compared. In a first scheme, it may be confirmed that two operations are used, but a calculation amount for a second operation is significant (i.e., an operation amount of 1000 X 10000). Through this, relatively less sequence identifiers found in 100 genomes may be selected in groups other than the target organism group.

In a second scheme as a scheme according to an embodiment of the present disclosure, the primary filtering operation and the secondary filtering operation are sequentially applied to select a sequence identifier specific to the target organism group. In such a second scheme, an operation related to which genomes among 100 genomes within vibrio species 1 each of a total of 1000 sequence identifiers being found in and a frequency thereof is first required. Further, in the second scheme, a primary filtering technique is used, which selects sequence identifiers which a lot of genomes have in the target organism group and a few genomes have in the higher organism group of the target organism group have. In the primary filtering technique, an operation related to which genomes among 500 genomes within vibrio genus each of a total of 1000 sequence identifiers being found in and a frequency thereof is required. As an additional embodiment, the operation may also be performed for 400 genomes acquired by excluding 100 genomes within vibrio species 1 from 500 genomes within the vibrio genus.

Through the primary filtering operation, sequence identifiers may be selected, which a lot of genomes have in the target organism group and a few genomes have in the higher organism group of the target organism group among a total of 1000 sequence identifiers. In the above-described assumption, it may be assumed that 980 sequence identifiers which are 98% of 1000 sequence identifiers are excluded through the primary filtering operation.

An operation related to whether each of 20 sequence identifiers selected through the primary filtering operation being found in 10000 genomes within the domain, the kingdom, or the whole range is additionally performed. In such an operation, 20 sequence identifiers and 10000 genomes will be compared, and through such a comparison, the secondary filtered sequence identifiers may be finally selected. In a second scheme, three operations are used, but it may be confirmed that a calculation amount for a last operation occupying a considerable calculation amount among the total calculation amount is rapidly reduced compared to the first scheme (i.e., an operation amount of 20 X 10000). As such, since the number of sequence identifiers to be compared with the genomes is reduced through the primary filtering, the operation amount in a last operation step of comparing all organism groups having a relatively many genomes and the sequence identifiers may be remarkably reduced.

As an additional embodiment, the operation may also be performed for 9900 genomes acquired by excluding 100 genomes within vibrio species 1 from 10000 genomes within the domain, the kingdom, or the whole range. As another additional embodiment, the operation may also be performed for 9500 genomes acquired by excluding 500 genomes within vibrio genus from 10000 genomes within the domain, the kingdom, or the whole range.

As such, according to an embodiment of the present disclosure, the complexity of the operation may be significantly reduced in selecting the sequence identifiers which are comprised specifically a lot in the organism group to which the target analyte belongs and which are comprised a few in other organism groups other than the organism group to which the target analyte belongs. In an embodiment of the present disclosure, since the sequence identifier specific to the target analyte may be selected by the efficient scheme, a utilization possibility may be increased in gene analysis, primary design, and/or comparison between base sequences in an in vitro diagnosis (IVD) field (in particular, a molecular diagnosis field). In the present disclosure, the expression "being specific" means that there is a high possibility that the sequence identifier will react only to a specific organism group.

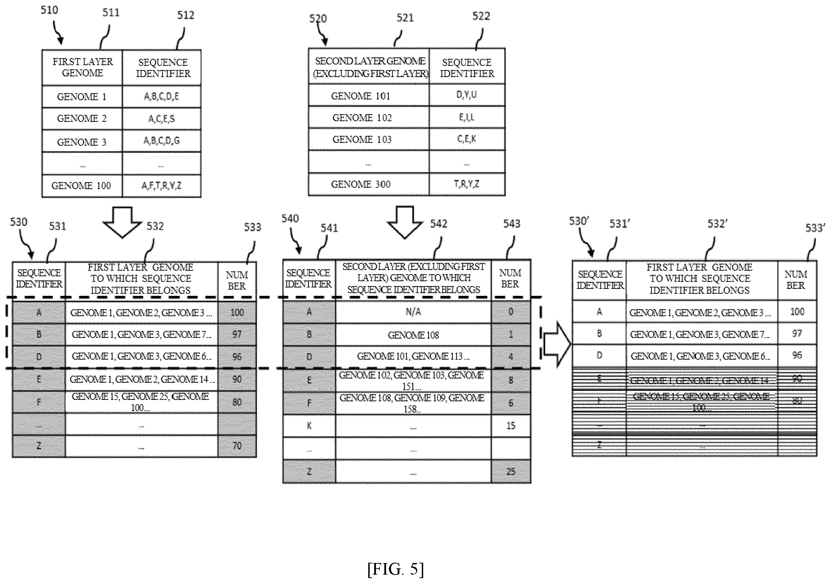

FIG. 5 illustrates an exemplary data structure used for computing an occurring frequency of sequence identifiers by considering a first layer and a second layer.

According to an embodiment of the present disclosure, the storage unit of the computing device may store a mapping relationship between the genome and the sequence identifier as exemplary table structures represented by reference numerals 510 and 520. In the present disclosure, the sequence ID may be used interchangeably with the sequence identifier.

As illustrated in FIG. 5, a table 510 representing a mapping relationship between a genome and a sequence identifier (i.e., a first sequence identifier) in the first layer is illustrated. The table 510 may comprise a column 511 representing a list of genomes and a column 512 representing a sequence identifier corresponding to the genome. The list of genomes which exist in the first layer may be found in the column corresponding to reference numeral 511. Further, the sequence identifier (i.e., first sequence identifier) found in each of the genomes in the first layer may be found in the column corresponding to reference numeral 512. As illustrated in FIG. 5, genome 1 of the table 510 may comprise sequence identifier A, sequence identifier B, sequence identifier C, sequence identifier D, and sequence identifier E.

Further, a table 520 representing a mapping relationship between a genome and a sequence identifier (i.e., a sequence identifier) in the second layer is illustrated. The table 520 may comprise a column 521 representing a list of genomes in the second layer and a column 522 representing a sequence identifier corresponding to the genome. The list of genomes which exist in the second layer may be found in the column corresponding to reference numeral 521. Further, the sequence identifier found in each of the genomes in the second layer may be found in the column corresponding to reference numeral 522. As illustrated in FIG. 5, genome 101 of the table 520 may comprise sequence identifier D, sequence identifier Y, and sequence identifier U.

In FIG. 5, it is illustrated that a separate table type data structure exists for each layer in the biological taxonomy of the organisms, but this is just an example for convenience of description, and according to an implement scheme or a design scheme, any type of data structure in which genomes and a sequence identifier found in a specific layer are enabled to be connected may be available, such as a data structure of managing the whole biological taxonomy as one table, a data structure in which a plurality of tables exists for one layer, or a graph type data structure constituted by nodes or edges.

Further, it is represented that the genomes in the first layer are excluded in order to express the genomes found inin the second layer as an example in the column 521 of the table 520 in FIG. 5. However, according to an aspect of implementation or application, in an additional embodiment of the present disclosure, the list of genomes found in the column 521 may also be found in all genomes found in the second layer (i.e., genomes from which the genomes in the first layer are not excluded). In this case, a table including the genomes from which the genomes in the first layer are not excluded in the second layer may be constructed in advance. The computing device may obtain the table 520 in a scheme in which the target analyte is input and the first layer is defined, and then the genomes found in the first layer are excluded. For example, the computing device may obtain the table 520 in which the first layer is excluded from the table of the second layer constructed in advance when the first layer is defined.

The computing device may obtain the list of genomes found in the first layer to which the target analyte belongs, and a first sequence identifier corresponding to each genome. The computing device may compute an occurring frequency in the first layer with respect to each of the first sequence identifiers.

The table 530 in FIG. 5 is a data structure for computing the number of genomes to which each of the first sequence identifiers found inin the first layer belongs. The table 530 may comprise a first column 531 including each of the sequence identifiers which belong to the first layer, a second column 532 representing a list of genomes to which each sequence identifier belongs, and a third column 533 representing the number of genomes to which each sequence identifier belongs. As an example, the table 530 may mean an index table in which an inverted index is applied to the table 510. As another example, the table 530 may also mean a pivot table for the table 510. As yet another example, the table 530 may also be a basic table representing the sequence identifier, the genomes in the first layer, and the number of genomes to which the sequence identifier belongs.

The computing device may identify the number of genomes in the first layer to which each of the sequence identifiers found in the first layer belongs by utilizing the table 530. Hereinafter, a scheme of computing the occurring frequency according to the example of FIG. 5 is illustrated as an example. In the example of FIG. 5, a sequence identifier annotated with A (i.e., sequence identifier A) may be a sequence identifier found in a total of 100 genomes among the genomes which belong to the first layer, a sequence identifier annotated with B (i.e., sequence identifier B) may be a sequence identifier found in a total of 97 genomes among the genomes which belong to the first layer, a sequence identifier annotated with D (i.e., sequence identifier D) may be a sequence identifier found in a total of 96 genomes among the genomes which belong to the first layer, a sequence identifier annotated with E (i.e., sequence identifier E) may be a sequence identifier found in a total of 90 genomes among the genomes which belong to the first layer, a sequence identifier F may be a sequence identifier found in a total of 80 genomes among the genomes which belong to the first layer, and a sequence identifier Z may be a sequence identifier found in a total of 70 genomes among the genomes which belong to the first layer. As illustrated in Table 510, the total number of genomes of the first layer is illustrated as 100.

The computing device may obtain the occurring frequency by computing a ratio of an appearance number of sequence identifiers corresponding to a specific sequence identifier for 100 which is the total number of genomes found in the first layer. In the example of FIG. 5, the sequence identifier annotated with A has an occurring frequency of 1.00 as 100/100, the sequence identifier annotated with B has an occurring frequency of 0.97 as 97/100, the sequence identifier annotated with D has an occurring frequency of 0.96 as 96/100, the sequence identifier annotated with E has an occurring frequency of 0.9 as 90/100, the sequence identifier annotated with F has an occurring frequency of 0.8 as 80/100, and the sequence identifier annotated with Z has an occurring frequency of 0.7 as 70/100.

In an embodiment of the present disclosure, the occurring frequency for each of the sequence identifiers in the first layer may be computed, and by comparing the computed occurring frequency and a first threshold, it may be considered that sequence identifiers having an occurring frequency less than the first threshold are excluded from a sequence identifier(s) to be finally selected. For example, when the first threshold is set to 0.9, E, F, and Z among the sequence identifiers in the example of FIG. 5 may be filtered and/or excluded according to the comparison with the first threshold. Here, the first threshold may mean a minimum reference value of the occurring frequency of the sequence identifiers in the first layer. When an occurring frequency for a specific sequence identifier does not satisfy the first threshold, the specific sequence identifier does not satisfy a minimum reference for the occurring frequency, so the specific sequence identifier corresponds to the sequence identifier excluded from the sequence identifier to be finally selected. The sequence identifier which satisfies the first threshold in the first layer may be considered as a sequence identifier which belongs to a lot of genomes, and a sequence identifier which does not satisfy a second threshold in the first layer may be considered as a sequence identifier which belongs to are relatively a few genomes.

The table 540 in FIG. 5 is a data structure for computing the number of genomes to which each of the sequence identifiers found inin the second layer belongs. As an example, the sequence identifiers found in the first layer and the sequence identifiers found inin the second layer may be partially different, and in the example of FIG. 5, it is illustrated that sequence identifier K is not found in the genomes in the first layer, but found in the genomes of the second layer.

According to an additional embodiment of the present disclosure, sequence identifiers found in a column 541 of the table 540 may also be enabled to be implemented as a form representing the sequence identifiers found inin the first layer or the sequence identifiers found in the second layer including the first layer according to an aspect of implementation or application. Further, according to an additional embodiment of the present disclosure, genomes found in a column 542 of the table 540 may also be enabled to be implemented as a form representing the genomes found in the second layer including the first layer.

Referring to FIG. 5, the table 540 may comprise a first column 541 including each of the sequence identifiers which belong to the second layer, a second column 542 representing a list of genomes in the second layer to which each sequence identifier belongs, and a third column 543 representing the number of genomes to which each sequence identifier belongs. As an example, the table 540 may mean an index table in which an inverted index is applied to the table 520. As another example, the table 540 may also mean a pivot table for the table 520. As yet another example, the table 540 may also be a basic table representing the sequence identifier, the genomes, and the number of genomes to which the sequence identifier belongs.

According to an embodiment of the present disclosure, as described above, the sequence identifier found in the column 541 in the table 540 may mean the sequence identifier found inin the second layer. According to an additional embodiment of the present disclosure, the sequence identifier found in the column 541 in the table 540 may also be enabled to be implemented as a form representing the sequence identifier found in the first layer.

By comparing the genomes in the second layer and the sequence identifier in the second layer, the number or a frequency of respective sequence identifiers in the second layer, which are found in the genomes in the second layer may be computed. The number (or frequency) computed as such may be expressed as a form of a column 543 of the table 540.

The computing device may obtain the number of genomes to which each sequence identifier found inin the first layer belongs among the genomes in the second layer by utilizing the table 540. In the example of the table 540 illustrated in FIG. 5, a sequence identifier annotated with A (i.e., sequence identifier A) may be a sequence identifier not found in the genomes which belong to the second layer, a sequence identifier annotated with B (i.e., sequence identifier B) may be a sequence identifier found in a total of 1 genome (e.g., genome 108) among the genomes which belong to the second layer, a sequence identifier annotated with D (i.e., sequence identifier D) may be a sequence identifier found in a total of 4 genomes among the genomes which belong to the second layer, a sequence identifier annotated with E (i.e., sequence identifier E) may be a sequence identifier found in a total of 8 genomes among the genomes which belong to the second layer, a sequence identifier F may be a sequence identifier found in a total of 6 genomes among the genomes which belong to the second layer, a sequence identifier K may be a sequence identifier found in a total of 15 genomes among the genomes which belong to the second layer, and a sequence identifier Z may be a sequence identifier found in a total of 25 genomes among the genomes which belong to the second layer.

In an embodiment of the present disclosure, shaded parts in the table 530 and the table 540 are used for expressing that data used for computing the occurring frequency in the table 540 is limited to the sequence identifiers found in the table 530. In an additional embodiment, shaded items in the table 530 and the table 540 which represent sequence identifiers of which occurring frequency is to be computed among the sequence identifiers which belong to the second layer may correspond to the sequence identifiers which belong to the first layer.

As illustrated in Table 520, a total number of genomes found inin the second layer is illustrated as 200. According to an embodiment of the present disclosure, the second layer may mean in which the first layer is excluded from the higher layer of the first layer. For example, the second layer may mean a remaining range in which the specifies corresponding to the first layer corresponding to the target analyte is excluded from the genus range in the biological taxonomy of the organisms. As illustrated in FIG. 5, the second layer which is the higher layer of the first layer may comprise the first layer and other lower layers which exist parallel to the first layer. In the example of FIG. 5, a total of 300 genomes may exist in the second layer, and the second layer may correspond to 200 genomes acquired by subtracting 100 genomes which belong to the first layer from 300 genomes.

In FIG. 5, it is illustrated that the second layer means a difference set or a complementary set of the first layer for the second layer, but according to an additional embodiment of the present disclosure, the second layer may also be all genomes which the higher layer of the first layer has. In such an example, the number of genomes which belong to the second layer comprises genome 1 to genome 300, so the total number may be 300.

As an additional embodiment, the second layer is illustrated as a higher layer adjacent to the first layer, but according to the implementation scheme or design scheme, it may also be considered that the first layer is a species layer, and the second layer is a family or order layer. In such an example, the second layer may mean genomes in which the first layer (species layer) is excluded from the family or order layer.

The computing device may obtain the occurring frequency by computing a ratio of an appearance number of sequence identifier of a specific identifier for 200 which is the total number of genomes found in the second layer. The occurring frequency may represent how many the respective sequence identifiers found inin the second layer are found in the genomes which belong to the second layer. Hereinafter, a scheme of computing the occurring frequency according to the example of the table 540 of FIG. 5 is illustrated as an example. In the example of the table 540 of FIG. 5, the sequence identifier annotated with A has an occurring frequency of 0 as 0/200, the sequence identifier annotated with B has an occurring frequency of 0.005 as 1/200, the sequence identifier annotated with D has an occurring frequency of 0.02 as 4/200, the sequence identifier annotated with E has an occurring frequency of 0.04 as 8/200, the sequence identifier annotated with F has an occurring frequency of 0.03 as 6/200, the sequence identifier annotated with K has an occurring frequency of 0.075 as 15/200, and the sequence identifier annotated with Z has an occurring frequency of 0.125 as 25/200.

By comparing the computed occurring frequency and the second threshold, it may be considered that sequence identifiers having an occurring frequency which exceeds the second threshold are excluded from the sequence identifier(s) to be finally selected. For example, when the second threshold is set to 0.05, sequence identifiers E, F, K, and Z among the sequence identifiers in the example of FIG. 5 may be excluded according to the comparison with the second threshold. Here, the second threshold may mean a maximum reference value of the occurring frequency of the sequence identifiers in the second layer. That is, the sequence identifier in the first layer which belongs to a lot of genomes in the second layer may be considered as a sequence identifier having a high occurring frequency, and the sequence identifier in the first layer which belongs to relatively a few genomes in the second layer may be considered as a sequence identifier having a low occurring frequency. Calculating the occurring frequency of the sequence identifier of the second layer range in the second layer range is to find sequence identifiers which do not exist in the second layer range or exist only a few in the second layer range.

As described above, the occurring frequency in the first layer for each of the sequence identifiers in the first layer and the occurring frequency in the second layer for each of the sequence identifiers in the second layer may be computed. The occurring frequency in the first layer may be compared with the first threshold, and the occurring frequency in the second layer may be compared with the second threshold.

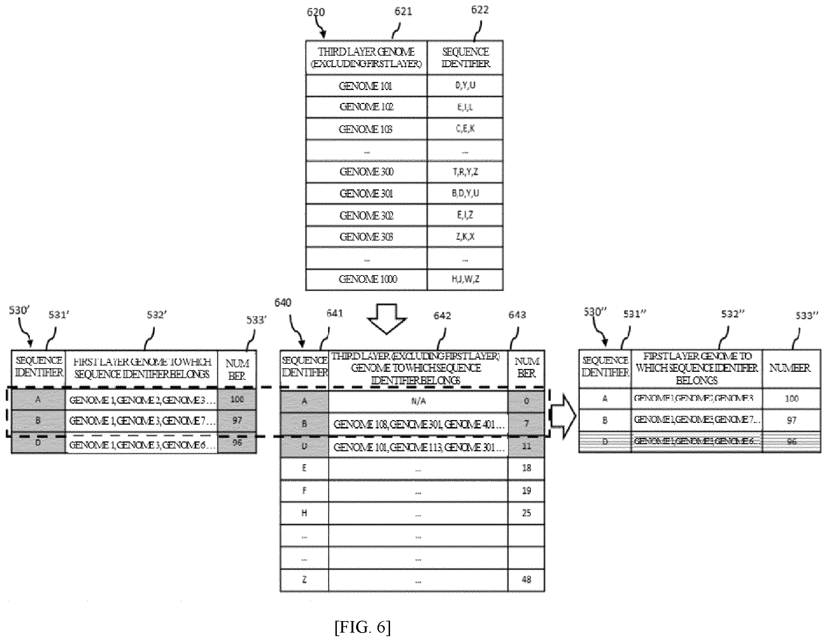

In an embodiment, a sequence identifier which satisfies a reference with the first threshold and satisfies a reference with the second threshold may be selected as the second sequence identifier in the operation step in FIG. 5. As some of the first sequence identifiers which belong to the first layer are filtered through the filtering operation in FIG. 5, some first sequence identifiers which satisfy the reference may be selected as the second sequence identifiers. Second sequence identifiers that pass through the filtering operation in FIG. 5 may become targets of a filtering operation in FIG. 6 to be described below.

A table 530' of FIG. 5 represents a table in which the occurring frequency related to the table 540 is reflected to the table 530. The occurring frequency related to the table 540 may be computed, and according to a result of comparison with the threshold, sequence identifiers E to Z may be considered as sequence identifiers which do not satisfy a reference value in the table 530. Shaded items in the table 530' are used for illustrating that sequence identifiers which do not satisfy the reference value are excluded.

In an embodiment of the present disclosure, the table 530' is not a separately generated table, but is used for expressing that some data is selected in the table 530.

In another embodiment of the present disclosure, the table 530' may exist as a separate object from the table 530.

In yet another embodiment of the present disclosure, the table 530' may exist as the same table as the table 530, and in this case, the table 530' may represent a form in which any type of data manipulation language (DML) operation including modification, insertion, and/or deletion is applied to the table 530.

In yet another embodiment of the present disclosure, the table 530' may also exist as a form of a database view or materialized view having the table 530 as the basic table. In this case, when changed contents for data found in the table 530 are generated, data in the table 530' may be refreshed and the changed contents may be reflected to the data in the table 530'.

The table 530' of FIG. 5 may comprise a first column 531' representing the sequence identifiers found inin the first layer, a second column 532' representing the genomes found inin the first layer to which the sequence identifiers belong, and a third column 533' representing the number of genomes in the first layer to which each of the sequence identifiers found inin the first layer belongs. As illustrated in FIG. 5, as the first threshold is applied to the data found in the table 530 and the second threshold is applied to the data found in the table 540, sequence identifier A, sequence identifier B, and sequence identifier D represented by dotted lines in FIG. 5 may be selected as a second sequence identifier to be described below in FIG. 6.

According to an embodiment of the present disclosure, an occurring frequency in the genomes which exist in the first layer and an occurring frequency in the genomes which exist in the second layer which is the higher layer may be computed, with respect to each of the first sequence identifiers which belong to the first layer. Alternatively, according to an embodiment of the present disclosure, an occurring frequency in the genomes which exist in the first layer with respect to each of the sequence identifiers which belong to the first layer and an occurring frequency in the genomes which exist in the second layer with respect to each of the sequence identifiers which belong to the second layer may be computed.

Through the comparison between the appearance frequencies and the thresholds, second sequence identifiers which have a high occurring frequency in the first layer and have a low occurring frequency in the higher layer of the first layer may be selected among the first sequence identifiers. Since the number of second sequence identifiers is much smaller than the number of first sequence identifiers, when an occurring frequency in a third layer which is a higher layer than the second layer is computed afterwards, the occurring frequency will be computed based on the second sequence identifiers. Accordingly, a complexity of a calculation for the computation of the occurring frequency in the third layer is reduced.

FIG. 6 illustrates an exemplary data structure used for computing an occurring frequency of sequence identifiers by considering a first layer and a third layer.

The second sequence identifiers selected among the first sequence identifiers by the filtering operation in FIG. 5 may become a target of the filtering operation in FIG. 6. For example, when sequence identifier A, sequence identifier B, and sequence identifier D in FIG. 5, sequence identifier A, sequence identifier B, and sequence identifier D may be used as the second sequence identifier to be considered in FIG. 6.

According to an embodiment of the present disclosure, the storage unit of the computing device may store a mapping relationship between the genome and the sequence identifier as exemplary table structures represented by reference numerals 620 and 640.

As illustrated in FIG. 6, a table 620 representing a mapping relationship between a genome and a sequence identifier in the third layer is illustrated. The table 620 may comprise a column 621 representing a list of genomes which belong to the third layer and a column 622 representing sequence identifiers found in the genome. the third layer in FIG. 6 may mean genomes acquired by excluding the genomes which belong to the first layer from all genomes which belong to the third layer which is the higher layer of the second layer, or the genomes.

In an additional embodiment of the present disclosure, unlike the example in FIG. 6, the third layer may also be enabled to be implemented as a form of representing all genomes which belong to the third layer or representing genomes acquired by excluding the genomes which belong to the second layer from all genomes which belong to the third layer.

In FIG. 6, it is illustrated that a separate table type data structure exists for each layer in the biological taxonomy of the organisms, but this is just an example for convenience of description, and according to an implement scheme or a design scheme, any type of data structure in which genomes and a sequence identifier found in a specific layer are enabled to be connected may be available, such as a data structure of managing all layers found in the biological taxonomy of the organism as one table, a data structure in which a plurality of tables exists for one layer, or a graph type data structure constituted by nodes and edges.

The computing device may obtain the second sequence identifiers (e.g., sequence identifier A, sequence identifier B, and sequence identifier D) selected according to the filtering operation result in FIG. 5.

In an embodiment, the computing device may compute an occurring frequency in the first layer with respect to each of the second sequence identifiers. The occurring frequency may mean an index indicating how many second sequence identifiers are found in the genomes found in the first layer.

In another embodiment, an occurring frequency in the first layer with respect to each second sequence identifier may be obtained by a scheme of utilizing the result of computing the occurring frequency in the first layer for each of the first sequence identifiers from the table 530 or the table 530' in FIG. 5 as it is. In this case, a separate operation process for computing the occurring frequency in the first layer for each of the second sequence identifiers may not be required.

A table 640 in FIG. 6 is a data structure for computing the number of genomes which belong to the third layer, to which each of the sequence identifiers found inin the third layer belongs. The table 640 may comprise a first column 641 including each of the sequence identifiers which belong to the third layer, a second column 642 representing a list of genomes to which each sequence identifier belongs, and a third column 643 representing the number of genomes to which each sequence identifier belongs. As an example, the table 640 may mean an index table in which an inverted index is applied to the table 620. As another example, the table 640 may also mean a pivot table for the table 620. As yet another example, the table 640 may also be a basic table representing the sequence identifier in the third layer, the genomes in the third layer, and the number of genomes to which each sequence identifier belongs.

Hereinafter, utilization examples for the table 620 and the table 640 in FIG. 6 will be described. In an embodiment of the present disclosure, shaded parts in the table 530' and the table 640 are used for expressing that data used for computing the occurring frequency in the table 640 is limited to the sequence identifiers found in the table 530 or the table 530'. In an additional embodiment, shaded items in the table 530' and the table 640 may represent the second sequence identifiers. Further, a shaded part in a table 530" is used for representing a sequence identifier which is not selected as a third sequence identifier among the second sequence identifiers.

The shaded part is used for representing the second sequence identifiers selected according to the result of performing filtering in FIG. 5, and the number of genomes including the second sequence identifiers. According to the filtering operation result in FIG. 5, sequence identifiers having sequence identifiers of A, B, and D are selected as the second sequence identifiers, sequence identifiers having IDs such as E, F, and Z correspond to the first sequence identifiers, but are excluded from the second sequence identifiers by filtering according to the filtering operation result in FIG. 5. In FIG. 6, it is described as an example that an additional filtering operation for each of the A, B, and D annotated sequence identifiers selected as the second sequence identifiers is performed. In an additional embodiment of the present disclosure, an embodiment to which considering and filtering the E, F, H, and Z annotated sequence identifiers found in the table 640 are additionally applied may also be enabled to be implemented.

In an embodiment of the present disclosure, the computing device may identify the number of genomes in the third layer to which each of the sequence identifiers found inin the third layer belongs by utilizing the table 620 and/or the table 640. Hereinafter, a computation scheme for the occurring frequency in the third layer according to the example of FIG. 6 will be described as an example.

The column 621 of the table 620 comprises genomes (e.g., genome 101 to genome 1000) which belong to the third layer acquired by excluding the genomes (e.g., genome 1 to genome 100) which belong to the first layer. Sequence identifiers found in each genome are illustrated in the column 622. In the table 640, the number of genomes which belong to the third layer, which comprise sequence identifier A is 0, the number of genomes which belong to the third layer, which comprise sequence identifier B is 7, the number of genomes which belong to the third layer, which comprise sequence identifier D is 11, the number of genomes which belong to the third layer, which comprise sequence identifier E is 18, the number of genomes which belong to the third layer, which comprise sequence identifier F is 19, the number of genomes which belong to the third layer, which comprise sequence identifier H is 25, and the number of genomes which belong to the third layer, which comprise sequence identifier Z is 48.

As illustrated in the table 530', the sequence identifier annotated with A may be a sequence identifier found in a total of 100 genomes among the genomes which belong to the first layer, the sequence identifier annotated with B may be a sequence identifier found in a total of 97 genomes among the genomes which belong to the first layer, and the sequence identifier annotated with D may be a sequence identifier found in a total of 96 genomes among the genomes which belong to the first layer. In the example of the table 530', the sequence identifier annotated with A has an occurring frequency of 1.00 as 100/100, the sequence identifier annotated with B has an occurring frequency of 0.97 as 97/100, and the sequence identifier annotated with D has an occurring frequency of 0.96 as 96/100.

As described above, in another embodiment of the present disclosure, the computation process for the occurring frequency may be omitted, and the occurring frequency for the second sequence identifiers may be obtained from the occurring frequency computed for the first sequence identifiers.

In an embodiment of the present disclosure, the occurring frequency for each of the second sequence identifiers in the first layer may be computed or obtained, and by comparing the computed or obtained occurring frequency and a fourth threshold, it may be considered that second sequence identifiers having an occurring frequency less than the fourth threshold are excluded or filtered from a sequence identifier(s) to be finally selected. For example, when the fourth threshold is set to 0.97, D among the sequence identifiers in the example of FIG. 6 may be removed according to the comparison with the fourth threshold. Here, the fourth threshold may mean a minimum reference value of the occurring frequency of the second sequence identifiers in the first layer. That is, the second sequence identifier which belongs to a lot of genomes in the first layer may be considered as a sequence identifier having a high occurring frequency, and the second sequence identifier which belongs to relatively a few genomes in the first layer may be considered as a sequence identifier having a low occurring frequency.

In the present disclosure, the third layer may mean a highest layer in the biological taxonomy of the organisms, for example. In such an example, the third layer may mean genomes acquired by excluding the genomes found in the first layer from the genomes found in the highest layer. Further, the third layer may also mean genomes acquired by excluding the genomes found in the second layer from the genomes found in the highest layer. Further, the third layer may also mean genomes acquired by excluding the genomes (i.e., the genomes acquired by excluding the genomes found in the first layer among the genomes found in the second layer) in the second layer from the genomes found in the highest layer. An example for the highest layer is the domain.

In another example, the third layer may mean a second highest layer in the biological taxonomy of the organisms. In such an example, the third layer may mean genomes acquired by excluding the genomes found in the first layer from the genomes found in the second highest layer. Further, the third layer may also mean genomes acquired by excluding the genomes found in the second layer from the genomes found in the second highest layer. Further, the third layer may also mean genomes acquired by excluding the genomes (i.e., the genomes acquired by excluding the genomes found in the first layer among the genomes found in the second layer) in the second layer from the genomes found in the second highest layer. An example for the second highest layer is the kingdom.

In yet another example, the third layer may mean a layer which is a higher than the second layer in the biological taxonomy of the organisms. In such an example, the third layer may mean genomes acquired by excluding the genomes which belong to the first layer from the genomes which belong to the third layer. As another example, the third layer may mean genomes acquired by excluding the genomes which belong to the second layer from the genomes which belong to the third layer. As yet another example, the third layer may also mean genomes acquired by excluding the genomes (i.e., the genomes acquired by excluding the genomes found in the first layer among the genomes found in the second layer) in the second layer from the genomes which belong to the third layer. An example for the third layer may comprise the family, the order, the class, the phylum, the kingdom, or the domain.

In an embodiment, an occurring frequency for second sequence identifiers in a difference set of the first layer for the third layer or a difference set of the second layer for the third layer may be computed.

According to an embodiment of the present disclosure, the sequence identifiers found in the column 641 in the table 640 may mean the sequence identifiers found inin the third layer. Sequence identifiers A, B, and D among the sequence identifiers found in the column 641 may correspond to the second sequence identifiers obtained through primary filtering, and E, F, H, and Z which are the remaining sequence identifiers may correspond to a sequence identifier excluded from the sequence identifiers found inin the first layer and/or a sequence identifier comprised only in the third layer. By comparing the genomes and the sequence identifiers in the third layer through the table 640, how many sequence identifiers are found in the genomes in the third layer may be computed.

The computing device may identify the number of genomes in the third layer to which each of the sequence identifiers belongs by utilizing the table 640. In the example of FIG. 6, the sequence identifier annotated with A may be a sequence identifier not found in the genomes which belong to the third layer, the sequence identifier annotated with B may be a sequence identifier found in a total of 7 genomes among the genomes which belong to the third layer, and the sequence identifier annotated with D may be a sequence identifier found in a total of 11 genomes among the genomes which belong to the third layer.

As illustrated in Table 620, a total number of genomes found inin the third layer is illustrated as 900. According to an embodiment of the present disclosure, the third layer may mean in which the first layer or the second layer is excluded from the higher layer of the second layer. For example, the third layer may mean a remaining range in which the specifies corresponding to the first layer is excluded from the domain or the kingdom in the biological taxonomy of the organisms. According to an embodiment of the present disclosure, the third layer may mean the highest layer in the biological taxonomy of the organisms.

As illustrated in FIG. 6, the third layer which is the higher layer of the second layer may comprise the second layer and other lower layers which exist parallel to the second layer. In the example of FIG. 6, a total of 1000 genomes may exist throughout the third layer, and the third layer may correspond to 900 genomes acquired by subtracting 100 genomes which belong to the first layer from 1000 genomes.

The computing device may obtain an occurring frequency by computing a ratio of an appearance number of sequence identifiers of a specific ID to 900 which is the number of genomes found inin the third layer. The occurring frequency may represent how many respective second sequence identifiers are found in the genomes of the third layer. Hereinafter, a scheme of computing the occurring frequency according to the example of FIG. 6 is described as an example.

In the example of FIG. 6, the sequence identifier annotated with A has an occurring frequency of 0 as 0/900, the sequence identifier annotated with B has an occurring frequency (rounding) of 0.0078 as 7/900, and the sequence identifier annotated with D has an occurring frequency (rounding) of 0.0122 as 11/900.

In an embodiment of the present disclosure, an occurring frequency in the third layer for each of the second sequence identifiers or for each of the sequence identifiers in the third layer may be computed, and by comparing the computed occurring frequency and a third threshold, it may be considered that sequence identifiers having an occurring frequency more than the third threshold are excluded from a sequence identifier(s) to be finally selected. For example, when the third threshold is set to 0.01, D among the sequence identifiers in the example of FIG. 6 may be excluded according to the comparison with the third threshold. According to the example of FIG. 6, sequence identifiers A and B may be selected as a final candidate sequence identifier (e.g., a third sequence identifier). Here, the third threshold may mean a maximum reference value of the occurring frequency of the sequence identifiers in the third layer. That is, the sequence identifier which belongs to a lot of genomes in the third layer may be considered as a sequence identifier having a high occurring frequency, and the sequence identifier which belongs to relatively a few genomes in the third layer may be considered as a sequence identifier having a low occurring frequency. Calculating the occurring frequency of the sequence identifier in the third layer is to find sequence identifiers which do not exist in the third layer range or exist relatively a few in the third layer range.

As described above, the occurring frequency in the first layer and the occurring frequency in the third layer for each of the second sequence identifiers may be obtained or computed. The appearance frequencies may be compared with the third threshold and the fourth threshold. In an embodiment, a second sequence identifier which satisfies a reference with the third threshold and satisfies a reference with the fourth threshold may not be excluded, but may be finally selected as the sequence identifier specific to the target analyte in the operation step in FIG. 6.

In an embodiment of the present disclosure, when the number of sequence identifiers output after application to the third threshold and the fourth threshold is less than a predetermined number or more than the predetermined number, the third threshold and/or the fourth threshold may be changed. As such, the computing device changes the third threshold and/or the fourth threshold to adjust a desired number of sequence identifiers to be output through filtering.

Sequence identifier A and sequence identifier B represented by dotted lines in FIG. 6 may be finally selected as a sequence identifier related to the target analyte, and sequence identifier D may be excluded through secondary filtering in FIG. 6. The table 530" in FIG. 6 illustrates a table in which a secondary filtering result is reflected to the table 530 or the table 530', and the column 531" represents sequence identifiers, the column 532" represent genomes including the sequence identifier, and the column 533" represents the number of genomes including the sequence identifier.

In an embodiment of the present disclosure, the table 530" is not a separately generated table, but is used for expressing that some data is selected in the table 530' or the table 530.

In another embodiment of the present disclosure, the table 530" may exist as a separate object from the table 530 or the table 530'.

In yet another embodiment of the present disclosure, the table 530" may exist as the same table as the table 530 and/or the table 530', and in this case, the table 530' may show a form in which any type of DML operation including modification, insertion, and/or deletion is applied to the table 530 or the table 530'.

In still yet another embodiment of the present disclosure, the table 530" may also exist as a form of a database view or materialized view having the table 530 or the table 530' as the basic table. In this case, when changed contents for data found in the table 530 or the table 530' are generated, data in the table 530" may be refreshed and the changed contents may be reflected to the data in the table 530".

In still yet another embodiment of the present disclosure, a first table may be constructed in advance, in which the genome corresponds to key and the sequence identifier corresponds to value with respect to organisms found in all layers. As in the table 510, the table 520, or the table 620, when information on the sequence identifier for each genome is required, information on the genome and the sequence identifier may be obtained by referring to the first table.

Further, a second table may be constructed in advance, in which the sequence identifier corresponds to a key and the genome corresponds to a value with respect to organisms found in all layers. As in the table 530, the table 540, or the table 640, when information on the genome for each sequence identifier is required, the information on the genome for each sequence identifier may be obtained by referring to the second table. The information constructed in advance is not limited thereto, and in some cases, a DB may be implemented in a scheme in which various information is constructed in advance, and required information is performed and obtained in a memory at a corresponding time.

In an embodiment of the present disclosure, the sequence identifier specific to the target analyte is described by taking some sequence identifiers as an example ' for convenience of description, but it will also be apparently understood by those skilled in the art that any number of sequence identifiers specific to the target analyte may also be selected. For example, in order to improve target coverage, in order to improve sensitivity, and/or in order to more excellently detect the target analyte, selecting various numbers of final sequence identifiers may also be found in the claims of the present disclosure.

As described above, according to an embodiment of the present disclosure, an occurring frequency in the genomes which exist in the first layer and an occurring frequency in the genomes which exist in the third layer which is the higher layer may be computed, with respect to each of the second sequence identifiers. Through the comparison between the appearance frequencies and the thresholds, final sequence identifiers which have a high occurring frequency in the first layer and have a low occurring frequency in the highest layer may be selected among the second sequence identifiers. The number of final sequence identifiers selected as such is adjustable according to the threshold. The selected sequence identifiers finally selected by such a scheme may be considered as sequence identifiers specific to the input target analyte.

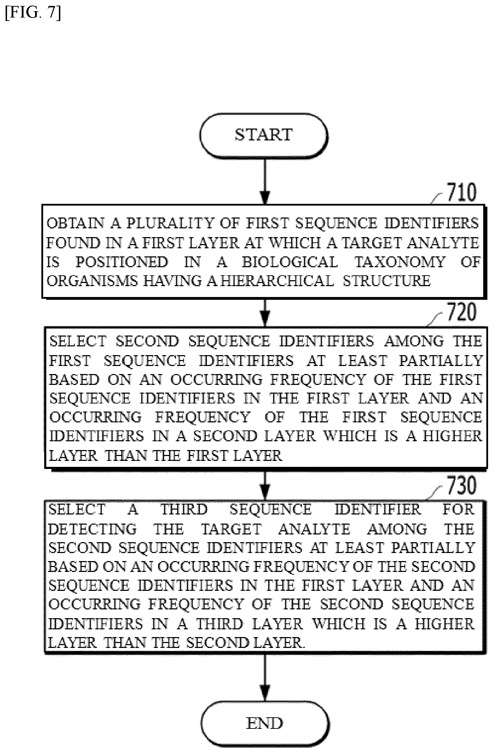

FIG. 7 is a flowchart exemplarily illustrating a method for selecting a candidate sequence identifier specific to a target analyte according to an embodiment of the present disclosure.

A method for selecting a sequence identifier described in FIG. 7 may be performed by the computing device.

The computing device may obtain a plurality of first sequence identifiers found in a first layer where a target analyte is positioned on a biological taxonomy of organisms having a hierarchical structure (710).

The computing device may receive an input related to the target analyte. For example, the computing device may receive an input such as the type of strain, or a name for a biological taxonomy of organism, such as species, genus, or family. As another example, input data may be a classification identifier (TAX ID) corresponding to the target analyte. As yet another example, the input data may be a name for the target analyte, and in this case, the computing device may determine a classification identifier corresponding to the name of the target analyte or any type of ID corresponding to the name of the target analyte in a DB.

The computing device may determine a layer for the biological taxonomy of the organism, to which the target analyte belongs according to the input related to the target analyte. When the target analyte is vibrio cholerae, the computing device may determine, as a first layer, a specific layer in the biological taxonomy of the organisms, to which vibrio cholerae belongs. For example, vibrio cholerae species corresponding to vibrio cholerae which is the target analyte may be determined as the first layer. As another example, vibrio genus that encompasses vibrio cholerae which is the target analyte may also be selected as the first layer.

In an embodiment of the present disclosure, the first layer may also be determined as one of lower layers in which at least two higher layers exist in the biological taxonomy of the organisms. The first layer used in FIG. 7 may comprise a specific layer in the biological taxonomy of the organisms determined to correspond to the target analyte. Further, the first layer used in FIG. 7 may also be used as meaning a lowest layer in the biological taxonomy of the organisms, to which the target analyte belongs.

In an embodiment of the present disclosure, the computing device may obtain first sequence identifiers found in the determined first layer. In FIG. 7, the first sequence identifier may mean sequence identifiers which the genomes found in the first layer have. Here, the sequence identifier is an ID for identifying base sequences, and various schemes for determining the identifier may be considered as described above.

The computing device may obtain sequence identifiers which all genomes found in the first layer have. The computing device may retrieve a list of all genomes which belong to the first layer and/or sequence identifiers which the all lists have, respectively by utilizing a storage unit positioned inside the computing device and/or a storage unit positioned outside the computing device. For example, the computing device inquires a list of genomes and/or sequence identifiers of a organism group corresponding to input information in a genome storage unit containing annotation information for a group of the base sequences to obtain the list of the genomes and/or the list of the sequence identifiers.

In FIG. 7, the computing device may select second sequence identifiers among first sequence identifiers at least partially based on an occurring frequency in a first layer and an occurring frequency in a second layer which is a higher layer than the first layer with respect to each of the first sequence identifiers (720).

The computing device computes the number of respective first sequence identifiers found in the genomes found in the first layer to determine an occurring frequency in the first layer. The occurring frequency as a value for determining the occurring frequency of the first sequence identifiers in the first layer at which the target analyte is positioned may be made by a scheme of determining the number of genomes in the first layer, which comprise the first sequence identifiers in the first layer. As an example, the occurring frequency may be a ratio of the number of genomes having the first sequence identifiers to the total number of genomes in the first layer. The scheme of determining the occurring frequency will be described in detail by referring the contents described in FIG. 5.

The computing device computes the number of respective first sequence identifiers found in the genomes found inin the second layer to determine an occurring frequency in the second layer. The occurring frequency as a value for determining the occurring frequency of the first sequence identifiers in the second layer which is a higher layer of the first layer at which the target analyte is positioned may be made by a scheme of determining the number of genomes in the second layer, which comprise the first sequence identifiers.

In an embodiment, the second layer may be one layer among the higher layers for the first layer in a hierarchical structure in which the higher layer encompasses the lower layer. In another embodiment, the second layer may be a layer corresponding to immediately higher hierarchical position of the first layer in the hierarchical structure. The second layer may be immediately higher hierarchical position of the first layer in which the number of genomes found in the second layer is more than a threshold number.

In yet another embodiment, the second layer may be a layer most adjacent to the first layer among higher layers of the first layer in which the number of genomes from which first genomes found in the first layer are excluded is more than a genome threshold number.

According to an embodiment of the present disclosure, the occurring frequency in the second layer may be a ratio of the number of genomes having the first sequence identifiers to the total number of genomes in the second layer. The scheme of determining the occurring frequency will be described in detail by referring the contents described in FIG. 5.

Through the occurring frequency in the first layer and occurring frequency in the second layer, a sequence identifier may be efficiently selected, which has a high occurring frequency in a region in a layer to which the target analyte belongs among the first sequence identifiers in the first layer to which the target analyte belongs and has a low occurring frequency in the other region in a higher layer from which the layer to which the target analyte belongs is excluded. When the occurring frequency is utilized, since the second sequence identifiers may be selected among the first sequence identifiers before calculating an occurring frequency in all organism group, an operation of acquiring an occurring frequency in all organism groups for the second sequence identifiers of which the number is relatively smaller may be enabled.

The computing device may select a third sequence identifier related to the target analyte among the second sequence identifiers at least partially based on an occurring frequency in a third layer which is a higher layer than the second layer and the occurring frequency in the first layer with respect to each of the second sequence identifiers (730).

An occurring frequency of the second sequence identifier in the third layer may mean the number of genomes to which each of the second sequence identifiers belongs among genomes acquired by excluding the first genomes or the second genomes from third genomes found in the third layer, and an occurring frequency of the second sequence identifier in the first layer may mean the number of genomes to which each of the second sequence identifiers belongs among the first genomes.

As described above, second sequence identifiers which are highly likely to be specific to the target analyte and not specific to other organisms among the first sequence identifiers related to the target analyte may be selected with a relatively smaller operation amount through step 720.

In step 730, a process of computing the appearance frequencies for the second sequence identifiers selected through step 720 is described. The computing device computes the number of genomes including each of the second sequence identifiers among the genomes found inin the third layer which is the higher layer of the second layer to determine an occurring frequency for the second sequence identifiers in the third layer.

In an embodiment, the third layer may mean the higher layer of the second layer. In another embodiment, the third layer may mean a highest layer or a second highest layer in the biological taxonomy of the organisms related to the target analyte.

In an embodiment, the third layer may mean a difference set of the first layer for the third layer or a difference set of the second layer for the third layer.

In an embodiment of the present disclosure, the computing device may obtain an occurring frequency of the second sequence identifier in the first layer from a result of the occurring frequency of the first sequence identifier in the first layer. In respect to all of the appearance frequencies, the ranges of the genomes which is the first layer correspond to each other, so in the embodiment, an occurring frequency corresponding to the second sequence identifiers may be obtained from an occurring frequency computation result for the first sequence identifiers including the second sequence identifiers without performing a separate operation of acquiring the occurring frequency.

In another embodiment, the computing device may also compute the occurring frequency by a scheme of acquiring the number of genomes including the second sequence identifiers, respectively among the genomes in the first layer with respect to each of the second sequence identifiers.

The scheme of determining the occurring frequency will be described in detail by referring the contents described in FIG. 6.

In an additional embodiment of the present disclosure, the computing device may also compute an occurring frequency representing the occurring frequency of each of the second sequence identifiers in the second layer with respect to each of the second sequence identifiers. According to such an embodiment, the computing device may select a third sequence identifier related to the target analyte among the second sequence identifiers by additionally considering the occurring frequency in the second layer for each of the second sequence identifiers at least partially based on the plurality of appearance frequencies. As an example, a second sequence identifier may be selected as the third sequence identifier, which has a highest occurring frequency in the first layer having a highest relevance with the target analyte, has a relatively low occurring frequency in the second layer having a second highest relevance with the target analyte, and has a lowest occurring frequency in the third layer which may have a lowest relevance with the target analyte.

The computing device calculates a score for each of the second sequence identifiers based on the plurality of appearance frequencies to select at least one third sequence identifier among the second sequence identifiers. As an example, as the size of the occurring frequency in the first layer for each of the second sequence identifiers is larger, a higher score may be calculated, as the size of the occurring frequency in the third layer for each of the second sequence identifiers is smaller, a higher score may be calculated, and as the size of the occurring frequency in the second layer for each of the second sequence identifiers is smaller, a higher score may be calculated. After the score for each of the second sequence identifiers is calculated, the second sequence identifier having a higher score may be selected as the third sequence identifier.

According to an embodiment of the present disclosure, an example algorithm for determining the score is described in Equation 1 below.

[Equation 1]

$$Score = \frac{\left(\frac{Fin}{Gin}\right) \times \left(\frac{Gtotal + Gin}{Gtotal}\right)}{\left(\frac{Fexp}{Gexp} \div \frac{Gtotal + Gexp}{Gtotal}\right) + \frac{Fext}{Gext}}$$

In Equation 1, Fin represents the number of genomes in the first layer for a sequence identifier which becomes an operation target of the score value among the second sequence identifiers (or first sequence identifiers), Gin, represents the number of genomes found in the first layer, Gtotal represents the number of genomes found in the third layer, Fexp represents the number of genomes in the second layer for the sequence identifier which becomes an operation target of the score value among the second sequence identifiers (or first sequence identifiers), Gexp represents the number of genomes found inin a difference set of the first layer for the second layer, Fext represents the number of sequence identifiers in the third layer for the sequence identifier which becomes the operation target of the score value among the second sequence identifiers (or first sequence identifiers), and Gext represents the number of genomes found inin a difference set of the first layer for the third layer or a difference set of the second layer for the third layer.

Specifically, in Equation 1 above, Fin/Gin may mean an occurring frequency of the second sequence identifier (or first sequence identifier) in the first layer. (Gtotal+Gin)/Gtotal may mean a value acquired by dividing a number acquired by aggregating the total number of genomes found in the third layer and the number of genomes found in the first layer by the number of genomes found in the third layer.

(Gtotal+Gin)/Gtotal may be a parameter representing a ratio of the genomes found in the first layer to the genomes found in the third layer. By multiplying Fin/Gin is multiplied by (Gtotal+Gin)/Gtotal showing a larger value as more genomes are found in the first layer, a value of Fin/Gin is converted into a larger value, and as a result, a relatively higher score may be calculated. By multiplying Fin/Gin is multiplied by (Gtotal+Gin)/Gtotal showing a smaller value as less genomes are found in the first layer, the value of Fin/Gin is converted into a smaller value, and as a result, a relatively lower score may be calculated.

Fexp/Gexp may mean an occurring frequency of the second sequence identifier (or first sequence identifier) in the second layer. (Gtotal+Gexp)/Gtotal may mean a value acquired by dividing a number acquired by aggregating the total number of genomes found in the third layer and the number of genomes found in the second layer by the number of genomes found in the third layer.

(Gtotal+Gexp)/Gtotal may be a parameter representing a ratio of the genomes included in the second layer to the genomes found in the third layer. By multiplying Fexp/Gexp is multiplied by (Gtotal+Gexp)/Gtotal showing a larger value as more genomes are found in the second layer, a value of Fexp/Gexp is converted into a larger value, and as a result, a relatively higher score may be calculated. By multiplying Fexp/Gexp is multiplied by (Gtotal+Gexp)/Gtotal showing a smaller value as less genomes are found in the second layer, a value of Fexp/Gexp is converted into a smaller value, and as a result, a relatively lower score may be calculated.

Fext/Gext may mean an occurring frequency of the second sequence identifier (or first sequence identifier) in the third layer.

As an example, as Fin/Gin that means the occurring frequency of the second sequence identifier (or first sequence identifier) in the first layer is larger, the score may be higher.. Further, as Fexp/Gexp that means the occurring frequency of the second sequence identifier (or first sequence identifier) in the second layer is larger, the score may be lower. Further, as Fext/Gext that means the occurring frequency of the second sequence identifier (or first sequence identifier) in the third layer is larger, the score may be lower.

The computing device may obtain variable values used in Equation 1 described above for respective sequence identifiers, and then compute score values for the respective sequence identifiers. In an embodiment of the present disclosure, a sequence identifier in which a quantitative value of the score in Equation 1 is large is likely to be selected as a final candidate sequence identifier (i.e., third sequence identifier). On the contrary, a sequence identifier in which the quantitative value of the score in Equation 1 is small is unlikely to be selected as the final candidate sequence identifier (i.e., third sequence identifier). The computing device may sort final candidate sequence identifiers based on the size of the score value.

As described above, the method for selecting the sequence identifier according to an embodiment of the present disclosure may select sequence identifiers which are most found in the target analyte among the first sequence identifiers related to the target analyte and which are not almost found in other organisms other than the target analyte by a calculation efficiency scheme. When all of the first sequence identifiers related to the target analyte are compared with the genomes in the whole organism group range, a significantly complicated calculation may be required. In the technique according to an embodiment of the present disclosure, the first sequence identifiers related to the target analyte are compared in the second layer in which the number of genomes is relatively smaller to select the second sequence identifier, and the genomes in the whole organism group are compared based on the selected second sequence identifiers, so a sequence identifier(s) specific to the target analyte may be selected within a relatively shorter time period while efficiently managing computing resources.

According to an embodiment of the present disclosure, the computing device may select the second sequence identifiers through primary filtering for the first sequence identifiers, and select at least one third sequence identifier specific to the target analyte through secondary filtering for the second sequence identifiers.

According to an additional embodiment of the present disclosure, an additional algorithm may also be applied to a sequence identifier continuously found in a human body. For example, an additional algorithm may also be applied to a sequence identifier (hereinafter, defined as 'a predetermined sequence identifier') found in resident flora such as bacteria or virus which should be resident in the human body or specific to the resident flora, or a housekeeping gene manifested even in any situation as a gene indispensable for cell survival. Specifically, in the case of human beings, beneficial bacteria such as lactic acid bacteria are resident flora which is continuously resident in the body, and the lactic acid bacteria (or a sequence identifier found in the lactic acid bacteria) may become the predetermined sequence identifier. When vibrio cholerae bacteria are intended to be detected from the human body as the target analyte for the human beings, the predetermined sequence identifier found in the lactic acid bacteria may be excluded from a final candidate sequence identifier for detecting vibrio cholerae. In an embodiment, the resident flora may exist in the skin, stomach, oral, upper airway, teeth, respiratory system, urinary tract and eye cornea and external auditory canal, and may comprise Staphylococci, Micrococci, Diphtheroids, Neisseria, Bordetella, Corynebacterium, Streptococcus spp, Helicobacter pylori, Candida, Escherichia coli, Haemophilus, and Staphylococcus, and is not limited thereto.

Further, when vibrio cholerae bacteria are intended to be detected from the human body as the target analyte for the human beings, the predetermined sequence identifier found in the housekeeping gene may be excluded from the final candidate sequence identifier for detecting vibrio cholerae. In an embodiment, the housekeeping gene may comprise GAPDH, B-actin, and a-tubuline, and the human body housekeeping gene may comprise NM_001101 Homo sapiens Mala, Beta (ACTB), mRNA6988, NM_000034 Homo sapiens Dola, fructose-bisphosphate (ALDOA), mRNA3425, NM_002046 Homo sapiens glyceraldehyde-3-phosphate dehydrogenase (GAPD), and mRNA828, and is not limited thereto.

The predetermined sequence identifier is described based on the lactic acid bacteria and the housekeeping gene, but is not limited thereto.

As such, the predetermined sequence identifiers may be excluded in the selection process of the sequence identifiers in spite of a situation in which the occurring frequency satisfies the threshold in the primary filtering and/or secondary filtering processes. In some embodiments, the predetermined sequence identifier may be excluded in a state in which the computation of the occurring frequency is completed in the process of obtaining the first sequence identifier, the process of selecting the second sequence identifier, or the process of selecting the third sequence identifier. Alternatively, the predetermined sequence identifier may be excluded without computing the occurring frequency.

According to an implementation scheme, a step of excluding the predetermined sequence identifier may be performed in a step of selecting the first sequence identifier, or performed in a step of selecting the second sequence identifier and performed in a step of selecting the third sequence identifier. According to the implementation scheme, a step of excluding the predetermined sequence identifier may be performed before and after the primary filtering process and/or the secondary filtering process, or performed jointly with the primary filtering process and/or the secondary filtering process, or performed regardless of primary filtering process and/or the secondary filtering process. As such, the step of excluding the predetermined sequence identifier may be performed in various orders.

In an embodiment, the selected second sequence identifier may not comprise a predetermined sequence identifier among the sequence identifiers in which the occurring frequency in the first layer for each of the first sequence identifiers is equal to or more than a first threshold and the occurring frequency in the second layer for each of the first sequence identifiers is equal to or less than a second threshold. The selected second sequence identifier may be a sequence identifier in which a sequence identifier of a specific organism in which exclusivity is predetermined is removed among the sequence identifiers in which the occurring frequency in the first layer for each of the first sequence identifiers is equal to or more than the first threshold and the occurring frequency in the second layer for each of the first sequence identifiers is equal to or less than the second threshold.

Further, the selected third sequence identifier may not comprise a predetermined sequence identifier among the sequence identifiers in which the occurring frequency in the third layer for each of the second sequence identifiers is equal to or less than a third threshold and the occurring frequency in the first layer for each of the second sequence identifiers is equal to or more than a fourth threshold. The selected third sequence identifier may be a sequence identifier in which a sequence identifier of a specific organism in which exclusivity is predetermined is removed among the sequence identifiers in which the occurring frequency in the third layer for each of the second sequence identifiers is equal to or less than the third threshold and the occurring frequency in the first layer for each of the second sequence identifiers is equal to or more than the fourth threshold.

Further, the first sequence identifier may not comprise the predetermined sequence identifier.

According to another embodiment of the present disclosure, the computing device may obtain a plurality of first sequence identifiers found in the first layer at which the target analyte is positioned in the biological taxonomy of the organisms having the hierarchical structure, select the second sequence identifiers among the first sequence identifiers at least partially based on the occurring frequency in the first layer and the occurring frequency in the second layer which is the higher layer than the first layer with respect to the first sequence identifiers, and select the third sequence identifier related to the target analyte among the second sequence identifiers at least partially based on the occurring frequency in the first layer and an occurring frequency in a whole organism database with respect to the second sequence identifiers.

In this case, the second layer may be a range from which the first layer is excluded, and the third layer may be a range from which the first layer (or the first layer and the second layer) are excluded.

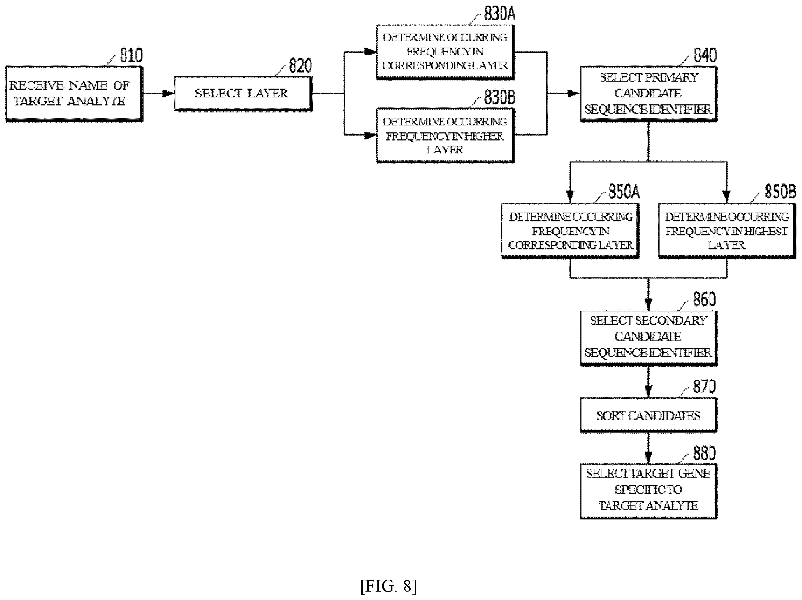

FIG. 8 is a flowchart exemplarily illustrating a method for selecting a target sequence identifier specific to a target analyte according to an embodiment of the present disclosure.

The method illustrated in FIG. 8 may be implemented by the computing device. Parts duplicated with FIG. 7 among contents described in FIG. 8 will be omitted in a process of describing FIG. 8 for simplification of description.

The computing device may receive a name for a target analyte (810). The above-described name as information for identifying the target analyte may comprise aTAX ID, a gene ID, a sequence identifier ID, a name representing the species of the analyte, and/or a genome ID.

The computing device may select a layer corresponding to the name for the target analyte (820). The computing device may select, as a first layer, a layer of a minimum range in the biological taxonomy of the organism to which the target analyte belongs. The computing device may select, as a second layer, a parent layer which is a higher layer of the first layer. Additionally, the computing device may select, as a third layer, a highest layer in the biological taxonomy of the organism related to the target analyte.

The computing device inquires an annotation storage unit to obtain a list of first sequence identifiers which all genomes in the first layer at which the target analyte is positioned have.

The computing device may determine an occurring frequency of each of the first sequence identifiers in the corresponding layer (i.e., the first layer) at which the target analyte is positioned (830A). The computing device may determine an occurring frequency of each of the first sequence identifiers in a higher layer selected as the second layer (830B). In an example where the target analyte is vibrio cholerae, the first layer may be a layer corresponding to vibrio cholerae species, and the occurring frequency of each of the first sequence identifiers in the first layer may be determined based on the number of genomes which belong to the vibrio cholerae species including respective sequence identifiers found in genomes which belong to the vibrio cholerae species. Further, the second layer as a higher layer adjacent to the layer to which vibrio cholerae belongs may mean a layer corresponding to the vibrio genus, and the second layer may mean genomes in which the genomes belonging to the vibrio species are excluded from the vibrio genus. The number of genomes including the respective first sequence identifiers among the genomes in which the genomes belonging to the vibrio species are excluded from the vibrio genus is computed to determine the occurring frequency of each of the first sequence identifiers in the second layer.

After determining the occurring frequency in the first layer and the occurring frequency in the second layer with respect to each of the first sequence identifiers, the computing device may select a primary candidate identifier which has a high occurring frequency for the target analyte and a relatively low occurring frequency for other organisms among the first sequence identifiers (840). For example, the primary candidate sequence identifier may correspond to the second sequence identifier in FIG. 6. When selecting the primary candidate sequence identifiers (the second sequence identifier of FIG. 6), at least two thresholds may be utilized. A first threshold is a threshold for determining a reference value for the occurring frequency of each of the first sequence identifiers in the first layer, and a second threshold is a threshold for determining a reference value for the occurring frequency of each of the first sequence identifiers in the second layer. The thresholds are applied to the appearance frequencies to filter sequence identifiers corresponding to being more than a specific occurring frequency or less than the specific occurring frequency.

The number of primary candidate identifiers selected among the first sequence identifiers may be variable according to the first threshold and the second threshold. The first threshold and the second threshold may be adjusted at least partially based on a target number of primary candidate identifiers, a target number of secondary candidate identifiers, a state of a computing resource, and/or the number of all genomes found in the biological taxonomy of the organisms to which the target analyte belongs. As such, as at least two thresholds are applied to the occurring frequency of the first sequence identifiers in the first layer and the occurring frequency of the first sequence identifiers in the second layer, sequence identifiers which are comprised as many as possible in the first layer and comprised as few as possible in the second layer may be primarily selected in the ranges of the first layer and the second layer.

As such, when the primary candidate identifier (e.g., the second sequence identifier) is selected according to the occurring frequency in the first layer and the occurring frequency in the second layer among the first sequence identifiers, the complexity of the calculation is reduced in determining the occurring frequency in the highest layer, and as a result, the computing resource may be efficiently managed.

The computing device may determine the number of genomes including the primary candidate sequence identifier in the corresponding layer (i.e., the first layer) with respect to each of the primary candidate sequence identifiers. The computing device may determine an occurring frequency of each of the primary candidate sequence identifiers in the first layer (850A). Further, the computing device may determine the number of genomes including the primary candidate sequence identifier in the highest layer (i.e., the third layer) with respect to each of the primary candidate sequence identifiers. The computing device may determine an occurring frequency of each of the primary candidate sequence identifiers in the highest layer (850B).

In the example in which the target analyte is vibrio cholerae, the third layer may be a layer corresponding to the highest layer (bacteria domain) or the second highest layer (eubacteria kingdom) of the vibrio cholerae species, and the occurring frequency in the third layer may be computed based on determining the number of genomes to which each of the primary candidate sequence identifiers belongs in genomes in which the genomes of the first layer or the second layer are excluded from the highest layer or the second highest layer. That is, the occurring frequency of each of the primary candidate sequence identifiers in the third layer may be determined according to the number of genomes including the primary candidate sequence identifier among the remaining genomes in which the genomes found in the vibrio genus are excluded from the genomes found in the bacteria domain.

The computing device may select at least one secondary candidate sequence identifier among the primary candidate sequence identifiers based on obtaining the occurring frequency in the first layer with respect to each of the primary candidate sequence identifiers and computing the occurring frequency in the highest layer (i.e., the third layer) (860). In an embodiment of the present disclosure, the secondary candidate sequence identifier may mean a sequence identifier which is likely to be specific to the target analyte. For example, the secondary candidate sequence identifier may correspond to the third sequence identifier in FIG. 7.

When selecting at least one secondary candidate sequence identifiers (the third sequence identifier of FIG. 7), at least two thresholds may be utilized. A third threshold is a threshold for determining a reference value for the occurring frequency of the primary candidate sequence identifiers in the third layer, and a fourth threshold is a threshold for determining a reference value for the occurring frequency of the primary candidate sequence identifiers in the first layer. The thresholds are applied to the appearance frequencies to remove sequence identifiers corresponding to being more than a specific occurring frequency or less than the specific occurring frequency in the filtering process. The number of secondary candidate identifiers selected among the primary candidate sequence identifiers may be variable according to the third threshold and the fourth threshold. The third threshold and the fourth threshold may be adjusted at least partially based on a target number of secondary candidate identifiers, a state of a computing resource, and/or the number of all genomes found in the biological taxonomy of the organisms to which the target analyte belongs.

In the sequence identifier selecting method according to an embodiment of the present disclosure, since the primary filtering operation and the secondary filtering operation are associated in series, computing resources required for selecting sequence identifiers specific to a specific target analyte may be efficiently used, so there may also be an advantage in that several target sequence patterns (e.g., sequence identifiers corresponding to a plurality of target analytes) having sensitivity and specificity at a predetermined level or more may be simultaneously confirmed.

In an embodiment of the present disclosure, the computing device may sort selected secondary candidate sequence identifiers (870). The computing device may sort the secondary candidate sequence identifiers based on a sorting scheme for any type of sequence identifiers. As an example, the computing device may sort the secondary candidate sequence identifiers (e.g., the third sequence identifiers) in the order of a highest score. As another example, the computing device may sort the secondary candidate sequence identifiers in ascending or descending order. In yet another example, the computing device may sort the secondary candidate sequence identifiers by a scheme of comparing base sequences for the selected secondary candidate sequence identifiers, respectively. As another example, the computing device may also sort the secondary candidate sequence identifiers by a scheme of inserting, deleting and changing at least some of the base sequences for the secondary candidate sequence identifiers. As yet another example, the computing device may also rearrange at least some of the secondary candidate sequence identifiers by applying operations such as reversal, transposition, transposition, etc., for at least some of the base sequences for the secondary candidate sequence identifiers.

In an embodiment of the present disclosure, the computing device may select a target gene specific to a target analyte (880). For example, the computing device may select one or more sequence identifiers among the secondary candidate sequence identifiers as the target gene specific to the target analyte. As another example, the computing device may select one or more sequence identifiers among the sorted secondary candidate sequence identifiers as the target gene specific to the target analyte after performing a sorting process for the secondary candidate sequence identifiers. In an embodiment, the target gene may be a sequence identifier which is very unlikely to be specific the target analyte and to exist for other organisms other than the target analyte.

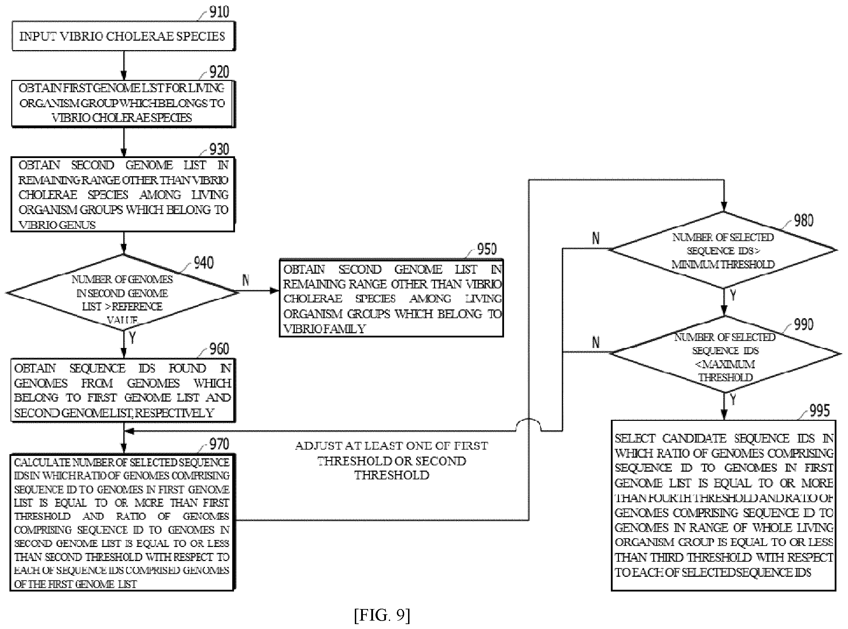

FIG. 9 illustrates an exemplary flowchart for selecting the candidate sequence identifier specific to the target analyte according to an embodiment of the present disclosure.

The descriptions disclosed in FIGS. 4, 5, 6, 7, and 8 are referenced for parts duplicated with FIGS. 4, 5, 6, 7, and 8 among parts expressed in FIG. 9, and in FIG. 9, the description of the duplicated parts will be omitted for convenience of description.

The computing device may receive an input for vibrio cholerae species (910). In FIG. 9, as an example for the target analyte, vibrio cholerae will be used. For example, the computing device may receive TAX ID representing vibrio cholerae.

The computing device may obtain a first genome list for a organism group to which vibrio cholerae species belong (920).

As described in FIG. 4, the computing device may obtain, as the first genome list, a whole list of genomes which belong to the vibrio cholerae species. The first genome list may mean information on all genomes which the vibrio cholerae species have. For example, the computing device may inquire a list for the genomes and the sequence identifiers of the organism group which belongs to the vibrio cholerae species to a genome storage containing annotation information. The first genome list may comprise, for example, the table 510 and/or the table 530 illustrated in FIG. 5.

The computing device may obtain a second genome list in the remaining range other than the vibrio cholerae species in the organism group which belongs to vibrio genus which is a parent layer of the vibrio cholerae species (930).

The computing device may obtain, as the second genome list, a whole list of genomes which belong to the vibrio genus which is a higher layer of the vibrio cholerae species. The vibrio genus may comprise a plurality of vibrio species. One vibrio species among the plurality of vibrio species corresponds to vibrio cholerae.

The second genome list may comprise the remaining genomes in which the genomes found inin the vibrio cholerae species are excluded from the genomes found inin the vibrio genus. The second genome list may mean information on genomes which the remaining species other than the vibrio cholerae species have among all genomes which the vibrio cholerae genus has. For example, the computing device may inquire a list for the remaining genomes acquired by subtracting the genomes of the organism group which belongs to the vibrio cholerae species from the genomes of the organism group which belongs to the vibrio genus, and sequence identifiers thereof to the genome storage containing the annotation information. The computing device may obtain the second genome list by inquiring a parent TAX ID which is an immediately higher layer of the corresponding TAX ID to the genome storage containing the annotation information. The second genome list may comprise, for example, the table 520 and/or the table 540 illustrated in FIG. 5.

According to an embodiment of the present disclosure, the computing device may compare the number of genomes found in the obtained second genome list and a predetermined reference value (940). The computing device may determine whether the number of genomes found in the second genome list exceeds the predetermined reference value. The predetermined reference value may mean a minimum number of genomes found inin the second layer for driving a significant result value. For example, when there are a relatively small number of genomes found inin the second layer, a filtering result of a primary filtering step of computing the occurring frequency in the first layer and the occurring frequency in the second layer may not be significant. When the number of genomes found inin the second layer is small, the number of first sequence identifiers corresponding to the first genome list will not be reduced through comparison with the second layer or only a very small number of sequence identifiers will be excluded. In this case, since the number of sequence identifiers to be compared with the genomes in the third layer becomes relatively larger, the complexity of the calculation in the secondary filtering step in the third layer cannot but increase. Accordingly, when the number of genomes found in the second genome list is not lager than the predetermined reference value, the computing device may select the second layer as a higher layer than the first layer by 2 steps rather than a higher layer adjacent to the first layer. As a result, before an operation step in the third layer, the number of sequence identifiers used for the operation in the third layer is reduced, thereby achieving the efficiency of the operation in the third layer.

According to an embodiment of the present disclosure, when determining that the number of genomes found in the second genome list does not exceed the predetermined reference value, the computing device may obtain a second genome list in the remaining range other than the vibrio cholerae species in the organism group which belongs to vibrio family which is the higher layer of the vibrio genus (950). The computing device extends a layer range in the biological taxonomy of the organisms to be selected as the second layer to obtain a second genome list including a sufficient number of genomes. After the second layer is extended to the organism group which belongs to the vibrio family, the computing device may compare the number of genomes which belong to the extended the second layer with the predetermined reference value once again. When it is determined that the number of genomes is not larger than the predetermined reference value according to a comparison result, the computing device may select vibrio order which is a layer higher than the vibrio family by one step as the second layer.

In an additional embodiment of the present disclosure, steps 940 and 950 may be repeated at a predetermined number of repetition times or according to a repetition reference. For example, steps 940 and 950 may also be predetermined to be repeated only up to a level corresponding to phylum in the biological taxonomy of the organisms.

When it is determined that the number of genomes in the obtained second genome list exceeds the predetermined reference value, the computing device may obtain the sequence identifier found in the genome from each of the genomes which belong to the first genome list and the second genome list (960).

As illustrated above in FIG. 5, the sequence identifier corresponding to the first genome list and the sequence identifier corresponding to the second genome list may be obtained. One or more sequence IDs (e.g., sequence identifiers) may be stored to be mapped to the genomes found in the first genome list, respectively. The computing device may obtain the sequence identifiers corresponding to the genomes found in the first genome list, respectively from a mapping relationship between the genomes and the sequence identifiers. As described above, there may be various methods of obtaining, from the genome list, a sequence identifier corresponding to the genome list, and the computing device according to the embodiments of the present disclosure may perform a method for obtaining the sequence identifier from a data structure which may indicate a connection relationship between the genome list and the sequence identifier.

The computing device may calculate the number of selected sequence identifiers, in which a ratio of genomes including, for each of the sequence identifiers found in the genomes of the first genome list, the corresponding sequence identifier to the genomes in the first genome list is equal to or more than a first threshold, and a ratio of the genomes including the corresponding sequence identifier to the genomes in the second genome list is equal to or less than a second threshold (970). Step 970 may comprise the primary filtering operation for the sequence identifiers (e.g., the first sequence identifiers).

Here, the first threshold may mean a minimum reference value for an occurring frequency of the sequence identifiers corresponding to the first genome list in the organism group to which the vibrio cholerae species belong, and the second threshold may mean a maximum reference value for an occurring frequency of the sequence identifiers corresponding to the first genome list in the organism group to which the vibrio genus (or a higher layer therethan) belongs. As another example, the first threshold and the second threshold may also be expressed as an index indicating a filtering strength for the primary filtering operation of selecting the second sequence identifiers among the first sequence identifiers.

For example, the first threshold which is initially set may be 0.95, and the second threshold may be 0.05. A such, a size of the fist threshold is larger than a size of the second threshold. The first threshold being larger indicates that the number of genomes found in the first genome list, which comprise the sequence identifiers should be large. The second threshold being smaller indicates that the number of genomes found in the second genome list, which comprise the sequence identifiers should be small.

Through step 970, the computing device may compute the number of first genomes of the first genome list, which comprise the sequence identifiers corresponding to the first genome list, respectively, and compute the number of second genomes of the second genome list, which comprise the sequence identifiers corresponding to the first genome list, respectively.

The computing device calculates the number of genomes including a specific sequence identifier among the first genomes as a specific sequence identifier unit to compute a ratio of the number of genomes including the specific sequence identifier to the number of first genomes. Here, the specific sequence identifier is the sequence identifiers found in the first genomes. The computing device may determine the selected sequence identifiers based on a scheme of excluding a specific sequence identifier corresponding to being less than the first threshold by comparing the computed ratio and the first threshold.

Further, the computing device calculates the number of genomes including a specific sequence identifier among the second genomes as a specific sequence identifier unit to compute a ratio of the number of genomes including the specific sequence identifier to the number of second genomes. Here, the specific sequence identifier is the sequence identifiers found in the first genomes. The computing device may determine the selected sequence identifiers based on a scheme of excluding a specific sequence identifier corresponding to being more than the second threshold by comparing the computed ratio and the second threshold.

The computing device may compare the number of selected sequence identifiers and a minimum threshold number (980). The computing device may compare the number of selected sequence identifiers and a maximum threshold number (990). When the number of selected sequence identifiers is smaller than the minimum threshold number, the computing device may increase the number of selected sequence identifiers by adjusting the first threshold used in step 970. When there are an excessively small number of selected sequence identifiers, the complexity of the calculation in a subsequent secondary filtering step (i.e., step 995) may not be reduced, but it may be difficult to select a candidate sequence identifier specific to the vibrio cholerae species. Further, when there are an excessively large number selected sequence identifiers, the complexity of the calculation in the subsequent secondary filtering step (i.e., step 995) may be caused. Accordingly, there is a need for guaranteeing the accuracy of the selected candidate sequence identifier while reducing the complexity of the calculation by setting the minimum threshold number and the maximum threshold number.

According to an embodiment of the present disclosure, for example, when the number of selected sequence identifiers is smaller than the minimum threshold number, the computing device may increase the number of selected sequence identifiers by adjusting the first threshold to be lowered and/or adjusting the second threshold to be raised. As another example, when the number of selected sequence identifiers is larger than the maximum threshold number, the computing device may reduce the number of selected sequence identifiers by adjusting the first threshold to be raised and/or adjusting the second threshold to be lowered. Here, the minimum threshold number may mean a minimum reference value for the number of selected sequence identifiers, and the maximum threshold number may mean a maximum reference value for the number of selected sequence identifiers. As such, the computing device adjusts the filtering strength of the filtering operation in step 970 through the minimum threshold number and the maximum threshold number (i.e., adjusts the size of the first threshold and/or the second threshold to be raised and/or lowered), so the complexity of the calculation in subsequent step 995 may be reduced and a result that the selection accuracy of the candidate sequence identifier may be guaranteed may be achieved.

As an example, as widths of the adjusted thresholds (the first threshold and the second threshold), values of 0.01 to 0.05 may be used. For example, when the number of selected sequence identifiers is smaller than the minimum threshold number, the first threshold and/or the second threshold may be adjusted with the width of 0.01 to 0.05. As another example, as the width of the adjusted thresholds, values of 0.001 to 0.01 may be used. For example, when the number of selected sequence identifiers is larger than the maximum threshold number, the first threshold and/or the second threshold may be adjusted with the width of 0.001 to 0.01.

The computing device may select a candidate sequence identifier, in which a ratio of genomes including, for each of the selected sequence identifiers, the corresponding sequence identifier to the genomes in the first genome list is equal to or more than a fourth threshold, and a ratio of the genomes including the corresponding sequence identifier to the genomes in the whole organism group range is equal to or less than a third threshold (995). When it is determined that the number of selected sequence identifiers is more than the minimum threshold number and the number of selected sequence identifiers is less than the maximum threshold number, step 995 may be performed. Step 995 may comprise the secondary filtering operation for the selected sequence identifiers (e.g., the second sequence identifiers).

In order to finally select the candidate sequence identifier, in step 995, the computing device may utilize a fourth threshold which is a minimum reference value for the occurring frequency of the selected sequence identifiers (e.g., the second sequence identifiers) in the organism group which belongs to the vibrio cholerae species and a third threshold which is a maximum reference value for the occurring frequency of the selected sequence identifiers (e.g., the second sequence identifiers) in the whole organism group range. The third threshold and the fourth threshold may be expressed as an index indicating the filtering strength of the secondary filtering operation corresponding to step 995.

In an additional embodiment of the present disclosure, additional filtering for the number of finally selected candidate sequence identifiers may also be applied in step 995. In this case, the number of finally selected candidate sequence identifiers and one or more thresholds may be compared. When it is determined that the comparison result satisfies a predetermined reference, the selected candidate sequence identifier may be output, and when it is determined that the comparison result does not satisfy the predetermined reference, the number of finally selected candidate sequence identifiers may be changed by adjusting the third threshold and/or the fourth threshold.

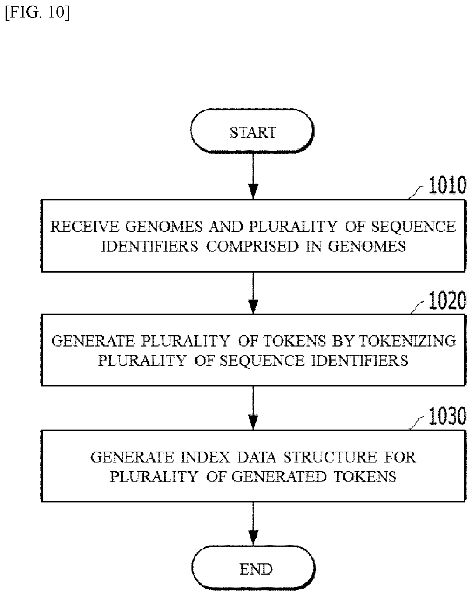

The processes described above in FIG. 9 are enabled to be implemented with a calculation amount at a level to be performed with performance of a general personal computer. Accordingly, the selection method of the sequence identifier according to an embodiment of the present disclosure may have an advantage in that the number of sequence identifiers of which all are to be searched is reduced by primarily calculating specificity in a higher classification group, and then a search process in the whole organism group range is performed only for sequence identifiers of which the number is reduced to reduce a processing time of a whole algorithm within 10 minutes, and accurately measure the specificity for the whole organism group. Further, the processes described above in FIG. 9 may have an additional advantage in that the processing time of the whole algorithm may be further reduced by utilizing an index data structure illustrated in FIGS. 10 to 13 to be described below.

The method according to an embodiment of the present disclosure reduces a time required for each search by utilizing a full text-indexing based database in a search process to calculate the sensitivity and specificity quickly without a large-scale calculation process such as a sequence similarity search in a whole range finally.

FIG. 10 is a flowchart exemplarily illustrating a method for generating an index data structure for searching a sequence identifier according to an embodiment of the present disclosure. A detailed description of the expressions defined and illustrated above, such as the first sequence identifier, the second sequence identifier, the first layer, the second layer, and/or the occurring frequency will be omitted.

An index is a data structure that increases a speed of an operation of a table storing data. Such an index may be generated by using one or more columns. For example, the index may be a list sorted and listed in order to quickly search a keyword in a document or sentence.

In an embodiment of the present disclosure, the index structure may be configured as a tree structure. The tree structure as a kind of graph is constituted by a node and a pointer indicating the node. Multiple nodes may not indicate one node and one node may indicate multiple nodes. When a large quantity of stored data need to be searched, a scheme that compares the data one by one is inefficient. When the data are stored in a sorted state through indexing using the tree structure, the data may be efficiently retrieved. A traverse means a series of processes of searching the index. The traverse is performed by a process of finding a next pointer by comparing a value of a search target and a division value by starting from a root node. When the index corresponding to the value of the search target is discovered through the traverse, the traverse ends.

The index according to an embodiment of the present disclosure may a B+ tree (balanced Tree) index and a bitmap index. The B+ tree index may refer to a hierarchical index technique into a root block, a branch block, and a leaf block. In the B+ tree index, the leaf block has a ROWED that can access the corresponding row of a data block, and the branch block and the root block have a key value (e.g., an address value of the block) to access each lower level. The bitmap index may refer to an index technique that stores a column value by using a bit which is the minimum unit used in a computer and automatically generates the ROWID by using the stored column value. In an embodiment of the present disclosure, the index structure may be implemented as a connection list such as the tree structure, and also implemented as an array structure such as the table. As an example, the index structure may also be configured as a data structure a B tree, a B* tree or a hash table, but an implementation form of the index structure is not limited thereto.

The index according to an embodiment of the present disclosure may comprise an inverted index. The inverted index is an index data structure that stores mapping information from contents such as separate words, numbers, or words in a specific point or a specific document of a database file. In order to efficiently perform a text search such as a full text search, the inverted index structure may be used. For example, a vector corresponding to each sequence identifier corresponding to an index word may be generated in the process of generating the inverted index, and one or more genome IDs may be mapped to each sequence identifier. The inverted index or inverted indexing in the present disclosure may be used interchangeably with a full text index or full text indexing.

In FIG. 10, a new data structure generation scheme for efficiently performing the search process of inquiring the TAX ID, the genome, and/or the sequence identifier to the annotation storage unit is described as an example.

According to an embodiment of the present disclosure, the index data structure may be generated by the processor 110 of the computing device 100 of FIG. 1, and stored in the memory 130. As another example, the index data structure may be generated by another external device of the computing device 100, received by the network unit 150 of the computing device 100, and stored in the memory 130.

According to an embodiment of the present disclosure, the index data structure may be generated by the server 220 of FIG. 2, and stored in the server 220. The index data structure may be stored and managed in the storage unit 340 in FIG. 3.

The computing device may obtain a genome and a plurality of sequence identifiers found in the genome (1010). The genome and the plurality of sequence identifiers found in the genome may be obtained from index target data. The index target data may mean data to be found in the index structure, and a genome ID of a genome and one or more sequence identifiers found in the genome may have a mapping relationship with each other. As mentioned in FIGS. 5 and 6, a table structure (reference numerals 510 and 520 in FIG. 5 or reference numeral 620 in FIG. 6) of a column structure including a column including information on the genome and one or more sequence identifiers corresponding to the genome is one example for the mapping relationship. As an example, the genome ID may be an identifier of a whole genome obtained from an external database, and the plurality of sequence identifiers may comprise base sequences found in the genomes, a base sequence for a base sequence pattern found in the genomes, a sequence identifier annotated with the base sequence pattern found in the genomes, data for the ID, and data for the sequence identifiers.

The computing device may generate a plurality of tokens by tokenizing the plurality of received sequence identifiers (1020).

The computing device may tokenize the plurality of sequence identifiers found in the index target data into the plurality of tokens. An index data structure including the plurality of tokens may be generated through tokenizing. Here, one sequence identifier may correspond to one token. In an embodiment, tokenizing may be performed based on a scheme of using space or comma which exists between the plurality of sequence identifiers as a stopword. That is, the plurality of respective sequence identifiers is divided through the space or comma in the column including the plurality of sequence identifiers. Accordingly, the space and/or comma may be used as the stopword for tokenizing each of the plurality of sequence identifiers into one token.

As illustrated in FIGS. 5 and 6, the index target data may comprise a first column representing the genome and the second column representing the plurality of sequence identifiers corresponding to the genome. The second column comprises the plurality of sequence identifiers. That is, since the plurality of sequence identifiers (e.g., a plurality of genes) may generally exist in a specific genome, the second column may not generally comprise only one datum, but may comprise a plurality of data.

That is, all whole genomes may comprise different genes (or sequence identifiers) by gene polymorphism or gene mutation, respectively even if the genomes are objects found in the same layer (the same species). In this case, the index target data may be information storing a plurality of genomes including different sequence identifiers. The method for searching and selecting the sequence identifiers according to an embodiment of the present disclosure may provide a search for the genome ID and the sequence identifiers while efficiently using the computing resources by applying a new indexing technique to the existing commercialized data structure. That is, the data structure of the existing commercialized data structure of the genome and the gene storage unit is constituted by the first column representing the genome and the second column representing the plurality of sequence identifiers corresponding to the genome. In this case, in order to compute an inclusion relationship of genomes for the plurality of respective sequence identifiers which exist a lot in the second column, a complicated operation caused by the data structure is particularly required. Resource efficiency for a new sequence identifier selection scheme may be additionally achieved through the indexing technique according to an embodiment of the present disclosure.

In the sequence identifier selecting method according to an embodiment of the present disclosure, a calculation amount used for a full search may be reduced by reducing subsequent identifiers subjected to the full search, and a usage of computing resources may be reduced by reducing the number of database I/O times utilizing the full text index data structure in the search process.

The computing device may generate an index data structure for the plurality of generated tokens (1030).

In an embodiment of the present disclosure, the index data structure may comprise a first data structure representing which source genome among the genomes in the first layer the plurality of first sequence identifiers belongs to, and a second data structure representing which source genome among the genomes in the second layer which is the higher layer than the first layer the plurality of first sequence identifiers belongs to. Here, the source genome refers a genome in which a predetermined sequence identifier appears among genomes in a predetermined layer. The source genome may refer to different genomes according to a targeted layer and the type of sequence identifier. For example, in the first data structure, a source genome is displayed, which is a genome to which the plurality of first sequence identifiers among the genomes in the first layer belongs. Further, in the second data structure, a source genome is displayed, which is a genome to which the plurality of first sequence identifiers among the genomes in the second layer belongs. For example, the first data structure may comprise data found in the column 531 and data found in the column 532 in FIG. 5. For example, the second data structure may comprise data found in the column 641 and data found in the column 642 in FIG. 6. As another example, the second data structure may comprise the data found in the column 531 and the data found in the column 532 in FIG. 5.

In an embodiment of the present disclosure, the index data structure may comprise a key and a value. For example, in the first data structure, at least some of the plurality of first sequence identifiers may correspond to the key, and a genome to which the sequence identifiers corresponding to the key in the first layer belongs may correspond to the value. For example, in the second data structure, at least some of the plurality of first sequence identifiers may correspond to the key, and a genome to which the sequence identifiers corresponding to the key in the second layer belongs may correspond to the value.

In an embodiment of the present disclosure, the index data structure may be a data structure in which index target data including a genome and a plurality of sequence identifiers found in the genome corresponding to the genome are indexed. For example, the index data structure may comprise a first column representing a tokenized sequence identifier and a second column representing one or more genomes to which the tokenized sequence identifier belongs. As another example, the index data structure may have a data structure in which one or more genomes are mapped to a single tokenized sequence identifier. As yet another example, the index data structure may be a data structure in which the tokenized sequence identifier indicates one or more genomes. As still yet another example, the index data structure may also be a data structure which stores data by using the key and the value, and data (i.e., a value of a column) and a position of data may be stored by using the key and the value.

According to an embodiment of the present disclosure, the index data structure may mean a data structure to which inverted-indexing is applied. The computing device may generate the index data structure by applying the inverted-indexing to a data structure in which a plurality of sequence identifiers are mapped to a plurality of genomes, respectively. For example, the data structure to which the inverted-indexing is applied may mean that the data structure in which the plurality of sequence identifiers are mapped to the plurality of genomes, respectively is inverted into a data structure in which the plurality of genomes are mapped to which the plurality of sequence identifiers, respectively.

In an embodiment of the present disclosure, the first data structure may be generated from a plurality of tokens for a plurality of first sequence identifiers listed to belong to each genome in the first layer. Here, one sequence identifier may correspond to one token. The second data structure may be generated from a plurality of tokens for a plurality of first sequence identifiers listed to belong to each genome in the second layer, and here, one sequence identifier may correspond to one token. The tokens may be tokenized based on a scheme of using the space or comma which exists between the plurality of first sequence identifiers as the stopword.

In an embodiment of the present disclosure, the second data structure may comprise data in a difference set of the first layer for the second layer.

In an additional embodiment of the present disclosure, the computing device may generate a first data structure in which the inverted-indexing is applied to a data structure in which the plurality of sequence identifiers are mapped to the plurality of genomes found in the first layer at which the target analyte is positioned, respectively in the biological taxonomy of the organisms having the hierarchical structure, and a second data structure in which the inverted-indexing is applied to a data structure in which the plurality of sequence identifiers are mapped to a plurality of genomes found in the second layer which is a higher layer than the first layer at which the target analyte is positioned, respectively. By utilizing the first data structure and the second data structure generated as such, the computing device may select second sequence identifiers related to the target analyte among the first sequence identifiers. For example, the computing device may select the second sequence identifiers related to the target analyte among the first sequence identifiers at least partially based on an occurring frequency in the first layer obtained from the first data structure and an occurring frequency in the second layer obtained from the second data structure with respect to the first sequence identifiers.

According to an embodiment of the present disclosure, the computing device may perform a method for selecting a sequence identifier specific to the target analyte described above in FIGS. 1 to 9 by utilizing the index data structure. When the index data structure according to an embodiment of the present disclosure is utilized, a process that computes the number of genomes including the sequence identifier may be efficiently performed, and as a result, efficient utilization of the computing resources becomes possible. The index data structure according to an embodiment of the present disclosure may enhance an operation speed of a process of computing the plurality of appearance frequencies, and reduce the complexity of the operation process.

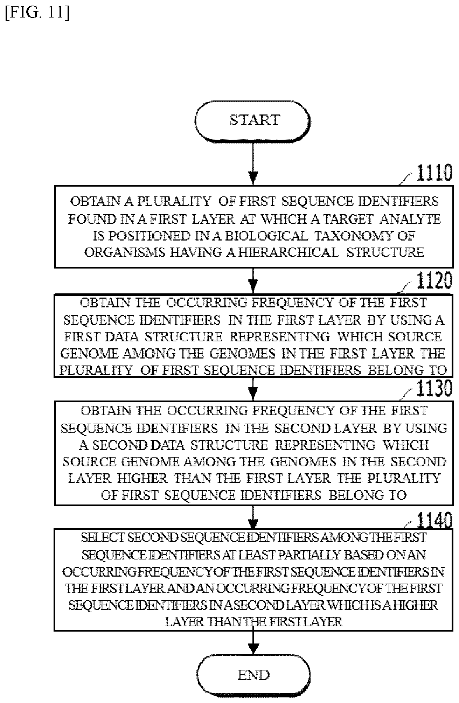

FIG. 11 is a flowchart exemplarily illustrating a method for filtering sequence identifiers by using the index data structure according to an embodiment of the present disclosure.

In the method for filtering the sequence identifiers in FIG. 11, FIGS, 7 to 9 are referenced for a description of the duplicated parts described in FIGS. 7 to 9, and the description will be omitted in FIG. 11.

The computing device may obtain a plurality of first sequence identifiers found in a first layer where a target analyte is positioned on a biological taxonomy of organisms having a hierarchical structure (1110).

As an example, the computing device inquires the Tax ID corresponding to the target analyte to the storage to obtain the plurality of first sequence identifiers related to the target analyte.

In an embodiment, the computing device may obtain the occurring frequency in the first layer for the first sequence identifiers by using a first data structure indicating which genome among the genomes in the first layer the plurality of first sequence identifiers belongs to (1120).

In obtaining the occurring frequency in the first layer, an index data structure may be used, in which the inverted-indexing is applied to a data structure in which the plurality of sequence identifiers are mapped to one genome ID. The computing device may perform a full text search for each of the first sequence identifiers by using the index data structure. For example, the computing device may obtain the occurring frequency in the first layer for the first sequence identifiers by utilizing the first data structure to which the inverted-indexing is applied. The first data structure is an index data structure indicating which source genome among the genomes in the first layer the plurality of first sequence identifiers belongs to. The first data structure is a data structure in which the inverted-indexing is applied to the data structure in which the plurality of sequence identifiers are mapped to the plurality of genomes, respectively. For example, the first data structure may comprise data found in the column 531 and data found in the column 532 in FIG. 5.

In an embodiment of the present disclosure, at least one of the first sequence identifiers may have at least one region including at least a partial region of a genic region, at least a partial region of an intergenic region, or a region including at least a part of each of a plurality of consecutive genic regions.

Genome data is connected to classification ID information for the organism groups, so a unique genome ID and classification ID information may be stored in a storage unit storing information on genomes. An annotation process of assigning a unique ID to respective genomes and respective base sequences found in the genome may be performed, and a genome ID and a sequence identifier ID may be stored in the storage unit as a result of performing the annotation process. According to an embodiment of the present disclosure, full text indexing for a data structure stored in a form of a list of unique IDs of the sequence identifiers by the unit of the genome is made. As such, in the method according to an embodiment of the present disclosure, efficiency for a process of searching a candidate gene may be maximized by reprocessing the information stored in the storage unit.

The computing device may use the index data structure in order to compute the number of genomes including the first sequence identifiers, respectively with respect to the first sequence identifiers, respectively. When an existing storage structure for the genome base sequence and gene data is used, it is almost impossible to search a candidate gene according to the organism group with respect to billions of gene data found in hundreds of thousands to millions of genome data. According to an embodiment of the present disclosure, when an index data structure of a scheme of recognizing respective sequence identifier IDs as individual tokens is utilized, a full search for all genic IDs (e.g., sequence identifier IDs) to which belong to a desired organism group may be conducted at a high speed. Accordingly, in the method according to an embodiment of the present disclosure, genomes and gene data are stored in a full text index based data structure to search a gene which may be utilized as a diagnostic marker at the high speed.

The computing device may obtain the occurring frequency in the second layer for the first sequence identifiers by using a second data structure indicating which source genome among the genomes in the second layer which is the higher layer than the first layer the plurality of first sequence identifiers belongs to (1130).

The second data structure is an index data structure indicating which source genome among the genomes in the second layer which is the higher layer than the first layer the plurality of first sequence identifiers belongs to. The second data structure is a data structure in which the inverted-indexing is applied to the data structure in which the plurality of sequence identifiers are mapped to the plurality of genomes, respectively. For example, the second data structure may comprise data found in the column 641 and data found in the column 642 in FIG. 6. As another example, the second data structure may comprise the data found in the column 531 and the data found in the column 532 in FIG. 5.

In obtaining the occurring frequency in the second layer, an index data structure may be used, in which the inverted-indexing is applied to a data structure in which the plurality of sequence identifiers are mapped to one genome ID. The computing device may perform a full text search for each of the first sequence identifiers by using the index data structure. For example, the computing device may obtain the occurring frequency in the second layer for the first sequence identifiers by utilizing the first data structure to which the inverted-indexing is applied.

In an additional embodiment of the present disclosure, the computing device may select, as the second sequence identifiers, sequence identifiers in which the occurring frequency in the first layer for each of the first sequence identifiers is equal to or more than a first threshold and the occurring frequency in the second layer for each of the first sequence identifiers is equal to or less than a second threshold, in selecting the second sequence identifiers. Here, the first threshold may mean a minimum reference value for the occurring frequency in the first layer of each of the first sequence identifiers, and the second threshold may mean a maximum reference value for the occurring frequency in the second layer for each of the first sequence identifiers.

As described above, the sequence identifier related to the target analyte may be efficiently selected by using the first threshold and/or the second threshold in addition to the index data structure. Here, the selected second sequence identifier(s) may mean a sequence identifier(s) in which a sequence identifier of a specific organism in which exclusivity is predetermined is removed among the sequence identifiers in which the occurring frequency in the first layer for each of the first sequence identifiers is equal to or more than the first threshold and the occurring frequency in the second layer for each of the first sequence identifiers is equal to or less than the second threshold. As an example, a sequence identifier of a predetermined specific organism may comprise a sequence identifier found in resident flora. As described above, the technique according to an embodiment of the present disclosure may select the sequence identifier by a more efficient scheme by removing the sequence identifier found in the resident flora.

In a technique according to an additional embodiment of the present disclosure, since the first threshold and/or the second threshold may be adaptively changed, sequence identifiers of a number suitable for a user's need may be selected. For example, the computing device may change at least one of the first threshold or the second threshold when the number of first sequence identifiers in which the occurring frequency in the first layer for each of the first sequence identifiers is equal to or more than the first threshold, and the occurring frequency in the second layer for each of the first sequence identifiers is equal to or less than the second threshold is less than a predetermined number, or when the number of first sequence identifiers in which the occurring frequency in the first layer for each of the first sequence identifiers is equal to or more than the first threshold, and the occurring frequency in the second layer for each of the first sequence identifiers is equal to or less than the second threshold is more than the predetermined number in selecting the second sequence identifiers.

In an embodiment of the present disclosure, when the occurring frequency is obtained, the full text search may be used. The full text search as one of natural language searches may be a search for returning a document or data which matches a text found in a query, and for example, when the full text search is performed in response to a query including a specific sequence identifier ID, a result indicating how many times a specific sequence identifier appears in a list of genomes in a specific range may be returned.

The computing device may select a second sequence identifier related to the target analyte among the first sequence identifiers at least partially based on the occurring frequency in the first layer and the occurring frequency in the second layer with respect to each of the first sequence identifiers (1140). Since the appearance frequencies may be computed at the high speed by utilizing the index data structure (e.g., the first data structure and/or the second data structure) to which the inverted-indexing technique is applied, the speed of the process of selecting the second sequence identifiers among the first sequence identifiers will also be enhanced.

In FIG. 11, for convenience of description, examples for a process of selecting the second sequence identifiers based on the occurring frequency in the first layer for each of the first sequence identifiers and the occurring frequency in the second layer for each of the first sequence identifiers are described. As the index data structure is utilized in the process of computing the occurring frequency in the third layer for each the second sequence identifiers, the occurring frequency in the first layer for each of the second sequence identifiers, and/or the occurring frequency in the second layer for each of the second sequence identifiers, the speed of a process of selecting at least one third sequence identifier among the second sequence identifiers may also be enhanced.

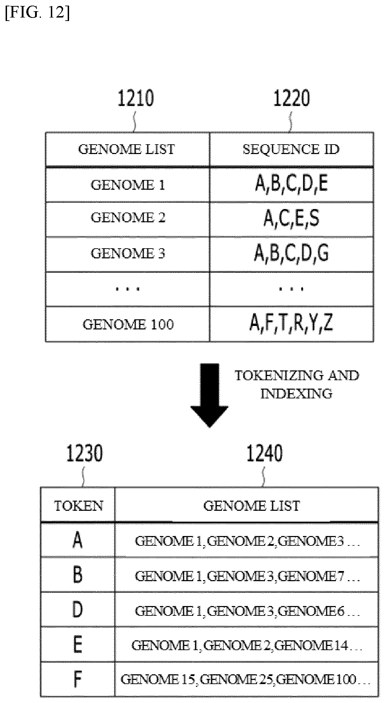

FIG. 12 illustrates an exemplary index data structure according to an embodiment of the present disclosure.

An index target table according to an embodiment of the present disclosure may comprise a first column 1210 representing genome IDs and a second column 1220 representing sequence identifiers mapped to the genome IDs, respectively. The index data structure may be generated based on data found in the first column 1210 and the second column 1220.

According to an embodiment of the present disclosure, inverted-indexing for the index target table may be performed. The inverted-indexing may be made by a scheme in which the data structure in which the plurality of sequence identifiers are mapped to the plurality of genomes, respectively is inverted into the data structure in which the plurality of genomes are mapped to which the plurality of sequence identifiers, respectively. In the inverted-indexing process, a tokenizing process and an indexing process for the data found in the second column 1220 may be performed. The tokenizing process may comprise a segmentation process for text data. During the tokenizing process, the text data may be segmented into a plurality of tokens according to a specific reference. The indexing process may comprise generating an index data structure including position information, mapping information and/or connection information for each of the plurality of tokens segmented through the tokenizing process. A separate index object including reference numerals 1230 and 1240 may be generated through the indexing process, and after the index object is generated, the search speed may be increased as the generated index object is utilized in the process of searching the sequence identifier.

For example, sequence identifiers corresponding to genome 1 are A, B, C, D, and E. The sequence identifiers may be segmented into a plurality of tokens based on the stopword such as the space or comma. Each sequence identifier corresponding to genome 1 may correspond to one token. After each sequence identifier is tokenized based on the space or comma, a column 1230 expressed by reference numeral 1230 may be generated based on each token. The computing device inserts information (e.g., a genome ID, a genome address, etc.) on the genomes including the tokens found in the generated column 1230 into a column 1240 expressed by reference numeral 1240 to generate an index data structure including the column 1230 and the column 1240. In such an example, data of the column 1230 may correspond to the key in the index data structure, and data of the column 1240 may correspond to the value in the index data structure.

For example, the index data structure may comprise a first data structure and a second data structure. The first data structure may represent which genome among the genomes in the first layer the first sequence identifiers belong to, and the second data structure may represent which genome among the genomes in the second layer the first sequence identifiers belong to.

In an embodiment, the first data structure and the second data structure may have a data structure of a form illustrated in FIG. 12 as an example, and may have different stored data values due to a difference in the layer. For example, the first data structure is an index data structure using the data found in the column 531 as the key and the data found in the column 532 as the value in FIG. 5. For example, the second data structure is an index data structure using the data found in the column 641 as the key and the data found in the column 642 as the value in FIG. 6. The occurring frequency (e.g., the value found in the column 533 in FIG. 5) in the first layer for the first sequence identifiers may be efficiently obtained by using the first data structure. Further, the occurring frequency (e.g., the value found in the column 643 in FIG. 6) in the second layer for the first sequence identifiers may be efficiently obtained by using the second data structure. The index data structures for the first layer for the first sequence identifiers and the second layer for the first sequence identifiers are described, but it will also be apparent to those skilled in the art that the plurality of appearance frequencies may be calculated by using the respective index data structures to which the inverted-indexing is applied.

When the index data structure is generated, information on or the number of genomes including a specific sequence identifier in response to a query for a specific sequence identifier may be obtained in the process of computing the plurality of appearance frequencies. When the index data structure is used, unnecessary computing resources may be prevented from being used in the full text search process. Through the index data structure according to an embodiment of the present disclosure, in the process of searching the gene ID for a whole database, the computing device may inquire, with respect to each gene, genomes including the corresponding gene and genomes not including the corresponding gene within several seconds.

In the method according to an embodiment of the present disclosure, the inverted-indexing technique using the space and/or comma which exist between the sequence identifiers as the stopword is taken as an example, but an N-gram technique of setting a token having a length of N may also be found in the indexing technique according to the embodiment of the present disclosure. The index data structure according to an embodiment of the present disclosure may comprise any type of index structure that may utilize the full text search for computing the occurring frequency for the sequence identifier ID.

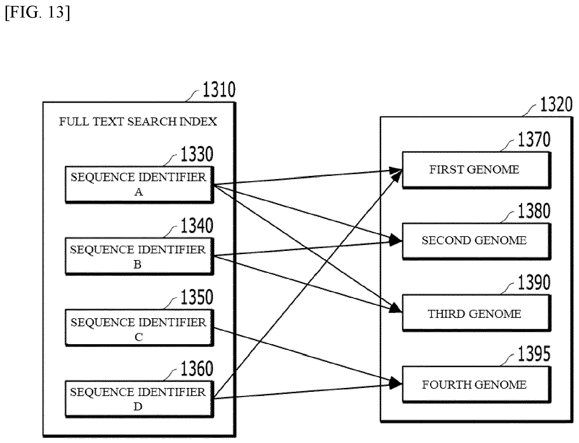

FIG. 13 exemplarily illustrates a conceptual view of performing a full text search according to an embodiment of the present disclosure.

A full text search index 1310 may comprise an index data structure to which the inverted-indexing technique is applied for the full text search. As illustrated in FIG. 13, the full text search index 1310 may comprise a link pointing an object 1320 including genomes 1370, 1380, 1390, and 1396 with respect to sequence identifiers 1330, 1340, 1350, and 1360, respectively.

For example, since sequence identifier A 1330 comprises a first genome 1370, a second genome 1380, and a third genome 1390, the full text search index 1310 may comprise link information for pointing a first genome 1370, a second genome 1380, and a third genome 1390 for sequence identifier A 1330.

Further, sequence identifier B 1340 is a sequence identifier found in the second genome 1380 and the third genome 1390. The full text search index 1310 may store link information pointing objects for the second genome 1380 and the third genome 1390 among the objects 1320 in the database jointly with sequence identifier B 1340.

As illustrated in FIG. 13, since the computing device may determine information on genomes including one sequence identifier through the index data structure more rapidly, a performing speed for the algorithm of selecting the sequence identifier may be increased rapidly.

When the database stores a large amount of data such as genome information and gene information, it may take a relatively long time to perform a query to retrieve data in which a user is interested. When it takes a long time for the database to respond to the query, numerous I/Os are performed, which may adversely affect performance of the database.

In the method according to an embodiment of the present disclosure, the full text indexing technique or the inverted-indexing technique is applied to the genome ID and the sequence identifier ID to rapidly search the sequence identifier specific to the target analyte in a storage containing vast genome data. In the method according to an embodiment of the present disclosure, by reducing the time processed to respond to the query, the performance of a database management system may also be enhanced.

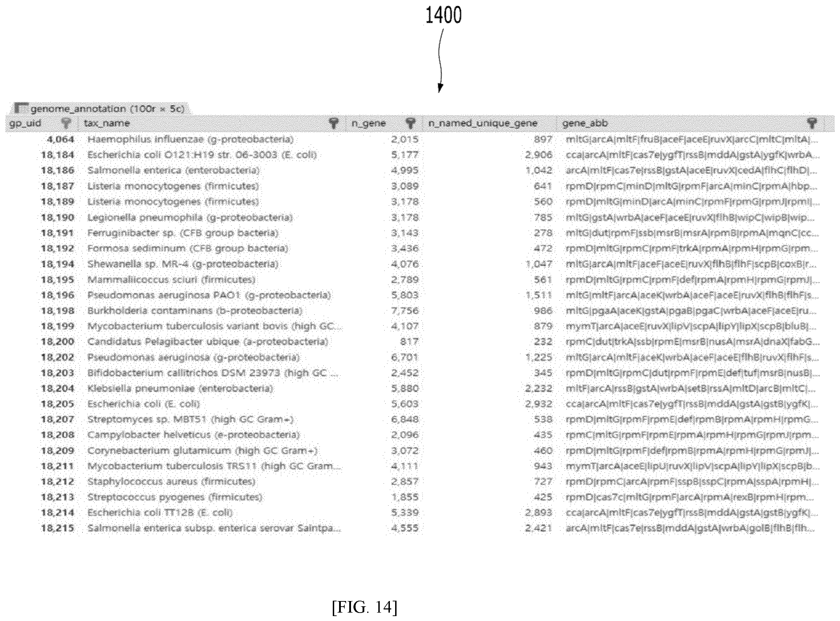

FIG. 14 exemplarily illustrates an inverted-indexing technique according to an embodiment of the present disclosure.

As illustrated FIG. 14, according to an embodiment of the present disclosure, in a gene annotation DB, the inverted-indexing technique may be performed by using a gene name itself.

Reference numeral 1400 exemplarily illustrates data obtained by the annotation DB. The above-described data may be expressed as a table format as in the example in FIG. 14, but may also be expressed as any type of object other than the table.

The data illustrated in reference numeral 1400 may comprise the number of genes (n_gene) corresponding to a name a target detection object (tax_name) detected in the annotation DB. Further, values found in a column expressed as gene_abb in reference numeral 1400 may represent a gene name (or gene ID). For example, the above-described gene name may represent a known gene abbreviation named in the NCBI. As another example, the gene name may also represent a code type gene ID which is arbitrarily assigned.

Further, n_named_unique_gene may represent the number of named unique genes.

In the example illustrated in reference numeral 1400, one or more gene IDs found in each target detection object or Tax ID are displayed. When the above-described inverted-indexing technique is applied according to an embodiment of the present disclosure, the index data structure (e.g., the full text search index) may be generated in the form of being capable of determining the information of the genome, information of the Tax ID, and/or the number of genomes based on each gene ID. As such, the full text indexing technique or the inverted-indexing technique is applied to the gene ID and/or the sequence identifier ID to derive a technical effect of rapidly searching the sequence identifier specific to the target analyte in the storage containing vast genome data.

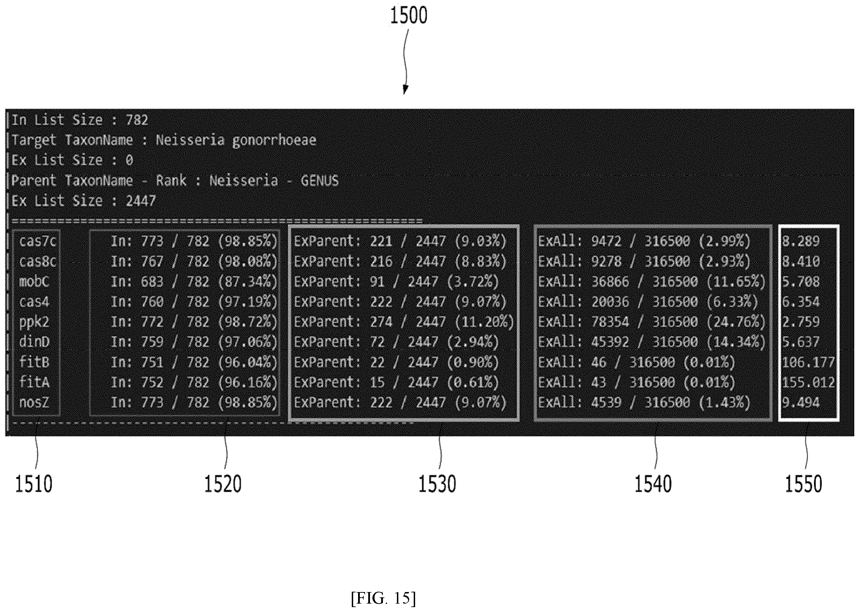

FIG. 15 exemplarily illustrates a sequence identifier selection result according to an embodiment of the present disclosure.

Reference numeral 1500 in FIG. 15 illustrates an implementation example showing a selection result of the sequence identifiers.

In List Size in FIG. 15 may represent the number of genomes found inin the first layer. Target TaxonName in FIG. 15 may represent Neisseria gonorrhoeae. That is, the first layer may correspond to Neisseria gonorrhoeae species to which Neisseria gonorrhoeae belongs, and Neisseria Genus expressed by Parent TaxoName may correspond to the second layer.

In an embodiment of the present disclosure, the third layer corresponding to the highest layer may correspond to bacteria domain.

As another example, according to an implementation aspect, the second layer or the third layer may also correspond to at least one of Neisseriaceae, Neisseriales, Betaproteobactria, or Proteobacteria.

Ex List Size in reference numeral 1500 may represent the number of genomes found inin the third layer (e.g., bacteria domain).

Reference numeral 1510 represents the list of the sequence identifiers. An occurring frequency 1520 in the first layer, an occurring frequency 1530 in the second layer, an occurring frequency 1540 in the third layer, and a score 1550 according to Equation 1 may be computed with respect to each of sequence identifiers such as cas7c, cas8c, and mobC

Reference numeral 1520 represents an occurring frequency in the first layer for each of the sequence identifiers disclosed in reference numeral 1510. For example, a sequence identifier named as cas7c is a sequence identifier found in 773 genomes among a total of 782 genomes found inin the first layer corresponding to the Neisseria gonorrhoeae species. As such, an occurring frequency computed for cas7c may be exemplified as 98.85% (or 0.9885).

Reference numeral 1530 represents an occurring frequency in the second layer for each of the sequence identifiers disclosed in reference numeral 1510. Reference numeral 1540 represents an occurring frequency in the third layer for each of the sequence identifiers disclosed in reference numeral 1510. In FIG. 15, sequence identifiers cas7c to nosZ 1510 are illustrated. The sequence identifier 1510 corresponds to some of the sequence identifiers found in Neisseria gonorrhoeae target analyte. Specifically, the sequence identifier 1510 may be a second sequence identifier 1510 primarily filtered based on the occurring frequency 1520 in the first layer and the occurring frequency 1530 in the second layer. That is, all sequence identifiers found in Neisseria gonorrhoeae are the first sequence identifiers, and among them the second sequence identifier 1510 may be selected by primary filtering based on the occurring frequency 1520 in the first layer and the occurring frequency 1530 in the second layer.

Further, the occurring frequency 1540 in the third layer for each of the second sequence identifiers 1510 may be computed. A score 1550 may be computed based on the occurring frequency 1520 in the first layer, the occurring frequency 1530 in the second layer, and the occurring frequency 1540 in the third layer computed as such.

Specifically, as in the case of cas7c, the occurring frequency in the first layer may have a relatively high value, but the occurring frequency in the second layer is expressed as 9.03% and the occurring frequency in third layer is expressed as 2.99%, cas7c may have a relatively higher second layer or third layer related occurring frequency value than other sequence identifiers. In this case, the sequence identifier of cas7c may pass through a first threshold related to the occurring frequency in the first layer, but not pass through a second threshold related to the occurring frequency in the second layer (or a third threshold related to the occurring frequency in the third layer), so there is a high possibility that cas7c will not be selected as a final third sequence identifier.

As described above, a sequence identifier in which the occurring frequency in the first layer is high, and the occurring frequency in the second layer and the occurring frequency in the third layer are low may be selected as a sequence identifier which is likely to be specific to the target detection object (e.g., Neisseria gonorrhoeae).

Reference numeral 1550 illustrates a score value acquired by applying Equation 1 described above to each of the sequence identifiers found in reference numeral 1510. As illustrated, fitB and fitA which are sequence identifiers having relatively high score values may be finally determined as the third sequence identifiers. In an additional embodiment, in addition to the scoring scheme based on Equation 1, a scheme of selecting the third sequence identifier may also be possible at least partially based on values expressed in a 1530 box and/or a 1540 box.

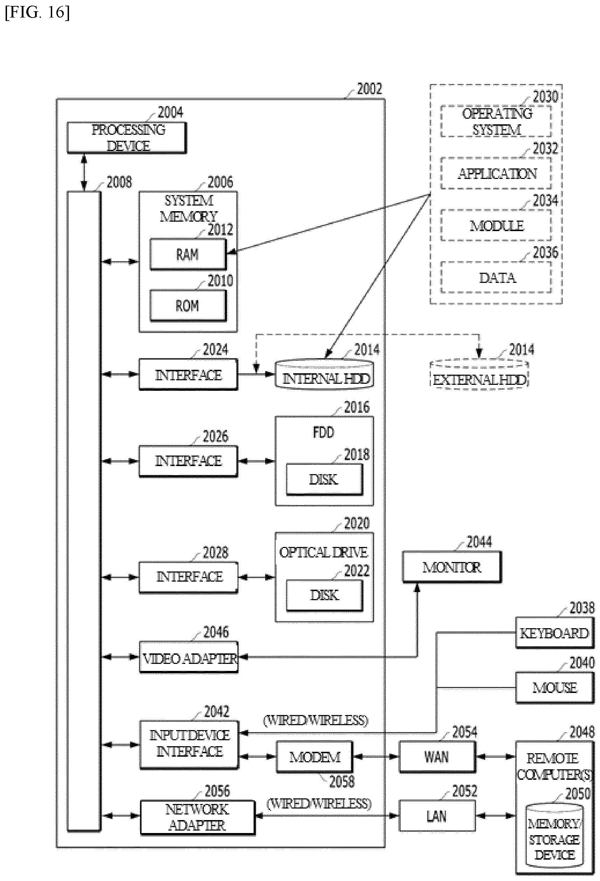

Fig. 16 is a general schematic view of an exemplary computing environment in which exemplary embodiments of the present disclosure may be implemented.

In the present disclosure, a component, a module or a unit comprises a routine, a procedure, a program, a component, a data structure, and the like that execute a specific task or implement a specific abstract data type. Further, it will be well appreciated by those skilled in the art that the method of the present disclosure can be implemented by other computer system configurations including a personal computer, a handheld computing device, microprocessor-based or programmable home appliances, and others (the respective devices may operate in connection with one or more associated devices as well as a single-processor or multi-processor computer system, a mini computer, and a main frame computer.

The exemplary embodiments described in the present disclosure may also be implemented in a distributed computing environment in which predetermined tasks are performed by remote processing devices connected through a communication network. In the distributed computing environment, the program module may be positioned in both local and remote memory storage devices.

The computer generally includes various computer readable media. The computer includes, as a computer accessible medium, volatile and non-volatile media, transitory and non-transitory media, and mobile and non-mobile media. As a non-limiting example, the computer readable media may include both computer readable storage media and computer readable transmission media.

The computer readable storage media include volatile and non-volatile media, transitory and non-transitory media, and mobile and non-mobile media implemented by a predetermined method or technology for storing information such as a computer readable instruction, a data structure, a program module, or other data. The computer readable storage media include a RAM, a ROM, an EEPROM, a flash memory or other memory technologies, a CD-ROM, a digital video disk (DVD) or other optical disk storage devices, a magnetic cassette, a magnetic tape, a magnetic disk storage device or other magnetic storage devices or predetermined other media which may be accessed by the computer or may be used to store desired information, but are not limited thereto.

The computer readable transmission media generally implement the computer readable instruction, the data structure, the program module, or other data in a carrier wave or a modulated data signal such as other transport mechanism and include all information transfer media. The term "modulated data signal" means a signal acquired by setting or changing at least one of characteristics of the signal so as to encode information in the signal. As a non-limiting example, the computer readable transmission media include wired media such as a wired network or a direct-wired connection and wireless media such as acoustic, RF, infrared and other wireless media. A combination of any media among the aforementioned media is also included in the computer readable transmission media.

An exemplary environment 2000 that implements various aspects of the present disclosure including a computer 2002 is shown and the computer 2002 includes a processing device 2004, a system memory 2006, and a system bus 2008. The system bus 2008 connects system components including the system memory 2006 (not limited thereto) to the processing device 2004. The processing device 2004 may be a predetermined processor among various commercial processors. A dual processor and other multi-processor architectures may also be used as the processing device 2004.

The system bus 2008 may be any one of several types of bus structures which may be additionally interconnected to a local bus using any one of a memory bus, a peripheral device bus, and various commercial bus architectures. The system memory 2006 includes a read only memory (ROM) 2010 and a random access memory (RAM) 2012. A basic input/output system (BIOS) is stored in the non-volatile memories 2010 including the ROM, the EPROM, the EEPROM, and the like and the BIOS includes a basic routine that assists in transmitting information among components in the computer 2002 at a time such as in-starting. The RAM 2012 may also include a high-speed RAM including a static RAM for caching data, and the like.

The computer 2002 also includes an internal hard disk drive (HDD) 2014 (for example, EIDE and SATA) - the internal hard disk drive 2014 may also be configured for an external purpose in an appropriate chassis (not illustrated), a magnetic floppy disk drive (FDD) 2016 (for example, for reading from or writing in a mobile diskette 2018), and an optical disk drive 2020 (for example, for reading a CD-ROM disk 2022 or reading from or writing in other high-capacity optical media such as the DVD). The hard disk drive 2014, the magnetic disk drive 2016, and the optical disk drive 2020 may be connected to the system bus 2008 by a hard disk drive interface 2024, a magnetic disk drive interface 2026, and an optical disk drive interface 2028, respectively. An interface 2024 for implementing an external drive includes, for example, at least one of a universal serial bus (USB) and an IEEE 1394 interface technology or both of them.

The drives and the computer readable media associated therewith provide non-volatile storage of the data, the data structure, the computer executable instruction, and others. In the case of the computer 2002, the drives and the media correspond to storing of predetermined data in an appropriate digital format. In the description of the computer readable storage media, the mobile optical media such as the HDD, the mobile magnetic disk, and the CD or the DVD are mentioned, but it will be well appreciated by those skilled in the art that other types of storage media readable by the computer such as a zip drive, a magnetic cassette, a flash memory card, a cartridge, and others may also be used in an exemplary operating environment and further, the predetermined media may include computer executable instructions for executing the methods of the present disclosure.

Multiple program modules including an operating system 2030, one or more application programs 2032, other program module 2034, and program data 2036 may be stored in the drive and the RAM 2012. All or some of the operating system, the application, the module, and/or the data may also be cached in the RAM 2012. It will be well appreciated that the present disclosure may be implemented in operating systems which are commercially usable or a combination of the operating systems.

A user may input instructions and information in the computer 2002 through one or more wired/wireless input devices, for example, pointing devices such as a keyboard 2038 and a mouse 2040. Other input devices (not illustrated) may include a microphone, an IR remote controller, a joystick, a game pad, a stylus pen, a touch screen, and others. These and other input devices are often connected to the processing device 2004 through an input device interface 2042 connected to the system bus 2008, but may be connected by other interfaces including a parallel port, an IEEE 1394 serial port, a game port, a USB port, an IR interface, and others.

A monitor 2044 or other types of display devices are also connected to the system bus 2008 through interfaces such as a video adapter 2046, and the like. In addition to the monitor 2044, the computer generally includes other peripheral output devices (not illustrated) such as a speaker, a printer, others.

The computer 2002 may operate in a networked environment by using a logical connection to one or more remote computers including remote computer(s) 2048 through wired and/or wireless communication. The remote computer(s) 2048 may be a workstation, a server computer, a router, a personal computer, a portable computer, a microprocessor based entertainment apparatus, a peer device, or other general network nodes and generally includes multiple components or all of the components described with respect to the computer 2002, but only a memory storage device 2050 is illustrated for brief description. The illustrated logical connection includes a wired/wireless connection to a local area network (LAN) 2052 and/or a larger network, for example, a wide area network (WAN) 2054. The LAN and WAN networking environments are general environments infices and companies and facilitate an enterprise-wide computer network such as Intranet, and all of them may be connected to a worldwide computer network, for example, the Internet.

When the computer 2002 is used in the LAN networking environment, the computer 2002 is connected to a local network 2052 through a wired and/or wireless communication network interface or an adapter 2056. The adapter 2056 may facilitate the wired or wireless communication to the LAN 2052 and the LAN 2052 also includes a wireless access point installed therein order to communicate with the wireless adapter 2056. When the computer 2002 is used in the WAN networking environment, the computer 2002 may include a modem 2058, is connected to a communication server on the WAN 2054, or has other means that configure communication through the WAN 2054 such as the Internet, etc. The modem 2058 which may be an internal or external and wired or wireless device is connected to the system bus 2008 through the serial port interface 2042. In the networked environment, the program modules described with respect to the computer 2002 or some thereof may be stored in the remote memory/storage device 2050. It will be well known that an illustrated network connection is exemplary and other means configuring a communication link among computers may be used.

The computer 2002 performs an operation of communicating with predetermined wireless devices or entities which are disposed and operated by the wireless communication, for example, the printer, a scanner, a desktop and/or a portable computer, a portable data assistant (PDA), a communication satellite, predetermined equipment or place associated with a wireless detectable tag, and a telephone. This at least includes wireless fidelity (Wi-Fi) and Bluetooth wireless technology. Accordingly, communication may be a predefined structure like the network in the related art or just ad hoc communication between at least two devices.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

[Mode for Invention]

Related contents in the best mode for carrying out the present disclosure are described as above.

[Industrial Applicability]

The present disclosure may be used in an apparatus, a system, etc., that select a sequence identifier specific to a target analyte in order to detect an analyte.

**Claims**

1. A method for selecting a sequence identifier for detecting a target analyte, which is performed by a computing device, the method comprising:

   obtaining a plurality of first sequence identifiers found in a first layer at which a target analyte is positioned in a biological taxonomy of organisms having a hierarchical structure;
   selecting second sequence identifiers among the first sequence identifiers at least partially based on an occurring frequency of the first sequence identifiers in the first layer and an occurring frequency of the first sequence identifiers in a second layer which is a higher layer than the first layer; and
   selecting a third sequence identifier for detecting the target analyte among the second sequence identifiers at least partially based on an occurring frequency of the second sequence identifiers inthe first layer and an occurring frequency of the second sequence identifiers in a third layer which is a higher layer than the second layer.

2. The method for selecting a sequence identifier for detecting a target analyte of claim 1, wherein the selecting of the second sequence identifiers comprises

   obtaining the occurring frequency of the first sequence identifiers in the first layer by using the number of genomes to which the first sequence identifiers belong among first genomes found in the first layer, and
   obtaining the occurring frequency of the first sequence identifiers in the second layer by using the number of genomes to which the first sequence identifiers belong among second genomes found in the second layer, and wherein the selecting of the third sequence identifiers comprises
obtaining the occurring frequency of the second sequence identifiers in the first layer by using the number of genomes to which the second sequence identifiers belong among the first genomes found in the first layer, and obtaining the occurring frequency of the second sequence identifiers in the third layer by using the number of genomes to which the second sequence identifiers belong among third genomes found in the third layer.

3.  The method for selecting a sequence identifier for detecting a target analyte of claim 2, wherein the obtaining of the occurring frequency of the first sequence identifiers in the second layer comprises
obtaining the occurring frequency of the first sequence identifiers in which the first genomes are excluded from the second genomes found in the second layer.

4.  The method for selecting a sequence identifier for detecting a target analyte of claim 3, wherein the obtaining of the occurring frequency of the second sequence identifiers in the third layer comprises
obtaining the occurring frequency of the second sequence identifiers in which the first genomes are excluded from the third genomes found in the third layer or in which the second genomes are excluded from the third genomes.

5.  The method for selecting a sequence identifier of claim 1, wherein the selecting of the second sequence identifiers comprises
calculating the occurring frequency of the first sequence identifiers in a difference set of the second layer.

6.  The method for selecting a sequence identifier of claim 1, wherein the selecting of the third sequence identifier comprises
calculating an occurring frequency of the second sequence identifiers in a difference set of the first layer for the third layer or a difference set of the second layer for the third layer.

7.  The method for selecting a sequence identifier for detecting a target analyte of claim 1, wherein the selecting of the second sequence identifiers comprises

    selecting, as the second sequence identifiers, sequence identifiers in which the occurring frequency of each of the first sequence identifiers in the first layer is equal to or more than a first threshold and the occurring frequency of each of the first sequence identifiers in the second layer is equal to or less than a second threshold, and wherein the first threshold is a minimum reference value for the occurring frequency of each of the first sequence identifiers in the first layer, and the second threshold is a maximum reference value for the occurring frequency of each of the first sequence identifiers in the second layer.

8.  The method for selecting a sequence identifier for detecting a target analyte of claim 7, wherein the selecting of the second sequence identifiers comprises
changing at least one of the first threshold or the second threshold when the number of the sequence identifiers is less than a predetermined number or more than the predetermined number.

9.  The method for selecting a sequence identifier for detecting a target analyte of claim 1, wherein the selecting of the second sequence identifiers further comprises
removing a sequence identifier of a specific organism from the selected second sequence identifiers in which the specific organism is predetermined as exclusivity.

10. The method for selecting a sequence identifier for detecting a target analyte of claim 9, wherein the predetermined sequence identifier comprises a sequence identifier found in a resident flora or a housekeeping gene.

11. The method for selecting a sequence identifier for detecting a target analyte of claim 1, wherein the selecting of the third sequence identifier comprises

    selecting, as the third sequence identifiers, sequence identifiers in which the occurring frequency of each of the second sequence identifiers in the third layer is equal to or less than a third threshold and the occurring frequency of each of the second sequence identifiers in the first layer is equal to or more than a fourth threshold, and wherein the third threshold is a maximum reference value for the occurring frequency of each of the second sequence identifiers in the third layer, and the fourth threshold is a minimum reference value for the occurring frequency of each of the second sequence identifiers in the first layer.

**12.** The method for selecting a sequence identifier for detecting a target analyte of claim 1, wherein the selecting of the third sequence identifier further comprises

removing the sequence identifier of the specific organism from the selected third sequence identifiers in which the specific organism is predetermined as exclusivity.

**13.** The method for selecting a sequence identifier for detecting a target analyte of claim 12, wherein the predetermined sequence identifier comprises a sequence identifier found in a resident flora or a housekeeping gene.

**14.** The method for selecting a sequence identifier for detecting a target analyte of claim 1, wherein the selecting of the third sequence identifier comprises

selecting the third sequence identifier for detecting the target analyte among the second sequence identifiers by further considering the occurring frequency of each of the second sequence identifiers in the second layer.

**15.** The method for selecting a sequence identifier for detecting a target analyte of claim 14, wherein the selecting of the third sequence identifier comprises

selecting the third sequence identifier among the second sequence identifiers by calculating a score of each of the second sequence identifiers based on the occurring frequency of each of the second sequence identifiers in the third layer, the occurring frequency of each of the second sequence identifiers in the first layer, and the occurring frequency of each of the second sequence identifiers in the second layer, and

wherein the more the occurring frequency of each of the second sequence identifiers in the first layer, a higher score is calculated, the lower the occurring frequency of each of the second sequence identifiers in the third layer, a higher score is calculated, and the lower the occurring frequency of each of the second sequence identifiers in the second layer, a higher score is calculated.

**16.** The method for selecting a sequence identifier for detecting a target analyte of claim 15, wherein the score is calculated by

[Equation 1]

$$Score = \frac{\left(\frac{Fin}{Gin}\right) \times \left(\frac{Gtotal + Gin}{Gtotal}\right)}{\left(\frac{Fexp}{Gexp} \div \frac{Gtotal + Gexp}{Gtotal}\right) + \frac{Fext}{Gext}},$$

wherein Fin is the number of genomes having a second sequence identifier in the first layer, Gin is the number of genomes found in the first layer, Gtotal is the number of genomes found in the third layer, Fexp is the number of genomes having a second sequence identifier in the second layer, Fext is the number of genomes having a second sequence identifier in the third layer, Gexp is the number of genomes found in a difference of a set of the second layer and a set of the first layer, and Gext is the number of genomes found in a difference of a set of the third layer and a set of the first layer or a difference of a set of the third layer and a set of the second layer.

**17.** The method for selecting a sequence identifier for detecting a target analyte of claim 1, wherein the hierarchical structure is a biological systematic structure in which a higher layer encompasses a lower layer.

**18.** The method for selecting a sequence identifier for detecting a target analyte of claim 1, wherein the second layer has a hierarchical position immediately higher from the first layer and the second layer has the number of genomes being more than a threshold number.

**19.** The method for selecting a sequence identifier for detecting a target analyte of claim 1, wherein the third layer is a highest layer in the hierarchical structure.

20. The method for selecting a sequence identifier for detecting a target analyte of claim 1, wherein the third sequence identifier is a candidate sequence identifier to be considered as a specific sequence identifier used for detecting the target analyte.

21. The method for selecting a sequence identifier for detecting a target analyte of claim 1, wherein the occurring frequency of the first sequence identifiers in the first layer is obtained from a first data structure representing which source genome among the genomes in the first layer the plurality of first sequence identifiers belongs to.

22. The method for selecting a sequence identifier for detecting a target analyte of claim 21, wherein the occurring frequency of the first sequence identifiers in the second layer is obtained from a second data structure representing which source genome among the genomes in the second layer the plurality of first sequence identifiers belongs to.

23. A computing device for selecting a sequence identifier for detecting a target analyte, comprising:

a memory; and
a processor, and
wherein the processor obtains a plurality of first sequence identifiers found in a first layer at which a target analyte is positioned in a biological taxonomy of organisms having a hierarchical structure;
selects second sequence identifiers among the first sequence identifiers at least partially based on an occurring frequency of the first sequence identifiers in the first layer and an occurring frequency of the first sequence identifiers in a second layer which is a higher layer than the first layer; and
selects a third sequence identifier for detecting the target analyte among the second sequence identifiers at least partially based on an occurring frequency of the second sequence identifiers inthe first layer and an occurring frequency of the second sequence identifiers in a third layer which is a higher layer than the second layer.

24. A method for selecting a sequence identifier for detecting a target analyte, which is performed by a computing device, the method comprising:

obtaining a plurality of first sequence identifiers found in a first layer at which a target analyte is positioned in a biological taxonomy of organisms having a hierarchical structure;
obtaining the occurring frequency of the first sequence identifiers in the first layer by using a first data structure representing which source genome among the genomes in the first layer the plurality of first sequence identifiers belong to;
obtaining the occurring frequency of the first sequence identifiers in the second layer by using a second data structure representing which source genome among the genomes in the second layer higher than the first layer the plurality of first sequence identifiers belong to; and
selecting second sequence identifiers for detecting the target analyte among the first sequence identifiers at least partially based on an occurring frequency of the first sequence identifiers in the first layer and an occurring frequency of the first sequence identifiers in a second layer.

25. The method for selecting a sequence identifier for detecting a target analyte of claim 24, wherein the first data structure, the plurality of first sequence identifiers corresponds to a key, and a genome to which the sequence identifiers as the key in the first layer belong corresponds to a value.

26. The method for selecting a sequence identifier for detecting a target analyte of claim 24, wherein the second data structure, the plurality of first sequence identifiers corresponds to a key, and a genome to which the sequence identifiers as the key in the second layer belong corresponds to a value.

27. The method for selecting a sequence identifier for detecting a target analyte of claim 24, wherein the first data structure and the second data structure are data structures in which inverted-indexing is applied to a data structure having a plurality of sequence identifiers mapped to each of a plurality of genomes.

28. The method for selecting a sequence identifier for detecting a target analyte of claim 27, wherein the inverted-indexing generates a data structure in which the plurality of genomes are mapped to each of the plurality of sequence identifiers.

29. The method for selecting a sequence identifier for detecting a target analyte of claim 24, wherein the first data structure is generated from a plurality of tokens for a plurality of first sequence identifiers belonging to each genome in the first layer, and wherein one sequence identifier corresponds to one token.

30. The method for selecting a sequence identifier for detecting a target analyte of claim 24, wherein the second data structure is generated from a plurality of tokens for a plurality of first sequence identifiers belonging to each genome in the second layer, and wherein one sequence identifier corresponds to one token.

31. The method for selecting a sequence identifier for detecting a target analyte of claim 29 or 30, wherein the token is generated by tokenization using space or comma as a stopword that exists in an array of the plurality of the first sequence identifiers.

32. The method for selecting a sequence identifier for detecting a target analyte of claim 24, wherein the second data structure comprises data in a difference of a set of the second layer and a set of the first layer.

33. The method for selecting a sequence identifier for detecting a target analyte of claim 24, wherein at least one of the plurality of first sequence identifiers is at least partial region of a genic region and/or at least partial region of an intergenic region.

34. The method for selecting a sequence identifier for detecting a target analyte of claim 24, wherein the selecting of the second sequence identifiers comprises

selecting, as the second sequence identifiers, sequence identifiers in which the occurring frequency of each of the first sequence identifiers in the first layer is equal to or more than a first threshold and the occurring frequency of each of the first sequence identifiers in the second layer is equal to or less than a second threshold, and wherein the first threshold is a minimum reference value for the occurring frequency of each of the first sequence identifiers in the first layer , and the second threshold is a maximum reference value for the occurring frequency of each of the first sequence identifiers in the second layer .

35. The method for selecting a sequence identifier for detecting a target analyte of claim 34, wherein the selecting of the second sequence identifiers comprises
changing at least one of the first threshold or the second threshold when the number of the sequence identifiers is less than a predetermined number or more than the predetermined number.

36. The method for selecting a sequence identifier for detecting a target analyte of claim 24, wherein the selecting of the second sequence identifiers further comprises
removing a sequence identifier of a specific organism from the selected second sequence identifiers in which the specific organism is predetermined as exclusivity.

37. The method for selecting a sequence identifier for detecting a target analyte of claim 36, wherein the sequence identifier of the specific organism predetermined comprises
a sequence identifier found in a resident flora or a housekeeping gene.

38. The method for selecting a sequence identifier for detecting a target analyte of claim 24, wherein the hierarchical structure is a biological systematic structure in which a higher layer encompasses a lower layer.

39. The method for selecting a sequence identifier for detecting a target analyte of claim 38, wherein the second layer is an immediately higher hierarchical position of the first layer, or a second highest layer or a highest layer on the hierarchical structure.

40. The method for selecting a sequence identifier for detecting a target analyte of claim 24, further comprising:
selecting a third sequence identifier for detecting the target analyte among the second sequence identifiers at least partially based on an occurring frequency of the second sequence identifiers inthe first layer and an occurring frequency of the second sequence identifiers in a third layer which is a higher layer than the second layer.

41. A method for generating a data structure for detecting a target analyte, which is performed by a computing device, the method comprising:

generating a first data structure in which inverted-indexing is applied to a data structure in which a plurality of first sequence identifiers are mapped to a plurality of genomes found in a first layer at which a target analyte is positioned, respectively in a biological taxonomy of organisms having a hierarchical structure;

generating a second data structure in which the inverted-indexing is applied to a data structure in which the plurality of sequence identifiers are mapped to a plurality of genomes found in a second layer which is a higher layer than the first layer at which the target analyte is positioned, respectively; and

selecting second sequence identifiers for detecting the target analyte among the first sequence identifiers at least partially based on an occurring frequency of the first sequence identifiers in the first layer obtained from the first data structure and an occurring frequency of the first sequence identifiers in a second layer obtained from the second data structure.

42. A computing device for selecting a sequence identifier for detecting a target analyte, comprising:

a memory; and

a processor, and

wherein the processor obtains a plurality of first sequence identifiers found in a first layer at which a target analyte is positioned in a biological taxonomy of organisms having a hierarchical structure;

obtains a first occurring frequency of the first sequence identifiers in the first layer by using a first data structure representing which source genome among the genomes in the first layer the plurality of first sequence identifiers belong to;

obtains a second occurring frequency of the first sequence identifiers in the second layer by using a second data structure representing which source genome among the genomes in the second layer higher than the first layer the plurality of first sequence identifiers belongs to; and

selects second sequence identifiers for detecting the target analyte among the first sequence identifiers at least partially based on an occurring frequency of the first sequence identifiers in the first layer and an occurring frequency of the first sequence identifiers in a second layer.

[FIG. 1]

[FIG. 2]

[FIG. 3]

[FIG. 4]

DOMAIN OR KINGDOM

VIBRIO GENUS

VIBRIO ADAPLATUS SPECIES

VIBRIO VULNIFICUS SPECIES

VIBRIO CHOLERA SPECIES

430

420

410

[FIG. 5]

**510 / 511 / 512**

| FIRST LAYER GENOME | SEQUENCE IDENTIFIER |
|---|---|
| GENOME 1 | A,B,C,D,E |
| GENOME 2 | A,C,E,S |
| GENOME 3 | A,B,C,D,G |
| ... | ... |
| GENOME 100 | A,F,T,R,Y,Z |

**520 / 521 / 522**

| SECOND LAYER GENOME (EXCLUDING FIRST LAYER) | SEQUENCE IDENTIFIER |
|---|---|
| GENOME 101 | D,Y,U |
| GENOME 102 | E,I,L |
| GENOME 103 | C,E,K |
| ... | ... |
| GENOME 300 | T,R,Y,Z |

**530 / 531 / 532 / 533**

| SEQUENCE IDENTIFIER | FIRST LAYER GENOME TO WHICH SEQUENCE IDENTIFIER BELONGS | NUMBER |
|---|---|---|
| A | GENOME 1, GENOME 2, GENOME 3 ... | 100 |
| B | GENOME 1, GENOME 3, GENOME 7... | 97 |
| D | GENOME 1, GENOME 3, GENOME 6... | 96 |
| E | GENOME 1, GENOME 2, GENOME 14 | 90 |
| F | GENOME 15, GENOME 25, GENOME 100... | 80 |
| ... | ... | |
| Z | ... | 70 |

**540 / 541 / 542 / 543**

| SEQUENCE IDENTIFIER | SECOND LAYER (EXCLUDING FIRST LAYER) GENOME TO WHICH SEQUENCE IDENTIFIER BELONGS | NUMBER |
|---|---|---|
| A | N/A | 0 |
| B | GENOME 108 | 1 |
| D | GENOME 101, GENOME 113 ... | 4 |
| E | GENOME 102, GENOME 103, GENOME 151... | 8 |
| F | GENOME 108, GENOME 109, GENOME 158... | 6 |
| K | ... | 15 |
| ... | ... | |
| Z | ... | 25 |

**530' / 531' / 532' / 533'**

| SEQUENCE IDENTIFIER | FIRST LAYER GENOME TO WHICH SEQUENCE IDENTIFIER BELONGS | NUMBER |
|---|---|---|
| A | GENOME 1, GENOME 2, GENOME 3 ... | 100 |
| B | GENOME 1, GENOME 3, GENOME 7... | 97 |
| D | GENOME 1, GENOME 3, GENOME 6... | 96 |
| E | GENOME 1, GENOME 2, GENOME 14 | 90 |
| F | GENOME 15, GENOME 25, GENOME 100... | 80 |
| ... | ... | |
| Z | ... | |

| THIRD LAYER GENOME (EXCLUDING FIRST LAYER) | SEQUENCE IDENTIFIER |
|---|---|
| GENOME 101 | D,Y,U |
| GENOME 102 | E,I,L |
| GENOME 103 | C,E,K |
| ... | ... |
| GENOME 300 | T,R,Y,Z |
| GENOME 301 | B,D,Y,U |
| GENOME 302 | E,I,Z |
| GENOME 303 | Z,K,X |
| ... | ... |
| GENOME 1000 | H,J,W,Z |

| SEQUENCE IDENTIFIER | FIRST LAYER GENOME TO WHICH SEQUENCE IDENTIFIER BELONGS | NUMBER |
|---|---|---|
| A | GENOME 1, GENOME 2, GENOME 3 ... | 100 |
| B | GENOME 1, GENOME 3, GENOME 7 ... | 97 |
| D | GENOME 1, GENOME 3, GENOME 6 ... | 96 |

| SEQUENCE IDENTIFIER | THIRD LAYER (EXCLUDING FIRST LAYER) GENOME TO WHICH SEQUENCE IDENTIFIER BELONGS | NUMBER |
|---|---|---|
| A | N/A | 0 |
| B | GENOME 108, GENOME 301, GENOME 401 ... | 7 |
| D | GENOME 101, GENOME 113, GENOME 301 ... | 11 |
| E | ... | 18 |
| F | ... | 19 |
| H | ... | 25 |
| ... | ... | |
| ... | ... | |
| Z | ... | 48 |

| SEQUENCE IDENTIFIER | FIRST LAYER GENOME TO WHICH SEQUENCE IDENTIFIER BELONGS | NUMBER |
|---|---|---|
| A | GENOME 1, GENOME 2, GENOME 3 | 100 |
| B | GENOME 1, GENOME 3, GENOME 7... | 97 |
| D | GENOME 1, GENOME 3, GENOME 6 | 96 |

[FIG. 6]

[FIG. 7]

START

**710**

OBTAIN A PLURALITY OF FIRST SEQUENCE IDENTIFIERS FOUND IN A FIRST LAYER AT WHICH A TARGET ANALYTE IS POSITIONED IN A BIOLOGICAL TAXONOMY OF ORGANISMS HAVING A HIERARCHICAL STRUCTURE

**720**

SELECT SECOND SEQUENCE IDENTIFIERS AMONG THE FIRST SEQUENCE IDENTIFIERS AT LEAST PARTIALLY BASED ON AN OCCURRING FREQUENCY OF THE FIRST SEQUENCE IDENTIFIERS IN THE FIRST LAYER AND AN OCCURRING FREQUENCY OF THE FIRST SEQUENCE IDENTIFIERS IN A SECOND LAYER WHICH IS A HIGHER LAYER THAN THE FIRST LAYER

**730**

SELECT A THIRD SEQUENCE IDENTIFIER FOR DETECTING THE TARGET ANALYTE AMONG THE SECOND SEQUENCE IDENTIFIERS AT LEAST PARTIALLY BASED ON AN OCCURRING FREQUENCY OF THE SECOND SEQUENCE IDENTIFIERS IN THE FIRST LAYER AND AN OCCURRING FREQUENCY OF THE SECOND SEQUENCE IDENTIFIERS IN A THIRD LAYER WHICH IS A HIGHER LAYER THAN THE SECOND LAYER.

END

[FIG. 8]

[FIG. 9]

[FIG. 10]

```
                              ┌─────────────┐
                              │    START    │
                              └──────┬──────┘
                                     │
                                     ▼                    ╭ 1010
        ┌────────────────────────────────────────────────────┐
        │   RECEIVE GENOMES AND PLURALITY OF SEQUENCE         │
        │     IDENTIFIERS COMPRISED IN GENOMES               │
        └────────────────────────┬───────────────────────────┘
                                 │
                                 ▼                        ╭ 1020
        ┌────────────────────────────────────────────────────┐
        │  GENERATE PLURALITY OF TOKENS BY TOKENIZING         │
        │     PLURALITY OF SEQUENCE IDENTIFIERS              │
        └────────────────────────┬───────────────────────────┘
                                 │
                                 ▼                        ╭ 1030
        ┌────────────────────────────────────────────────────┐
        │     GENERATE INDEX DATA STRUCTURE FOR              │
        │       PLURALITY OF GENERATED TOKENS               │
        └────────────────────────┬───────────────────────────┘
                                 │
                                 ▼
                              ┌─────────────┐
                              │     END     │
                              └─────────────┘
```

[FIG. 11]

START

1110

OBTAIN A PLURALITY OF FIRST SEQUENCE IDENTIFIERS FOUND IN A FIRST LAYER AT WHICH A TARGET ANALYTE IS POSITIONED IN A BIOLOGICAL TAXONOMY OF ORGANISMS HAVING A HIERARCHICAL STRUCTURE

1120

OBTAIN THE OCCURRING FREQUENCY OF THE FIRST SEQUENCE IDENTIFIERS IN THE FIRST LAYER BY USING A FIRST DATA STRUCTURE REPRESENTING WHICH SOURCE GENOME AMONG THE GENOMES IN THE FIRST LAYER THE PLURALITY OF FIRST SEQUENCE IDENTIFIERS BELONG TO

1130

OBTAIN THE OCCURRING FREQUENCY OF THE FIRST SEQUENCE IDENTIFIERS IN THE SECOND LAYER BY USING A SECOND DATA STRUCTURE REPRESENTING WHICH SOURCE GENOME AMONG THE GENOMES IN THE SECOND LAYER HIGHER THAN THE FIRST LAYER THE PLURALITY OF FIRST SEQUENCE IDENTIFIERS BELONG TO

1140

SELECT SECOND SEQUENCE IDENTIFIERS AMONG THE FIRST SEQUENCE IDENTIFIERS AT LEAST PARTIALLY BASED ON AN OCCURRING FREQUENCY OF THE FIRST SEQUENCE IDENTIFIERS IN THE FIRST LAYER AND AN OCCURRING FREQUENCY OF THE FIRST SEQUENCE IDENTIFIERS IN A SECOND LAYER WHICH IS A HIGHER LAYER THAN THE FIRST LAYER

END

[FIG. 12]

| GENOME LIST | SEQUENCE ID |
|---|---|
| GENOME 1 | A,B,C,D,E |
| GENOME 2 | A,C,E,S |
| GENOME 3 | A,B,C,D,G |
| . . . | . . . |
| GENOME 100 | A,F,T,R,Y,Z |

1210     1220

TOKENIZING AND INDEXING

1230     1240

| TOKEN | GENOME LIST |
|---|---|
| A | GENOME 1, GENOME 2, GENOME 3 ... |
| B | GENOME 1, GENOME 3, GENOME 7 ... |
| D | GENOME 1, GENOME 3, GENOME 6 ... |
| E | GENOME 1, GENOME 2, GENOME 14 ... |
| F | GENOME 15, GENOME 25, GENOME 100 ... |

[FIG. 13]

1400

genome_annotation (100r × 5c)

| gp_uid | tax_name | n_gene | n_named_unique_gene | gene_abb |
|---|---|---|---|---|
| 4,064 | Haemophilus influenzae (g-proteobacteria) | 2,015 | 897 | mltG\|arcA\|mltF\|fruB\|aceF\|aceE\|ruvX\|arcC\|mltC\|mltA\|... |
| 18,184 | Escherichia coli O121:H19 str. 06-3003 (E. coli) | 5,177 | 2,906 | cca\|arcA\|mltF\|cas7e\|ygfT\|rssB\|mddA\|gstA\|ygfK\|wrbA... |
| 18,186 | Salmonella enterica (enterobacteria) | 4,995 | 1,042 | arcA\|mltF\|cas7e\|rssB\|gstA\|aceE\|ruvX\|cedA\|flhC\|flhD\|... |
| 18,187 | Listeria monocytogenes (firmicutes) | 3,089 | 641 | rpmD\|rpmC\|minD\|mltG\|rpmF\|arcA\|minC\|rpmA\|hbp... |
| 18,189 | Listeria monocytogenes (firmicutes) | 3,178 | 560 | rpmD\|mltG\|minD\|arcA\|minC\|rpmF\|rpmG\|rpmJ\|rpmI\|... |
| 18,190 | Legionella pneumophila (g-proteobacteria) | 3,178 | 785 | mltG\|gstA\|wrbA\|aceF\|aceE\|ruvX\|flhB\|wipC\|wip8\|wip... |
| 18,191 | Ferruginibacter sp. (CFB group bacteria) | 3,143 | 278 | mltG\|dut\|rpmF\|ssb\|msrB\|msrA\|rpmB\|rpmA\|mqnC\|cc... |
| 18,192 | Formosa sediminum (CFB group bacteria) | 3,436 | 472 | rpmD\|mltG\|rpmC\|rpmF\|trkA\|rpmA\|rpmH\|rpmG\|rpm... |
| 18,194 | Shewanella sp. MR-4 (g-proteobacteria) | 4,076 | 1,047 | mltG\|arcA\|mltF\|aceF\|aceE\|ruvX\|flhB\|flhF\|scpB\|coxB\|r... |
| 18,195 | Mammaliicoccus sciuri (firmicutes) | 2,789 | 561 | rpmD\|mltG\|rpmC\|rpmF\|def\|rpmA\|rpmH\|rpmG\|rpmJ\|... |
| 18,196 | Pseudomonas aeruginosa PAO1 (g-proteobacteria) | 5,803 | 1,511 | mltG\|mltF\|arcA\|aceK\|wrbA\|aceF\|aceE\|ruvX\|flhB\|flhF\|s... |
| 18,198 | Burkholderia contaminans (b-proteobacteria) | 7,756 | 986 | mltG\|pgaA\|aceK\|gstA\|pgaB\|pgaC\|wrbA\|aceF\|aceE\|ru... |
| 18,199 | Mycobacterium tuberculosis variant bovis (high GC... | 4,107 | 879 | mymT\|arcA\|aceE\|ruvX\|lipV\|scpA\|lipY\|lipX\|scpB\|bluB\|... |
| 18,200 | Candidatus Pelagibacter ubique (a-proteobacteria) | 817 | 232 | rpmC\|dut\|trkA\|ssb\|rpmE\|msrB\|nusA\|msrA\|dnaX\|fabG... |
| 18,202 | Pseudomonas aeruginosa (g-proteobacteria) | 6,701 | 1,225 | mltG\|arcA\|mltF\|aceK\|wrbA\|aceF\|aceE\|flhB\|ruvX\|flhF\|s... |
| 18,203 | Bifidobacterium callitrichos DSM 23973 (high GC... | 2,452 | 345 | rpmD\|mltG\|rpmC\|dut\|rpmF\|rpmE\|def\|tuf\|msrB\|nusB\|... |
| 18,204 | Klebsiella pneumoniae (enterobacteria) | 5,880 | 2,232 | mltF\|arcA\|rssB\|gstA\|wrbA\|setB\|rssA\|mltD\|arcB\|mltC\|... |
| 18,205 | Escherichia coli (E. coli) | 5,603 | 2,932 | cca\|arcA\|mltF\|cas7e\|ygfT\|rssB\|mddA\|gstA\|gstB\|ygfK\|... |
| 18,207 | Streptomyces sp. MBT51 (high GC Gram+) | 6,848 | 538 | rpmD\|mltG\|rpmF\|rpmE\|def\|rpmB\|rpmA\|rpmH\|rpmG... |
| 18,208 | Campylobacter helveticus (e-proteobacteria) | 2,096 | 435 | rpmC\|mltG\|rpmF\|rpmE\|rpmA\|rpmH\|rpmG\|rpmJ\|rpm... |
| 18,209 | Corynebacterium glutamicum (high GC Gram+) | 3,072 | 460 | rpmD\|mltG\|rpmF\|def\|rpmB\|rpmA\|rpmH\|rpmG\|rpmJ\|... |
| 18,211 | Mycobacterium tuberculosis TRS11 (high GC Gram... | 4,111 | 943 | mymT\|arcA\|aceE\|lipU\|ruvX\|lipV\|scpA\|lipY\|lipX\|scpB\|b... |
| 18,212 | Staphylococcus aureus (firmicutes) | 2,857 | 727 | rpmD\|rpmC\|arcA\|rpmF\|ssp8\|sspC\|rpmA\|sspA\|rpmH\|... |
| 18,213 | Streptococcus pyogenes (firmicutes) | 1,855 | 425 | rpmD\|cas7c\|mltG\|rpmF\|arcA\|rpmA\|rexB\|rpmH\|rpm... |
| 18,214 | Escherichia coli TT12B (E. coli) | 5,339 | 2,893 | cca\|arcA\|mltF\|cas7e\|ygfT\|rssB\|mddA\|gstA\|gstB\|ygfK\|... |
| 18,215 | Salmonella enterica subsp. enterica serovar Saintpa... | 4,555 | 2,421 | arcA\|mltF\|cas7e\|rssB\|mddA\|gstA\|wrbA\|golB\|flhB\|flh... |

[FIG. 14]

EP 4 432 290 A1

```
In List Size : 782
Target TaxonName : Neisseria gonorrhoeae
Ex List Size : 0
Parent TaxonName - Rank : Neisseria - GENUS
Ex List Size : 2447
```

| (1510) | In: (1520) | ExParent: (1530) | ExAll: (1540) | (1550) |
|---|---|---|---|---|
| cas7c | In: 773 / 782 (98.85%) | ExParent: 221 / 2447 (9.03%) | ExAll: 9472 / 316500 (2.99%) | 8.289 |
| cas8c | In: 767 / 782 (98.08%) | ExParent: 216 / 2447 (8.83%) | ExAll: 9278 / 316500 (2.93%) | 8.410 |
| mobC | In: 683 / 782 (87.34%) | ExParent: 91 / 2447 (3.72%) | ExAll: 36866 / 316500 (11.65%) | 5.708 |
| cas4 | In: 760 / 782 (97.19%) | ExParent: 222 / 2447 (9.07%) | ExAll: 20036 / 316500 (6.33%) | 6.354 |
| ppk2 | In: 772 / 782 (98.72%) | ExParent: 274 / 2447 (11.20%) | ExAll: 78354 / 316500 (24.76%) | 2.759 |
| dinD | In: 759 / 782 (97.06%) | ExParent: 72 / 2447 (2.94%) | ExAll: 45392 / 316500 (14.34%) | 5.637 |
| fitB | In: 751 / 782 (96.04%) | ExParent: 22 / 2447 (0.90%) | ExAll: 46 / 316500 (0.01%) | 106.177 |
| fitA | In: 752 / 782 (96.16%) | ExParent: 15 / 2447 (0.61%) | ExAll: 43 / 316500 (0.01%) | 155.012 |
| nosZ | In: 773 / 782 (98.85%) | ExParent: 222 / 2447 (9.07%) | ExAll: 4539 / 316500 (1.43%) | 9.494 |

1500

[FIG. 15]

[FIG. 16]

## INTERNATIONAL SEARCH REPORT

| International application No. |
| --- |
| **PCT/KR2022/016787** |

| A. | CLASSIFICATION OF SUBJECT MATTER |
| --- | --- |

**G16B 40/00**(2019.01)i; **G16B 30/10**(2019.01)i; **G16B 20/20**(2019.01)i

According to International Patent Classification (IPC) or to both national classification and IPC

| B. | FIELDS SEARCHED |
| --- | --- |

Minimum documentation searched (classification system followed by classification symbols)

G16B 40/00(2019.01); G06F 19/10(2011.01); G06F 19/12(2011.01); G06F 19/22(2011.01); G06F 19/24(2011.01); G16B 20/10(2019.01); G16B 30/00(2019.01); G16B 30/10(2019.01); G16B 40/20(2019.01); G16B 50/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 컴퓨팅 장치(computing device), 서열 식별자(sequence identifier), 선정(selection), 타겟 분석물(target analyte), 검출(detection), 계층 구조(hierarchical structure), 출현 빈도(frequency of occurrence), 유전체(gene), 스코어값(score), 데이터 구조(data structure), 역 인덱싱(inverted-indexing), 차세대 염기서열 분석(next generation sequencing, NGS)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2021-0109207 A (UNIVERSITY-INDUSTRY COOPERATION GROUP OF KYUNG HEE UNIVERSITY) 06 September 2021 (2021-09-06)<br>See abstract, claim 1, and figure 1. | 1-42 |
| A | KR 10-2015-0086164 A (ELECTRONICS AND TELECOMMUNICATIONS RESEARCH INSTITUTE) 27 July 2015 (2015-07-27)<br>See entire document. | 1-42 |
| A | KR 10-2017-0000707 A (SAMSUNG ELECTRONICS CO., LTD.) 03 January 2017 (2017-01-03)<br>See entire document. | 1-42 |
| A | KR 10-2021-0094783 A (THE CATHOLIC UNIVERSITY OF KOREA INDUSTRY-ACADEMIC COOPERATION FOUNDATION) 30 July 2021 (2021-07-30)<br>See entire document. | 1-42 |

☑ Further documents are listed in the continuation of Box C.    ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **13 February 2023** | **13 February 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2022/016787** |

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2017-0046315 A (REPUBLIC OF KOREA(MANAGEMENT : RURAL DEVELOPMENT ADMINISTRATION)) 02 May 2017 (2017-05-02)<br>    See entire document. | 1-42 |
| A | KR 10-2019-0069929 A (SOONCHUNHYANG UNIVERSITY INDUSTRY ACADEMY COOPERATION FOUNDATION) 20 June 2019 (2019-06-20)<br>    See entire document. | 1-42 |

Form PCT/ISA/210 (second sheet) (July 2022)

**INTERNATIONAL SEARCH REPORT**
**Information on patent family members**

International application No.

**PCT/KR2022/016787**

| Patent document cited in search report | | | Publication date (day/month/year) | Patent family member(s) | | | Publication date (day/month/year) |
|---|---|---|---|---|---|---|---|
| KR | 10-2021-0109207 | A | 06 September 2021 | CN | 115280419 | A | 01 November 2022 |
| | | | | WO | 2021-172780 | A1 | 02 September 2021 |
| KR | 10-2015-0086164 | A | 27 July 2015 | US | 2015-0199476 | A1 | 16 July 2015 |
| KR | 10-2017-0000707 | A | 03 January 2017 | US | 2016-0378914 | A1 | 29 December 2016 |
| KR | 10-2021-0094783 | A | 30 July 2021 | KR | 10-2345994 | B1 | 03 January 2022 |
| | | | | WO | 2021-149913 | A1 | 29 July 2021 |
| KR | 10-2017-0046315 | A | 02 May 2017 | None | | | |
| KR | 10-2019-0069929 | A | 20 June 2019 | None | | | |

Form PCT/ISA/210 (patent family annex) (July 2022)